# EUROPEAN PATENT APPLICATION

(11) **EP 4 552 657 A1**
(43) Date of publication of application: **14.05.2025**
(21) Application number: 23208366.7
(22) Date of filing: 07.11.2023
(51) Int. Cl.: A61K 51/10, A61P 35/00

(54) **RADIOPHARMACEUTICAL COMPLEXES AND COMBINATIONS**

(71) Applicant: Bayer Aktiengesellschaft, 51373 Leverkusen (DE); Bayer AS, 0283 Oslo (NO)
(72) Inventor: The designation of the inventor has not yet been filed
(74) Representative: BIP Patents

(57) **Abstract**

The present invention relates to tissue-targeting compounds. In particular, the present invention relates to tissue-targeting compounds comprising a monoclonal antibody or an antigen-binding fragment thereof having binding affinity for HER2, GPC3, CEACAM5, TROP2, GUCY2C, cMet, FoIRa, DLL3, Nectin-4 or STEAP1. Further, the present invention relates to combinations, preferably pharmaceutical combinations comprising a tissue-targeting compound of formula (I) and a further pharmaceutical agent. Said combinations are useful in therapy, preferably in treating hyperproliferative diseases such as cancer.

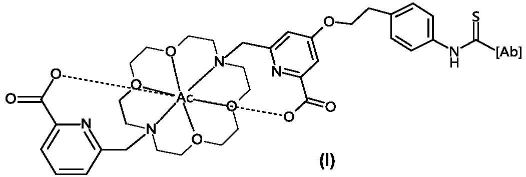

## Description

### FIELD OF THE INVENTION

The present invention relates to tissue-targeting compounds. In particular, the present invention relates to tissue-targeting compounds comprising a monoclonal antibody or an antigen-binding fragment thereof having binding affinity for HER2, GPC3, CEACAM5, TROP2, GUCY2C, cMet, FolRa, DLL3, Nectin-4 or STEAP1. Further, the present invention relates to combinations, preferably pharmaceutical combinations comprising a tissue-targeting compound of formula (I) and a further pharmaceutical agent. Said tissue-targeting compounds and/or combinations are useful in diagnostics and/or therapy, preferably in therapy, more preferably in treating hyperproliferative diseases such as cancer.

Further, the present invention relates to a process for the preparation of said tissue-targeting compounds and to their use in diagnostics and/or in the treatment of disease, preferably in the treatment of disease, particularly against hyperproliferative diseases, in particular against cancer.

### BACKGROUND OF THE INVENTION

Specific cell killing can be essential for the successful treatment of a variety of diseases in mammalian subjects. Typical examples of this are the treatment of malignant diseases such as sarcomas and carcinomas. However, the selective elimination of certain cell types can also play a key role in the treatment of other diseases, especially hyperplastic and neoplastic diseases. I

The most common methods of selective treatment are currently surgery and external beam irradiation. Targeted radionuclide therapy is, however, a promising and developing area with the potential to deliver highly cytotoxic radiation specifically to cell types associated with disease. The most common forms of radiopharmaceuticals currently authorized for use in humans employ beta-emitting and/or gamma-emitting radionuclides. There has, however, been some interest in the use of alpha-emitting radionuclides in therapy because of their potential for more potent cell killing.

The radiation range of typical alpha emitters in physiological surroundings is generally less than 100 micrometers, the equivalent of only a few cell diameters. This makes these sources well suited for the treatment of tumors, including micro-metastases, because they have the range to reach neighboring cells within a tumor but if they are well targeted then little of the radiated energy will pass beyond the target cells. Thus, not every tumor cell needs to be targeted and damage to surrounding healthy tissue may be minimized (see Feinendegen et al., Radiat. Res. 148:195-201 (1997)). In contrast, a beta particle has a range of 1 mm or more in water (see Wilbur, Antibody Immunocon. Radiopharm. 4: 85-96 (1991)).

The energy of alpha-particle radiation is high in comparison with that carried by beta particles, gamma rays and X-rays, typically being 5-8 MeV, or 5 to 10 times that of a beta particle and 20 or more times the energy of a gamma ray. Thus, this deposition of a large amount of energy over a very short distance gives alpha-radiation an exceptionally high linear energy transfer (LET), high relative biological efficacy (RBE) and low oxygen enhancement ratio (OER) compared to gamma and beta radiation (see Hall, "Radiobiology for the radiologist", Fifth edition, Lippincott Williams & Wilkins, Philadelphia PA, USA, 2000). This explains the exceptional cytotoxicity of alpha emitting radionuclides and also imposes stringent demands on the biological targeting of such isotopes and upon the level of control and study of alpha emitting radionuclide distribution which is necessary in order to avoid unacceptable side effects.

So far, with regards to the application in radioimmunotherapy the main attention has been focused on 211At, 213Bi and 225Ac and these three nuclides have been explored in clinical immunotherapy trials. However, although targeted radiotherapy has been practiced for some time using macrocyclic complexes of radionuclides, the compounds currently in use, which typically employ the macrocyclic chelator DOTA (2,2',2",2"'-(1,4,7,10-Tetraazacyclododecane-1,4,7,10-tetrayl)tetraacetic acid) are lacking in stability, leading to the dissociation of the radionuclide from the chelating macrocycle, which results in a reduced selectivity and activity to the targeted tissue and an increased toxicity to the non-targeted tissue.

For example, it has been reported that a high chelator-to-antigen (CAR) of 10 has been used in a pre-clinical study using a one-step radiolabeling procedure of a DOTA antibody chelator conjugate (ACC) (William F. Maguire, Michael R. McDevitt, Peter M. Smith-Jones, David A. Scheinberg. "Efficient one-step radiolabeling of monoclonal antibodies to high specific activity with Actinium-225 for alpha-particle radioimmunotherapy of cancer", J. Nucl. Med. 2014; 55(9): 1492-1498). However, after 2 h labeling at 37°C, quantitative labeling was not achieved which subsequentially requires a purification step to remove non-labeled Ac-225.

Thus, there is a need to provide new antibody chelator conjugates which can be radiolabeled under more favorable conditions, and which exhibit lower CAR ratios.

The present invention provides tissue-targeting compounds in which Ac-225 can be chelated quantitatively at room temperature after one hour leading to complexes with significantly lower CAR ratios (<1).

While the use of larger macrocycles has led to more stable complexation of especially radionuclides of larger size, there is a need for the development of targeting compounds with improved stability, selectivity and efficacy, especially in the field of hyperproliferative diseases such as cancer. For some types of cancer, patients initially respond to therapy but eventually develop resistances (Swami U, McFarland TR, Nussenzveig R, Agarwal N. Advanced Prostate Cancer: Treatment Advances and Future Directions. Trends Cancer 2020;6(8):702-15 doi 10.1016/j.trecan.2020.04.010). Thus, there is a need for new agents capable of selectively targeting specific cells and cell types in malignant diseases which are highly effective and reduce, mitigate and/or avoid the prevalence of resistances. In particular, there is a need to provide new therapeutic agents for the treatment of hyperproliferative diseases, especially cancer and more especially bladder, brain, breast, colon, liver, lung, pancreatic, prostate, HNSCC and stomach cancers, which are currently among the most prevalent types of cancer. The tissue-targeting compounds of the present invention have high affinities for HER2, GPC3, CEACAM5, TROP2, GUCY2C, cMet, FolRa, DLL3, Nectin-4 or STEAP1, making them suitable compounds to address hyperproliferative diseases such as cancer, especially bladder, brain, breast, colon, liver, lung, pancreatic, prostate, HNSCC and stomach cancer.

The targets for the tissue-targeting compounds of formula (I) of the present invention, i.e., HER2, GPC3, CEACAM5, TROP2, GUCY2C, cMet, FolRa, DLL3, Nectin-4 or STEAP1 are of particular interest:
Human epidermal growth factor receptor 2 (HER2) is a transmembrane tyrosine kinase receptor that influences cell growth, division, and repair; with increased expression, it can promote tumorigenesis. HER2 is frequently overexpressed or amplified and occasionally mutated in a variety of human cancers and is a well-established therapeutic target in breast and gastric cancers.

Glypican-3 (GPC3) is a surface heparan sulfate proteoglycan expressed in approximately 80% of hepatocellular carcinomas (HCC) and is associated with poorer prognoses. It is postulated that GPC3 plays a role in the regulation of cell division and growth. GPC3 is a clinically relevant biomarker for imaging and therapeutics, as its expression is HCC tumor-specific and absent from normal and other pathological liver tissues.

CEACAM5, also known as CEA or carcinoembryonic antigen, is a cell surface glycoprotein that has gained significant attention in the field of cancer research. CEACAM5 is frequently overexpressed in various cancers, including colorectal, lung, breast, and pancreatic cancers. Its expression has been linked to the prognosis of certain cancers. Studies have shown that higher levels of CEACAM5 are associated with more aggressive tumor behavior, including increased invasiveness and metastasis. Due to its presence on the surface of tumor cells, CEACAM5 can serve as an attractive target for antibody-based therapies. One advantage of targeting CEACAM5 is its relatively restricted expression in healthy tissues, predominantly limited to gastrointestinal epithelial cells during fetal development.

Trophoblast cell surface antigen 2 (TROP2) is overexpressed in different tumor tissues (breast, cervix, colorectal, esophagus, gastric and lung cancer) and plays an important role in tumor cell proliferation, apoptosis, and invasion, thereby impacting the prognosis and treatment of cancer patients. As a result, TROP2 has become an attractive therapeutic target in cancer treatment, in particular for the treatment of HNSCC (head and neck squamous cell carcinoma).

GUCY2C is primarily expressed in the gastrointestinal tract and is elevated in various types of gastrointestinal cancers, including colorectal, gastric, and pancreatic cancers. This characteristic makes it an attractive target due to its specificity to tumor cells and limited expression in normal tissues. Its expression is associated with more aggressive tumor phenotypes, including increased invasiveness and metastasis, as well as poor patient prognosis.

c-Met (mesenchymal-epithelial transition factor) is a receptor tyrosine kinase for hepatocyte growth factor (HGF)/Scatter Factor (SF). The normal functions of the c-Met pathway are largely restricted to organ morphogenesis during development as well as to tissue damage repair and regeneration in adults. In normal adult tissues expression is generally low. c-Met is a well-known target and has been found to be aberrantly activated in human cancers via mutation, amplification or protein overexpression. It is highly expressed in multiple cancers, including lung adeno (38%), lung squamous (11%), CRC (23%), RCC (54-78%), melanoma (20%), mesothelioma (46%), thyroid (74%), gastric cancer (12%), pancreatic cancer (21%) and oesophageal cancer (22%). c-Met is an oncogenic driver in some patients, making it less likely to develop resistance through loss of target expression. c-Met expression on the cell membrane makes it an accessible target for targeting modalities binding to c-Met.

FOLR1 is an internalizing GPI anchored membrane protein. FOLR1 is responsible for binding to folic acid and its derivatives, which becomes crucial during fetal development. By adding folate supplementation during pregnancy, neural tube defects in the fetus are prevented. Anti folates are used for treatment of various tumor indications. High FOLR1 levels are associated with poor differentiation, aggressive tumors & resistance to conventional chemotherapy. FOLR1 is a clinical validated target through the approval of mirvetuximab soravtansine. Elevated FOLR1 expression levels are seen in ovarian serous cystadenocarcinoma and a subset of breast invasive carcinoma, lung adenocarcinoma, and endometrial cancer.

It has surprisingly been found that the tissue-targeting compounds of formula (I) and the combinations comprising a tissue-targeting compound of formula (I) and a further pharmaceutical agent of the present invention show advantageous properties with regard to their stability, activity, efficacy and selectivity. In particular, the tissue-targeting compounds of formula (I) of the present invention show high values for the immunoreactive fraction (IRF) at low chelator-to-antigen (CAR) ratios and high monomeric purity, thus leading to a higher fraction of radionuclide-labelled compound having affinity for HER2, GPC3, CEACAM5, TROP2, GUCY2C, cMet, FolRa, DLL3, Nectin-4 or STEAP1. Further, the tissue-targeting compounds of formula (I) of the present invention show a favorable biodistribution in vivo and low accumulation in the liver, which is a sign of reduced dissociation of the radionuclide from the chelating moiety and therefore of increased stability. In contrast to previously reported examples in the literature, the tissue-targeting compounds of formula (I) of the present invention can be labelled with the radionuclide under very mild conditions and at reduced temperatures (i.e., room temperature), thereby reducing antibody denaturation and the amount of non-binding fraction in the final product. Thus, the tissue-targeting compounds of formula (I) of the present invention show a better tumor to liver ratio and can therefore treat disease more effectively and selectively, reducing damage to non-targeted tissue while maintaining a high potency against cancer cells in the targeted tissue. In combination with one or more further pharmaceutical agents, a greater therapeutic efficacy can be achieved than when either compound is used alone.

In the field of radiopharmaceuticals, there is an increased need for the provision of radionuclide-containing compounds and/or combinations comprising radionuclide-containing compounds which are stable over sufficient periods of time that allow their delivery to the administering physician and the patient without suffering from radiopharmaceutical degradation, e.g., by decomplexation of the radionuclide from the chelator. Increasing the stability of the radiopharmaceutical in radionuclide-containing compounds and/or combinations comprising radionuclide-containing compounds is especially desirable from a logistical perspective, since this would allow for the reliable delivery of the radiopharmaceutical-containing drug product across countries and/or continents. It would be desirable to have radionuclide-containing compounds and/or combinations comprising radionuclide-containing compounds available which are stable for at least 48 hours, preferably for 96 hours and/or which have a monomer content of at least 85% for at least 48 hours, preferably a monomer content of at least 90% for at least 48 hours.

Further, it has been surprisingly found that the compounds of formula (I) of the present invention are capable of chelating 213Bismuth (213Bi) much more efficiently than their counterparts featuring a DOTA chelator. 213Bi is a decay product of the alpha-particle-emitting radionuclide 225Ac present in the compounds of formula (I) of the present invention and is already present within a few hours after the preparation of the 225Ac conjugates. If left uncomplexed, it can accumulate in the tubular cells of the kidneys and lead to undesired, hazardous side effects. Since radionuclide-containing drug conjugates are not administered to the patient immediately after their preparation and need to be stored until dosing, it is crucial that the 213Bi produced until administered to the patient is effectively chelated. The compounds of formula (I) of the present invention provide for means to chelate 213Bi in a more effective and efficient manner than their DOTA counterparts.

The present invention solves these issues by providing tissue-targeting compounds of formula (I) and combinations comprising a tissue-targeting compound of formula (I) and a further pharmaceutical agent.

In particular, combinations comprising a tissue-targeting compound of formula (I) and a further pharmaceutical agent of the present invention will serve to:
(1) yield better efficacy in reducing the growth of a tumor or even eliminate the tumor as compared to administration of either agent alone,
(2) provide for the administration of lesser amounts of the administered targeted compounds and/or chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time until tumor progression, and/or
(8) yield efficacy and tolerability results at least as good as those of the agents used alone, compared to known instances where other cancer agent combinations produce inhibitory effects.

### SUMMARY OF THE INVENTION

The present invention relates to tissue-targeting compounds of formula (I) 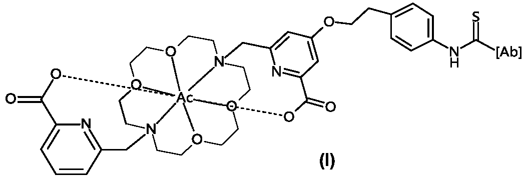 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1.

### DEFINITIONS

The term "comprising" when used in the specification includes "consisting of".

If within the present text any item is referred to as "as mentioned herein", it means that it may be mentioned anywhere in the present text.

In the context of the present invention, "tissue targeting" is used herein to indicate that the substance in question (i.e. a tissue-targeting compound, a tissue-targeting actinium complex and/or a tissue-targeting moiety, particularly when in the form of a tissue-targeting complex as described herein), serves to localize itself (and particularly to localize any conjugated actinium complex) preferentially to at least one tissue site at which its presence (e.g. to deliver a radioactive decay) is desired. Thus, a tissue-targeting compound, complex, group or moiety serves to provide greater localization to at least one desired site in the body of a subject following administration to that subject in comparison with the concentration of an equivalent complex not having the targeting moiety. The targeting moiety in the present case will be preferably selected to bind specifically to cell-surface receptors associated with cancer cells or other receptors associated with the tumor microenvironment.

In the context of the present invention, the term "HER2" refers to "Receptor tyrosine-protein kinase erbB"-2 antigen encoded by the ERBB2 gene; UniProt ID: P04626 · ERBB2_HUMAN.

In the context of the present invention, the term "GPC3" refers to "Glypican-3"antigen encoded by the GPC3 gene; UniProt ID: P51654 · GPC3_HUMAN.

In the context of the present invention, the term "CEACAM5" refers to "Carcinoembryonic antigen-related cell adhesion molecule 5" antigen encoded by the CEACAM5 gene; UniProt ID: P06731 · CEAM5_HUMAN.

In the context of the present invention, the term "GUCY2C" refers to "Guanylyl cyclase C" antigen encoded by the GUCY2C gene; UniProt ID: P25092 · GUC2C_HUMAN.

In the context of the present invention, the term "cMet" refers to "Hepatocyte growth factor receptor" antigen encoded by the MET gene; UniProt ID: P08581 · MET_HUMAN.

In the context of the present invention, the term "FolRa" refers to "Folate receptor alpha" antigen encoded by the FOLR1gene; UniProt ID: P15328 · FOLR1_HUMAN.

In the context of the present invention, the term "DLL3" refers to "Delta-like protein 3" antigen encoded by the DLL3 gene; UniProt ID: Q9NYJ7 · DLL3_HUMAN.

In the context of the present invention, the term "Nectin-4" refers to "Nectin-4" antigen encoded by the NECTIN4 gene; UniProt ID: Q96NY8 . NECT4_HUMAN.

In the context of the present invention, the term "STEAP1" refers to "Metalloreductase STEAP1" antigen encoded by the STEAP1 gene; UniProt ID: Q9UHE8 . STEA1_HUMAN.

In the context of the present invention, the term "TROP2" refers to "tumor associated calcium signal transducer 2" antigen encoded by the TASTD2 gene; UniProt ID: P09758 · TACD2_HUMAN.

The compounds of formula (I) of the present invention comprise a chelating moiety comprising a complexed actinium atom (Ac). In the context of the present invention, the term "Ac" means an ion of at least one alpha-emitting actinium isotope. Preferably, the alpha-emitting actinium isotope is 225Ac. Preferably, the ion of at the least one alpha-emitting actinium isotope is ²²⁵Ac having a triple positive charge, i.e., ²²⁵Ac³⁺. Thus, in the context of the present invention, the term "Ac" preferably, but not exclusively, means ²²⁵Ac³⁺.

In the compounds of formula (I) of the present invention, the actinium (Ac) atom is shown as being complexed to four oxygen and two nitrogen atoms of the macrocyclic ring as well as to two carboxylate groups. While this is the assumed complexation pattern for the actinium atom, this depiction includes all possible and conceivable cases in which one or more of the bonds is not present, e.g., where the actinium atom is not bound to all heteroatoms of the macrocyclic ring, or to the carboxylate groups, etc.

In the context of the present invention, the term IRF refers to the Immunoreactive Fraction i.e., the fraction of the labelled product (i.e., the compounds of formula (I) of the present invention) which is capable of binding to the target. The Lindmo assay (Lindmo T., et al. (1984) "Determination of the immunoreactive fraction of radiolabeled monoclonal antibodies by linear extrapolation to binding at infinite antigen excess." J. Immunol. Methods. 72, 77-89) is the most commonly used method for assessing the immunoreactive fraction.

In the context of the present invention, the term CAR refers to the Chelator-to-Antigen Ratio, which is a measure of the specific activity of the radiolabeled compound (e.g., the compounds of formula (I) of the present invention).

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. Unless otherwise indicated, a particular polypeptide sequence also implicitly encompasses conservatively modified variants thereof.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "antibody", as used herein, is intended to refer to immunoglobulin molecules including, but not limited to, full-length antibodies and monovalent antibodies. "Full-length antibodies" are preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g., three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4. "Monovalent antibodies" as used herein are preferably comprised of three polypeptide chains, two heavy (H) chains and one light (L) chain which are typically inter-connected by disulfide bonds. One heavy chain is comprised of a heavy chain variable region (abbreviated herein as VH) and a heavy chain constant region. The heavy chain constant region can comprise e.g., three domains CH1, CH2 and CH3. The other heavy chain is comprised of a heavy chain constant region only. The light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4.

As used herein, the term "Complementarity Determining Regions" (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immunolological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop.

Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA1, and IgA2. A preferred class of immunoglobulins for use in the present invention is IgG.

The heavy-chain constant domains that correspond to the different classes of antibodies are called [alpha], [delta], [epsilon], [gamma], and [mu], respectively. The subunit structures and three-dimensional configurations of different classes of immunoglobulins are well known. As used herein antibodies are conventionally known antibodies and functional fragments thereof.

A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs.

"Functional fragments", "antigen-binding antibody fragments", or "antibody fragments" of the invention include but are not limited to Fab, Fab', Fab'-SH, F(ab')₂, and Fv fragments; diabodies; single domain antibodies (DAbs), linear antibodies; single-chain antibody molecules (scFv); and multi-specific, such as bi- and tri-specific, antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "multi-specific" or "multi-functional" antibody is understood to have each of its binding sites identical. The F(ab')₂ or Fab may be engineered to minimize or completely remove the intermolecular disulfide interactions that occur between the CH1 and CL domains.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal Lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

Variants of the antibodies or antigen-binding antibody fragments contemplated in the invention are molecules in which the binding activity of the antibody or antigen-binding antibody fragment is maintained.

"Binding proteins" contemplated in the invention are for example antibody mimetics, such as Affibodies, Adnectins, Anticalins, DARPins, Avimers, Nanobodies (reviewed by Gebauer M. et al., Curr. Opinion in Chem. Biol. 2009; 13:245-255; Nuttall S.D. et al., Curr. Opinion in Pharmacology 2008; 8:608-617).

A "human" antibody or antigen-binding fragment thereof is hereby defined as one that is not chimeric (e.g., not "humanized") and not from (either in whole or in part) a non-human species. A human antibody or antigen-binding fragment thereof can be derived from a human or can be a synthetic human antibody. A "synthetic human antibody" is defined herein as an antibody having a sequence derived, in whole or in part, in silico from synthetic sequences that are based on the analysis of known human antibody sequences. In silico design of a human antibody sequence or fragment thereof can be achieved, for example, by analyzing a database of human antibody or antibody fragment sequences and devising a polypeptide sequence utilizing the data obtained there from. Another example of a human antibody or antigen-binding fragment thereof is one that is encoded by a nucleic acid isolated from a library of antibody sequences of human origin (e.g., such library being based on antibodies taken from a human natural source). Examples of human antibodies include antibodies as described in Söderlind et al., Nature Biotech. 2000, 18:853-856.

A "humanized antibody" or humanized antigen-binding fragment thereof is defined herein as one that is (i) derived from a non-human source (e.g., a transgenic mouse which bears a heterologous immune system), which antibody is based on a human germline sequence; (ii) where amino acids of the framework regions of a non-human antibody are partially exchanged to human amino acid sequences by genetic engineering or (iii) CDR-grafted, wherein the CDRs of the variable domain are from a non-human origin, while one or more frameworks of the variable domain are of human origin and the constant domain (if any) is of human origin.

A "chimeric antibody" or antigen-binding fragment thereof is defined herein as one, wherein the variable domains are derived from a non-human origin and some or all constant domains are derived from a human origin.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. The term monoclonal antibody specifically includes chimeric, humanized and human antibodies.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or non-proteinaceous solutes.

An "isolated" nucleic acid is one that has been identified and separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As used herein, an antibody "binds specifically to", is "specific to/for" or "specifically recognizes" an antigen of interest, e.g. a tumor-associated polypeptide antigen target or an antigen-binding polypeptide target (as e.g. an antigen-binding antibody), is one that binds the antigen-target with sufficient affinity such that the antibody is useful as a therapeutic agent in targeting a cell or tissue expressing the antigen or one that binds an antigen-binding polypeptide target with sufficient affinity such that the antibody is useful as a reversal agent to neutralize the therapeutic activity of this antigen-binding polypeptide (e.g. an antigen-binding antibody) and does not significantly cross-react with other proteins or does not significantly cross-react with proteins other than orthologs and variants (e.g. mutant forms, splice variants, or proteolytically truncated forms) of the aforementioned target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent K_{D} for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less. An antibody "binds specifically to," is "specific to/for" or "specifically recognizes" an antigen if such antibody is able to discriminate between such antigen and one or more reference antigen(s). In its most general form, "specific binding", "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to, surface plasmon resonance (SPR), Western blots, ELISA-, RIA-, ECL-, IRMA-tests and peptide scans. For example, a standard ELISA assay can be carried out. The scoring may be carried out by standard color development (e.g., secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

"Binding affinity" or "affinity" refers to the strength of the total sum of non-covalent interactions between a single binding site of a molecule and its binding partner. Unless indicated otherwise, as used herein, "binding affinity" refers to intrinsic binding affinity which reflects a 1:1 interaction between members of a binding pair (e.g., an antibody and an antigen). The dissociation constant "K_{D}" is commonly used to describe the affinity between a molecule (such as an antibody) and its binding partner (such as an antigen) i.e., how tightly a ligand binds to a particular protein. Ligand-protein affinities are influenced by non-covalent intermolecular interactions between the two molecules. Affinity can be measured by common methods known in the art, including those described herein. In one embodiment, the "K_{D}" or "K_{D} value" according to this invention is measured by using surface plasmon resonance assays using suitable devices including but not limited to Biacore instruments like Biacore T100, Biacore T200, Biacore 2000, Biacore 4000, a Biacore 3000 (GE Healthcare Biacore, Inc.), or a ProteOn XPR36 instrument (Bio-Rad Laboratories, Inc.).

"Antibody-dependent cell-mediated cytotoxicity" or "ADCC" refers to a form of cytotoxicity in which secreted Ig bound onto Fc gamma receptors (FcγRs) present on certain cytotoxic cells (e.g. NK cells, neutrophils, and macrophages) enable these cytotoxic effector cells to bind specifically to an antigen-bearing target cell and subsequently kill the target cell e.g. with cytotoxins. To assess ADCC activity of an antibody of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202: 163 (1996), may be performed. Polypeptide variants with altered Fc region amino acid sequences (polypeptides with a variant Fc region) and increased or decreased C1q binding are described, e.g., in US Patent No. 6,194,551 B1 and WO 1999/51642.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full-length of the sequences being compared.

Substantial sequence identity/similarity may be taken as having a sequence similarity/identity of at least 80% to the complete sequences and/or at least 90% to the specific binding regions (e.g., the CDR regions). Preferable sequence similarity or more preferably identity may be at least 92%, 95%, 97%, 98% or 99%. Sequence similarity and/or identity may be determined using the "BestFit" program of the Genetics Computer Group Version 10 software package from the University of Wisconsin. The program uses the local had algorithm of Smith and Waterman with default values: Gap creation penalty=8, Gap extension penalty=2, Average match=2.912, average mismatch 2.003.

The terms "polynucleotide" or "nucleic acid", as used interchangeably herein, refer to chains of nucleotides of any length, and include DNA and RNA. The nucleotides can be deoxyribonucleotides, ribonucleotides, modified nucleotides or bases, and/or their analogs, or any substrate that can be incorporated into a chain by DNA or RNA polymerase. A polynucleotide may comprise modified nucleotides, such as methylated nucleotides and their analogs.

"Sequence homology" indicates the percentage of amino acids that either is identical or that represent conservative amino acid substitutions.

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding fragments thereof or variants thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g., bacteria, examples for eukaryotic hosts cells are yeasts, insects and insect cells, plants and plant cells, transgenic animals, or mammalian cells. In some embodiments, the DNAs encoding the heavy and light chains are inserted into separate vectors. In other embodiments, the DNA encoding the heavy and light chains is inserted into the same vector. It is understood that the design of the expression vector, including the selection of regulatory sequences is affected by factors such as the choice of the host cell, the level of expression of protein desired and whether expression is constitutive or inducible.

Useful expression vectors for bacterial use are constructed by inserting a DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include but are not limited to E. coli, Bacillus subtilis, Salmonella typhimurium and various species within the genera Pseudomonas, Streptomyces, and Staphylococcus.

Bacterial vectors may be, for example, bacteriophage-, plasmid- or phagemid-based. These vectors can contain a selectable marker and a bacterial origin of replication derived from commercially available plasmids typically containing elements of the well-known cloning vector pBR322 (ATCC 37017). Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable.

Antibodies of the present invention or antigen-binding fragments thereof or variants thereof include naturally purified products, products of chemical synthetic procedures, and products produced by recombinant techniques from a prokaryotic host, including, for example, E. coli, Bacillus subtilis, Salmonella typhimurium and various species within the genera Pseudomonas, Streptomyces, and Staphylococcus, preferably, from E. coli cells.

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. Expression of the antibodies may be constitutive or regulated (e.g. inducible by addition or removal of small molecule inductors such as Tetracyclin in conjunction with Tet system). For further description of viral regulatory elements, and sequences thereof, see e.g., U.S. 5,168,062 by Stinski, U.S. 4,510,245 by Bell et al. and U.S. 4,968,615 by Schaffner et al.. The recombinant expression vectors can also include origins of replication and selectable markers (see e.g., U.S. 4,399,216, 4,634,665 and U.S. 5,179,017). Suitable selectable markers include genes that confer resistance to drugs such as G418, puromycin, hygromycin, blasticidin, zeocin/bleomycin or methotrexate or selectable marker that exploit auxotrophies such as Glutamine Synthetase (Bebbington et al., Biotechnology (N Y). 1992 Feb;10(2):169-75), on a host cell into which the vector has been introduced. For example, the dihydrofolate reductase (DHFR) gene confers resistance to methotrexate, neo gene confers resistance to G418, the bsd gene from Aspergillus terreus confers resistance to blasticidin, puromycin N-acetyl-transferase confers resistance to puromycin, the Sh ble gene product confers resitance to zeocin, and resistance to hygromycin is conferred by the E. coli hygromycin resistance gene (hyg or hph). Selectable markers like DHFR or Glutamine Synthetase are also useful for amplification techniques in conjunction with MTX and MSX.

Transfection of the expression vector into a host cell can be carried out using standard techniques such as electroporation, nucleofection, calcium-phosphate precipitation, lipofection, polycation-based transfection such as polyethlylenimine (PEI)-based transfection and DEAE-dextran transfection.

Suitable mammalian host cells for expressing the antibodies, antigen binding fragments thereof or variants thereof provided herein include but are not limited to Chinese Hamster Ovary (CHO cells) such as CHO-K1, CHO-S, CHO-K1SV [including dhfr- CHO cells, described in Urlaub and Chasin, (1980) Proc. Natl. Acad. Sci. USA 77:4216-4220 and Urlaub et al., Cell. 1983 Jun;33(2):405-12, used with a DHFR selectable marker, e.g., as described in R. J. Kaufman and P. A. Sharp (1982) Mol. Biol. 159:601-621; and other knockout cells exemplified in Fan et al., Biotechnol Bioeng. 2012 Apr;109(4):1007-15], NS0 myeloma cells, COS cells, HEK293 cells, HKB11 cells, BHK21 cells, CAP cells, EB66 cells, and SP2 cells.

Expression might also be transient or semi-stable in expression systems such as HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 or CAP-T cells (for instance Durocher et al., Nucleic Acids Res. 2002 Jan 15;30(2):E9).

In some embodiments, the expression vector is designed such that the expressed protein is secreted into the culture medium in which the host cells are grown. The antibodies, antigen binding fragments thereof or variants thereof can be recovered from the culture medium using standard protein purification methods.

Antibodies of the invention or antigen-binding fragments thereof or variants thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography, mixed mode chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Antibodies of the present invention or antigen-binding fragments thereof or variants thereof include naturally purified products, products of chemical synthetic procedures, and products produced by recombinant techniques from an eukaryotic host, including, for example, yeast, higher plant, insect and mammalian cells. Depending upon the host employed in a recombinant production procedure, the antibody of the present invention can be glycosylated or can be non-glycosylated. Such methods are described in many standard laboratory manuals, such as Sambrook, supra, Sections 17.37-17.42; Ausubel, supra, Chapters 10, 12, 13, 16, 18 and 20.

In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or non-reducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

As used in the context of the present invention, the term "Ac225-Macropa-Trastuzumab", "Ac225-Trastuzumab-Macropa", "Ac225-Macropa-Trastuzumab emtansine" or "Ac225-Trastuzumab-Macropa-emtansine" refers to the following compound: 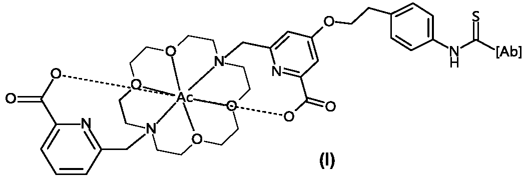 wherein [Ab] is the anti-HER2 monoclonal antibody Trastuzumab or an antigen-binding fragment thereof which is preferably bound to the rest of the molecule via a terminal amino group of a Lysine residue of the monoclonal antibody or antigen-binding fragment thereof.

In the context of the present invention, the term Trastuzumab or an antigen-binding fragment thereof refers to the monoclonal antibody Trastuzumab or an antigen-binding fragment thereof comprising at least the three CDR heavy chain sequences according to SEQ ID NO. 1, SEQ ID NO. 2 and SEQ ID NO. 3 and the three CDR light chain sequences according to SEQ ID NO. 4, SEQ ID NO. 5 and SEQ ID NO. 6.

Trastuzumab (HER2) CDR heavy chain sequences
SEQ ID NO. 1: DTYIH
SEQ ID NO. 2: RIYPTNGYTRYADSVKG
SEQ ID NO. 3: WGGDGFYAMDY

Trastuzumab (HER2) CDR light chain sequences
SEQ ID NO. 4: RASQDVNTAVA
SEQ ID NO. 5: SASFLYS
SEQ ID NO. 6: QQHYTTPPT

As used in the context of the present invention, the term "Ac225-Macropa-Labetuzumab" or "Ac225-Labetuzumab-Macropa" refers to the following compound: 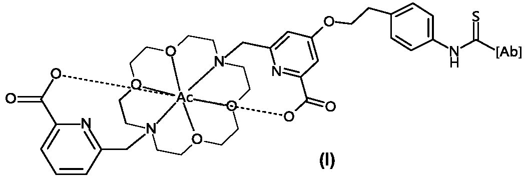 wherein [Ab] is the anti-CEACAM5 monoclonal antibody Labetuzumab or an antigen-binding fragment thereof which is preferably bound to the rest of the molecule via a terminal amino group of a Lysine residue of the monoclonal antibody or antigen-binding fragment thereof.

In the context of the present invention, the term Labetuzumab or an antigen-binding fragment thereof refers to the monoclonal antibody Labetuzumab or an antigen-binding fragment thereof comprising at least the three CDR heavy chain sequences according to SEQ ID NO. 7, SEQ ID NO. 8 and SEQ ID NO. 9 and the three CDR light chain sequences according to SEQ ID NO. 10, SEQ ID NO. 11 and SEQ ID NO. 12.

Labetuzumab (CEACAM5) CDR heavy chain sequences
SEQ ID NO. 7: TYWMS
SEQ ID NO. 8: EIHPDSSTINYAPSLKD
SEQ ID NO. 9: LYFGFPWFAY

Labetuzumab (CEACAM5) CDR light chain sequences
SEQ ID NO. 10: KASQDVGTSVA
SEQ ID NO. 11: WTSTRHT
SEQ ID NO. 12: QQYSLYRS

As used in the context of the present invention, the term "Ac225-Macropa-Sacituzumab" or "Ac225-Sacituzumab-Macropa" refers to the following compound: 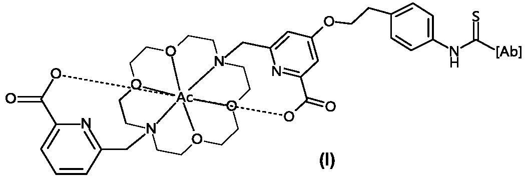 wherein [Ab] is the anti-TROP2 monoclonal antibody Sacituzumab or an antigen-binding fragment thereof which is preferably bound to the rest of the molecule via a terminal amino group of a Lysine residue of the monoclonal antibody or antigen-binding fragment thereof.

In the context of the present invention, the term Sacituzumab or an antigen-binding fragment thereof refers to the monoclonal antibody Sacituzumab or an antigen-binding fragment thereof comprising at least the three CDR heavy chain sequences according to SEQ ID NO. 13, SEQ ID NO. 14 and SEQ ID NO. 16 and the three CDR light chain sequences according to SEQ ID NO. 16, SEQ ID NO. 17 and SEQ ID NO. 18.

Sacituzumab, TROP2) CDR heavy chain sequences
SEQ ID NO. 13: NYGMN
SEQ ID NO. 14: WINTYTGEPTYTDDFKG
SEQ ID NO. 15: GGFGSSYWYFDV

Sacituzumab TROP2) CDR light chain sequences
SEQ ID NO. 16: KASQDVSIAVA
SEQ ID NO. 17: SASYRYT
SEQ ID NO. 18: QQHYITPLT

As used in the context of the present invention, the term "Ac225-Macropa-Indusatumab" or "Ac225-Indusatumab-Macropa" refers to the following compound: 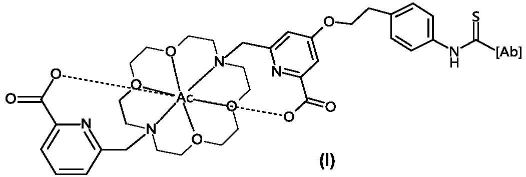 wherein [Ab] is the anti-GUCY2C monoclonal antibody Indusatumab or an antigen-binding fragment thereof which is preferably bound to the rest of the molecule via a terminal amino group of a Lysine residue of the monoclonal antibody or antigen-binding fragment thereof.

In the context of the present invention, the term Indusatumab or an antigen-binding fragment thereof refers to the monoclonal antibody Indusatumab or an antigen-binding fragment thereof comprising at least the three CDR heavy chain sequences according to SEQ ID NO. 19, SEQ ID NO. 20 and SEQ ID NO. 21 and the three CDR light chain sequences according to SEQ ID NO. 22, SEQ ID NO. 23 and SEQ ID NO. 24.

Indusatumab (5F9, GUCY2C) CDR heavy chain sequences
SEQ ID NO. 19: GYYWS
SEQ ID NO. 20: EINHRGNTNDNPSLKS
SEQ ID NO. 21: ERGYTYGNFDH

Indusatumab (5F9, GUCY2C) CDR light chain sequences
SEQ ID NO. 22: RASQSVSRNLA
SEQ ID NO. 23: GASTRAT
SEQ ID NO. 24: QQYKTWPRT

As used in the context of the present invention, the term "Ac225-Macropa-Telisotuzumab" or "Ac225-Telisotuzumab-Macropa" refers to the following compound: 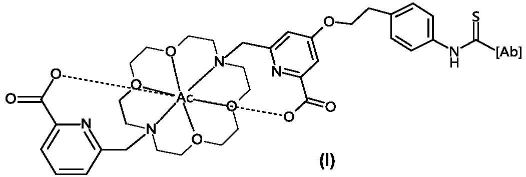 wherein [Ab] is the anti-cMet monoclonal antibody Telisotuzumab or an antigen-binding fragment thereof which is preferably bound to the rest of the molecule via a terminal amino group of a Lysine residue of the monoclonal antibody or antigen-binding fragment thereof.

In the context of the present invention, the term Telisotuzumab or an antigen-binding fragment thereof refers to the monoclonal antibody Telisotuzumab or an antigen-binding fragment thereof comprising at least the three CDR heavy chain sequences according to SEQ ID NO. 25, SEQ ID NO. 26 and SEQ ID NO. 27 and the three CDR light chain sequences according to SEQ ID NO. 28, SEQ ID NO. 29 and SEQ ID NO. 30.

Telisotuzumab (cMet) CDR heavy chain sequences
SEQ ID NO. 25: AYTMH
SEQ ID NO. 26: WIKPNNGLANYAQKFQG
SEQ ID NO. 27: SEITTEFDY

Telisotuzumab (cMet) CDR light chain sequences
SEQ ID NO. 28: KSSESVDSYANSFLH
SEQ ID NO. 29: RASTRES
SEQ ID NO. 30: QQSKEDPLT

As used in the context of the present invention, the term "Ac225-Macropa-Farletuzumab" or "Ac225-Farletuzumab-Macropa" refers to the following compound: 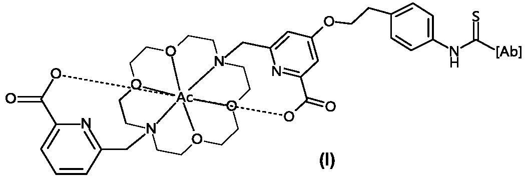 wherein [Ab] is the anti-FolRa monoclonal antibody Farletuzumab or an antigen-binding fragment thereof which is preferably bound to the rest of the molecule via a terminal amino group of a Lysine residue of the monoclonal antibody or antigen-binding fragment thereof.

In the context of the present invention, the term Farletuzumab or an antigen-binding fragment thereof refers to the monoclonal antibody Farletuzumab or an antigen-binding fragment thereof comprising at least the three CDR heavy chain sequences according to SEQ ID NO. 31, SEQ ID NO. 32 and SEQ ID NO. 33 and the three CDR light chain sequences according to SEQ ID NO. 34, SEQ ID NO. 35 and SEQ ID NO. 36.

Farletuzumab (FolRa) CDR heavy chain sequences
SEQ ID NO. 31: GYGLS
SEQ ID NO. 32: MISSGGSYTYYADSVKG
SEQ ID NO. 33: HGDDPAWFAY

Farletuzumab (FoIRa) CDR light chain sequences
SEQ ID NO. 34: SVSSSISSNNLH
SEQ ID NO. 35: GTSNLAS
SEQ ID NO. 36: QQWSSYPYMYT

As used in the context of the present invention, the term "Ac225-Macropa-Mirvetuximab" or "Ac225-Mirvetuximab-Macropa" refers to the following compound: 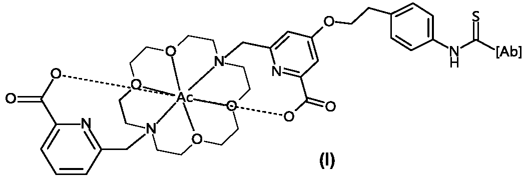 wherein [Ab] is the anti-FolRa monoclonal antibody Mirvetuximab or an antigen-binding fragment thereof which is preferably bound to the rest of the molecule via a terminal amino group of a Lysine residue of the monoclonal antibody or antigen-binding fragment thereof.

In the context of the present invention, the term Mirvetuximab or an antigen-binding fragment thereof refers to the monoclonal antibody Mirvetuximab or an antigen-binding fragment thereof comprising at least the three CDR heavy chain sequences according to SEQ ID NO. 37, SEQ ID NO. 38 and SEQ ID NO. 39 and the three CDR light chain sequences according to SEQ ID NO. 40, SEQ ID NO. 41 and SEQ ID NO. 42.

Mirvetuximab (FolRa) CDR heavy chain sequences
SEQ ID NO. 37: GYFMN
SEQ ID NO. 38: RIHPYDGDTFYNQKFQG
SEQ ID NO. 39: YDGSRAMDY

Mirvetuximab (FoIRa) CDR light chain sequences
SEQ ID NO. 40: KASQSVSFAGTSLMH
SEQ ID NO. 41: RASNLEA
SEQ ID NO. 42: QQSREYPYT

As used in the context of the present invention, the term "Ac225-Macropa-Rovalpituzumab" or "Ac225-Rovalpituzumab-Macropa" refers to the following compound: 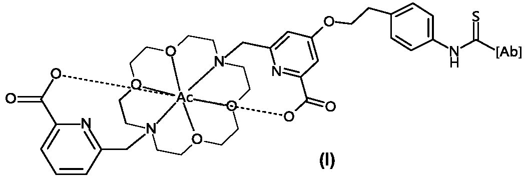 wherein [Ab] is the anti-DLL3 monoclonal antibody Rovalpituzumab or an antigen-binding fragment thereof which is preferably bound to the rest of the molecule via a terminal amino group of a Lysine residue of the monoclonal antibody or antigen-binding fragment thereof.

In the context of the present invention, the term Rovalpituzumab or an antigen-binding fragment thereof refers to the monoclonal antibody Rovalpituzumab or an antigen-binding fragment thereof comprising at least the three CDR heavy chain sequences according to SEQ ID NO. 43, SEQ ID NO. 44 and SEQ ID NO. 45 and the three CDR light chain sequences according to SEQ ID NO. 46, SEQ ID NO. 47 and SEQ ID NO. 48.

Rovalpituzumab (DLL3) CDR heavy chain sequences
SEQ ID NO. 43: NYGMN
SEQ ID NO. 44: WINTYTGEPTYADDFKG
SEQ ID NO. 45: IGDSSPSDY

Rovalpituzumab (DLL3) CDR light chain sequences
SEQ ID NO. 46: KASQSVSNDVV
SEQ ID NO. 47: YASNRYT
SEQ ID NO. 48: QQDYTSPWT

As used in the context of the present invention, the term "Ac225-Macropa-Enfortumab" or "Ac225-Enfortumab-Macropa" refers to the following compound: 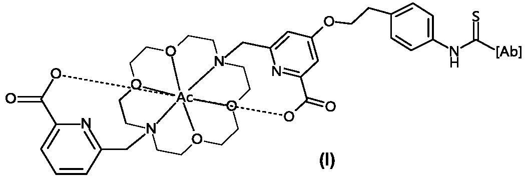 wherein [Ab] is the anti-Nectin-4 monoclonal antibody Enfortumab or an antigen-binding fragment thereof which is preferably bound to the rest of the molecule via a terminal amino group of a Lysine residue of the monoclonal antibody or antigen-binding fragment thereof.

In the context of the present invention, the term Enfortumab or an antigen-binding fragment thereof refers to the monoclonal antibody Enfortumab or an antigen-binding fragment thereof comprising at least the three CDR heavy chain sequences according to SEQ ID NO. 49, SEQ ID NO. 50 and SEQ ID NO. 51 and the three CDR light chain sequences according to SEQ ID NO. 52, SEQ ID NO. 53 and SEQ ID NO. 54.

Enfortumab (Nectin-4) CDR heavy chain sequences
SEQ ID NO. 40: SYNMN
SEQ ID NO. 50: YISSSSSTIYYADSVKG
SEQ ID NO. 51: AYYYGMDV

Enfortumab (Nectin-4) CDR light chain sequences
SEQ ID NO. 52: RASQGISGWLA
SEQ ID NO. 53: AASTLQS
SEQ ID NO. 54: QQANSFPPT

As used in the context of the present invention, the term "Ac225-Macropa-Vandortuzumab" or "Ac225-Vandortuzumab-Macropa" refers to the following compound: 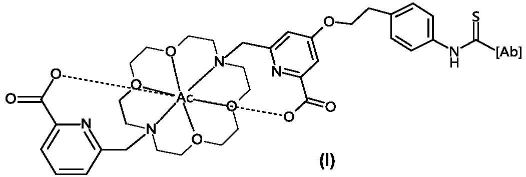 wherein [Ab] is the anti-STEAP1 monoclonal antibody Vandortuzumab or an antigen-binding fragment thereof which is preferably bound to the rest of the molecule via a terminal amino group of a Lysine residue of the monoclonal antibody or antigen-binding fragment thereof.

In the context of the present invention, the term Vandortuzumab or an antigen-binding fragment thereof refers to the monoclonal antibody Vandortuzumab or an antigen-binding fragment thereof comprising at least the three CDR heavy chain sequences according to SEQ ID NO. 55, SEQ ID NO. 56 and SEQ ID NO. 57 and the three CDR light chain sequences according to SEQ ID NO. 58, SEQ ID NO. 59 and SEQ ID NO. 60.

Vandortuzumab (STEAP1) CDR heavy chain sequences
SEQ ID NO. 55: SDYAWN
SEQ ID NO. 56: YISNSGSTSYNPSLKS
SEQ ID NO. 57: ERNYDYDDYYYAMDY

Vandortuzumab (STEAP1) CDR light chain sequences
SEQ ID NO. 58: KSSQSLLYRSNQKNYLA
SEQ ID NO. 59: WASTRES
SEQ ID NO. 60: QQYYNYPRT

As used in the context of the present invention, the term "Ac225-Macropa-Codrituzumab" or "Ac225-Codrituzumab-Macropa" refers to the following compound: 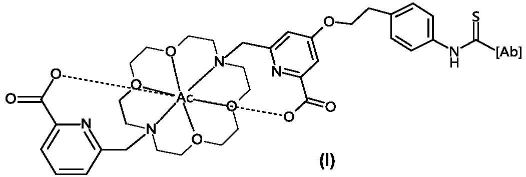 wherein [Ab] is the anti-GPC3 monoclonal antibody Codrituzumab or an antigen-binding fragment thereof which is preferably bound to the rest of the molecule via a terminal amino group of a Lysine residue of the monoclonal antibody or antigen-binding fragment thereof.

In the context of the present invention, the term Codrituzumab or an antigen-binding fragment thereof refers to the monoclonal antibody Codrituzumab or an antigen-binding fragment thereof comprising at least the three CDR heavy chain sequences according to SEQ ID NO. 61, SEQ ID NO. 62 and SEQ ID NO. 63 and the three CDR light chain sequences according to SEQ ID NO. 64, SEQ ID NO. 65 and SEQ ID NO. 66.

Codrituzumab CDR Heavy Chain Sequences
SEQ ID NO. 61: DYEMH
SEQ ID NO. 62: ALDPKTGDTAYSQKFKG
SEQ ID NO. 63: FYSYTY

Codrituzumab CDR Light Chain Sequences
SEQ ID NO. 64: RSSQSLVHSNRNTYLH
SEQ ID NO. 65: KVSNRFS
SEQ ID NO. 66: SQNTHVPPT

As used in the context of the present invention, the terms "Ac225-DOTA-Trastuzumab", "Ac225-DOTA-Codrituzumab", "Ac225-DOTA-Labetuzumab", "Ac225-DOTA-Sacituzumab", "Ac225-DOTA-Indusatumab", "Ac225-DOTA-Telisotuzumab", "Ac225-DOTA-Farletuzumab", "Ac225-DOTA-Mirvetuximab", "Ac225-DOTA-Rovalpituzumab", "Ac225-DOTA-Enfortumab" and "Ac225-DOTA-Vandortuzumab", refer to the following compounds: 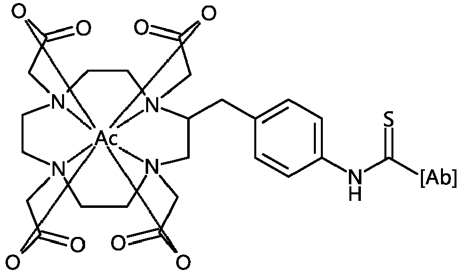 wherein [Ab] is the monoclonal antibody Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof and Vandortuzumab or an antigen-binding fragment thereof, respectively, which is preferably bound to the rest of the molecule via a terminal amino group of a Lysine residue of the monoclonal antibody or antigen-binding fragment thereof.

The present invention comprises combinations comprising a tissue-targeting compound of formula (I) as described herein and one or more further compounds having biological activity (i.e., a further pharmaceutical agent), preferably against cancer, especially bladder, brain, breast, colon, liver, lung, pancreatic, prostate, HNSCC and stomach cancers. These compounds are defined by their classes, thus allowing a skilled person to identify such compounds belonging to said class.

In the context of the present invention, the term "combination" means not only a dosage form which contains all the components (i.e., the tissue-targeting compound of formula (I) and a further pharmaceutical agent, also called fixed-dose combinations), and combination packs containing the components separate from one another, but also components which are administered simultaneously or sequentially, as long as they are employed for the prophylaxis or treatment of the same disease.

The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

### DESCRIPTION OF THE INVENTION

### Compounds

In accordance with a first aspect, the present invention relates to a tissue-targeting compound of formula (I) 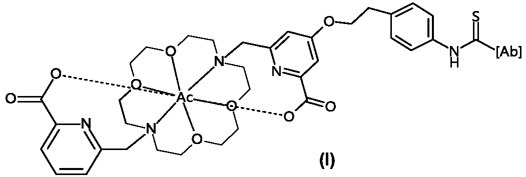 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1.

In a further embodiment, the present invention relates to a tissue-targeting compound of formula (I) 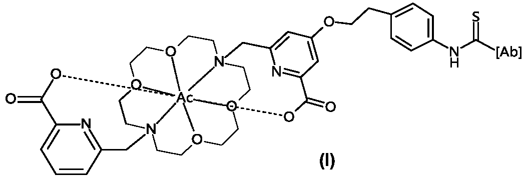 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1, wherein [Ab] is bound to the rest of the molecule via a terminal amino group of a Lysine residue of the monoclonal antibody or antigen-binding fragment thereof.

In a further embodiment, the present invention relates to a tissue-targeting compound of formula (I) 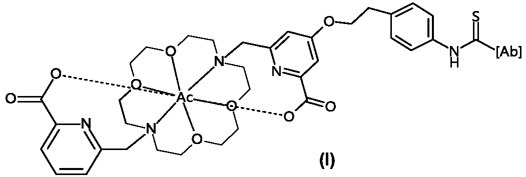 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1, preferably wherein the monoclonal antibody is Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof or Vandortuzumab or an antigen-binding fragment thereof.

In a further embodiment, the present invention relates to a tissue-targeting compound of formula (I) 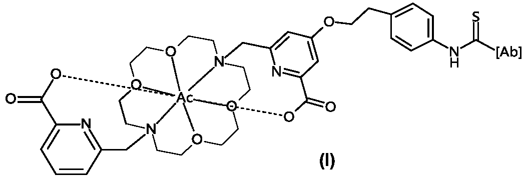 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1, wherein [Ab] is bound to the rest of the molecule via a terminal amino group of a Lysine residue of the monoclonal antibody or antigen-binding fragment thereof, preferably wherein the monoclonal antibody is Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof or Vandortuzumab or an antigen-binding fragment thereof.

In a further embodiment, the present invention relates to a tissue-targeting compound of formula (I) 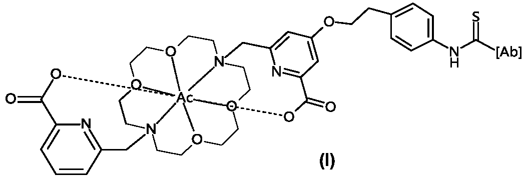 wherein [Ab] is bound to the rest of the molecule via a terminal amino group of a Lysine residue of the monoclonal antibody or antigen-binding fragment thereof and wherein [Ab] is Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof or Vandortuzumab or an antigen-binding fragment thereof.

In a further embodiment, the present invention relates to a tissue-targeting compound of formula (I) 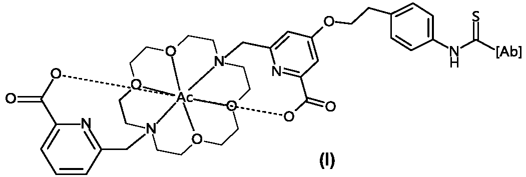 wherein [Ab] is Trastuzumab or an antigen-binding fragment thereof comprising at least the three CDR heavy chain sequences according to SEQ ID NO. 1, SEQ ID NO. 2 and SEQ ID NO. 3 and the three CDR light chain sequences according to SEQ ID NO. 4, SEQ ID NO. 5 and SEQ ID NO. 6., Labetuzumab or an antigen-binding fragment thereof comprising at least the three CDR heavy chain sequences according to SEQ ID NO. 7, SEQ ID NO. 8 and SEQ ID NO. 9 and the three CDR light chain sequences according to SEQ ID NO. 10, SEQ ID NO. 11 and SEQ ID NO. 12., Sacituzumab or an antigen-binding fragment thereof comprising at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18., Indusatumab or an antigen-binding fragment thereof comprising at least the three CDR heavy chain sequences according to SEQ ID NO. 19, SEQ ID NO. 20 and SEQ ID NO. 21 and the three CDR light chain sequences according to SEQ ID NO. 22, SEQ ID NO. 23 and SEQ ID NO. 24., Telisotuzumab or an antigen-binding fragment thereof comprising at least the three CDR heavy chain sequences according to SEQ ID NO. 25, SEQ ID NO. 26 and SEQ ID NO. 27 and the three CDR light chain sequences according to SEQ ID NO. 28, SEQ ID NO. 29 and SEQ ID NO. 30., Farletuzumab or an antigen-binding fragment thereof comprising at least the three CDR heavy chain sequences according to SEQ ID NO. 31, SEQ ID NO. 32 and SEQ ID NO. 33 and the three CDR light chain sequences according to SEQ ID NO. 34, SEQ ID NO. 35 and SEQ ID NO. 36., Mirvetuximab or an antigen-binding fragment thereof comprising at least the three CDR heavy chain sequences according to SEQ ID NO. 37, SEQ ID NO. 38 and SEQ ID NO. 39 and the three CDR light chain sequences according to SEQ ID NO. 40, SEQ ID NO. 41 and SEQ ID NO. 42., Rovalpituzumab or an antigen-binding fragment thereof comprising at least the three CDR heavy chain sequences according to SEQ ID NO. 43, SEQ ID NO. 44 and SEQ ID NO. 45 and the three CDR light chain sequences according to SEQ ID NO. 46, SEQ ID NO. 47 and SEQ ID NO. 48., Enfortumab or an antigen-binding fragment thereof comprising at least the three CDR heavy chain sequences according to SEQ ID NO. 49, SEQ ID NO. 50 and SEQ ID NO. 51 and the three CDR light chain sequences according to SEQ ID NO. 52, SEQ ID NO. 53 and SEQ ID NO. 54., Vandortuzumab or an antigen-binding fragment thereof comprising at least the three CDR heavy chain sequences according to SEQ ID NO. 55, SEQ ID NO. 56 and SEQ ID NO. 57 and the three CDR light chain sequences according to SEQ ID NO. 58, SEQ ID NO. 59 and SEQ ID NO. 60 or Codrituzumab or an antigen-binding fragment thereof comprising at least the three CDR heavy chain sequences according to SEQ ID NO. 61, SEQ ID NO. 62 and SEQ ID NO. 63 and the three CDR light chain sequences according to SEQ ID NO. 64, SEQ ID NO. 65 and SEQ ID NO. 66.

In a further embodiment, the present invention relates to a tissue-targeting compound of formula (I) 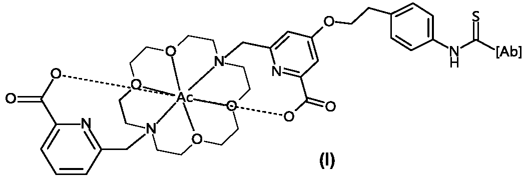 wherein [Ab] is a monoclonal antibody or an antigen-binding fragment thereof having binding affinity for HER2, preferably wherein the monoclonal antibody is Trastuzumab or an antigen-binding fragment thereof.

In a further embodiment, the present invention relates to a tissue-targeting compound of formula (I) 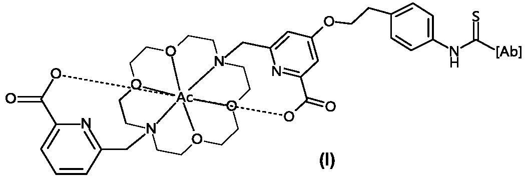 wherein [Ab] is a monoclonal antibody or an antigen-binding fragment thereof having binding affinity for HER2, preferably wherein the monoclonal antibody is Trastuzumab or an antigen-binding fragment thereof, wherein the monoclonal antibody or an antigen-binding fragment thereof having binding affinity for HER2 is bound to the rest of the molecule via a terminal amino group of a Lysine residue of the monoclonal antibody or antigen-binding fragment thereof.

In a further embodiment, the present invention relates to a tissue-targeting compound of formula (I) 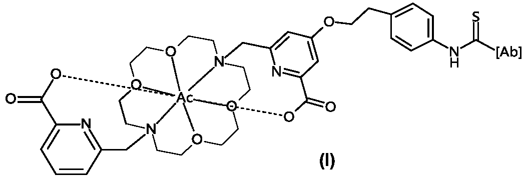 wherein [Ab] is the monoclonal antibody Trastuzumab or an antigen-binding fragment thereof comprising at least the three CDR heavy chain sequences according to SEQ ID NO. 1, SEQ ID NO. 2 and SEQ ID NO. 3 and the three CDR light chain sequences according to SEQ ID NO. 4, SEQ ID NO. 5 and SEQ ID NO. 6.

In a further embodiment, the present invention relates to a tissue-targeting compound of formula (I) 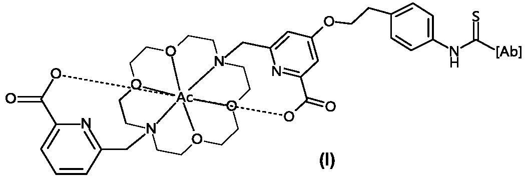 wherein [Ab] is a monoclonal antibody or an antigen-binding fragment thereof having binding affinity for CEACAM5, preferably wherein the monoclonal antibody is Labetuzumab or an antigen-binding fragment thereof.

In a further embodiment, the present invention relates to a tissue-targeting compound of formula (I) 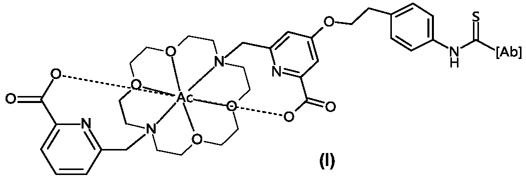 wherein [Ab] is a monoclonal antibody or an antigen-binding fragment thereof having binding affinity for CEACAM5, preferably wherein the monoclonal antibody is Labetuzumab or an antigen-binding fragment thereof, wherein the monoclonal antibody or an antigen-binding fragment thereof having binding affinity for CEACAM5 is bound to the rest of the molecule via a terminal amino group of a Lysine residue of the monoclonal antibody or antigen-binding fragment thereof.

In a further embodiment, the present invention relates to a tissue-targeting compound of formula (I) 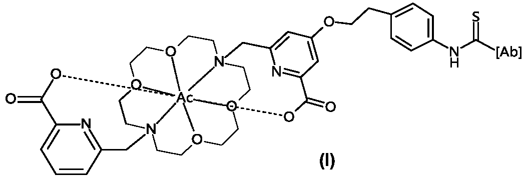 wherein [Ab] is the monoclonal antibody Labetuzumab or an antigen-binding fragment thereof comprising at least the three CDR heavy chain sequences according to SEQ ID NO. 7, SEQ ID NO. 8 and SEQ ID NO. 9 and the three CDR light chain sequences according to SEQ ID NO. 10, SEQ ID NO. 11 and SEQ ID NO. 12.

In a further embodiment, the present invention relates to a tissue-targeting compound of formula (I) 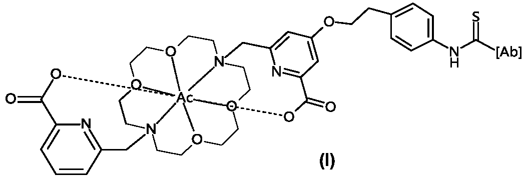 wherein [Ab] is a monoclonal antibody or an antigen-binding fragment thereof having binding affinity for TROP2, preferably wherein the monoclonal antibody is Sacituzumab or an antigen-binding fragment thereof.

In a further embodiment, the present invention relates to a tissue-targeting compound of formula (I) 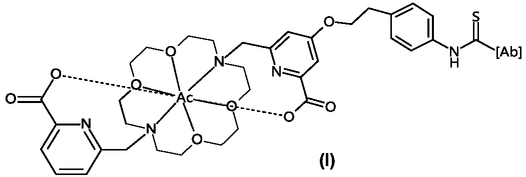 wherein [Ab] is a monoclonal antibody or an antigen-binding fragment thereof having binding affinity for TROP2, preferably wherein the monoclonal antibody is Sacituzumab or an antigen-binding fragment thereof, wherein the monoclonal antibody or an antigen-binding fragment thereof having binding affinity for TROP2 is bound to the rest of the molecule via a terminal amino group of a Lysine residue of the monoclonal antibody or antigen-binding fragment thereof.

In a further embodiment, the present invention relates to a tissue-targeting compound of formula (I) 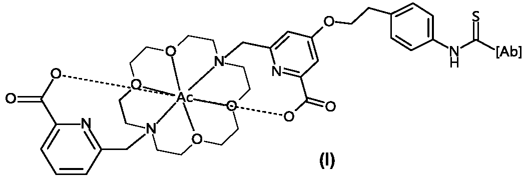 wherein [Ab] is the monoclonal antibody Sacituzumab or an antigen-binding fragment thereof comprising at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18.

In a further embodiment, the present invention relates to a tissue-targeting compound of formula (I) 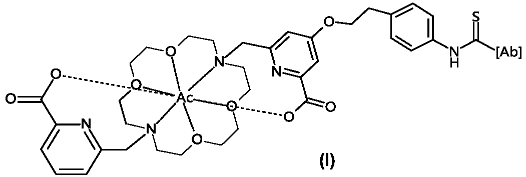 wherein [Ab] is a monoclonal antibody or an antigen-binding fragment thereof having binding affinity for GUCY2C, preferably wherein the monoclonal antibody is Indusatumab or an antigen-binding fragment thereof.

In a further embodiment, the present invention relates to a tissue-targeting compound of formula (I) 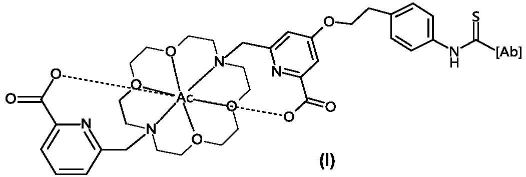 wherein [Ab] is a monoclonal antibody or an antigen-binding fragment thereof having binding affinity for GUCY2C, preferably wherein the monoclonal antibody is Indusatumab or an antigen-binding fragment thereof, wherein the monoclonal antibody or an antigen-binding fragment thereof having binding affinity for GUCY2C is bound to the rest of the molecule via a terminal amino group of a Lysine residue of the monoclonal antibody or antigen-binding fragment thereof.

In a further embodiment, the present invention relates to a tissue-targeting compound of formula (I) 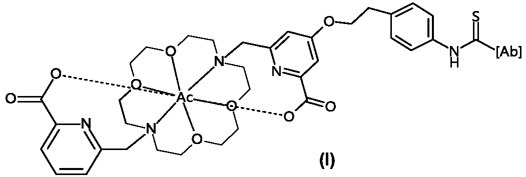 wherein [Ab] is the monoclonal antibody Indusatumab or an antigen-binding fragment thereof comprising at least the three CDR heavy chain sequences according to SEQ ID NO. 19, SEQ ID NO. 20 and SEQ ID NO. 21 and the three CDR light chain sequences according to SEQ ID NO. 22, SEQ ID NO. 23 and SEQ ID NO. 24.

In a further embodiment, the present invention relates to a tissue-targeting compound of formula (I) 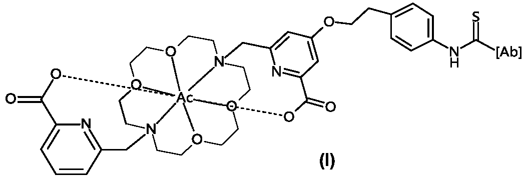 wherein [Ab] is a monoclonal antibody or an antigen-binding fragment thereof having binding affinity for cMet, preferably wherein the monoclonal antibody is Telisotuzumab or an antigen-binding fragment thereof.

In a further embodiment, the present invention relates to a tissue-targeting compound of formula (I) 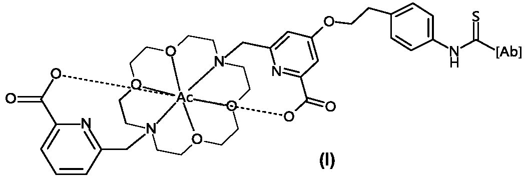 wherein [Ab] is a monoclonal antibody or an antigen-binding fragment thereof having binding affinity for cMet, preferably wherein the monoclonal antibody is Telisotuzumab or an antigen-binding fragment thereof, wherein the monoclonal antibody or an antigen-binding fragment thereof having binding affinity for cMet is bound to the rest of the molecule via a terminal amino group of a Lysine residue of the monoclonal antibody or antigen-binding fragment thereof.

In a further embodiment, the present invention relates to a tissue-targeting compound of formula (I) 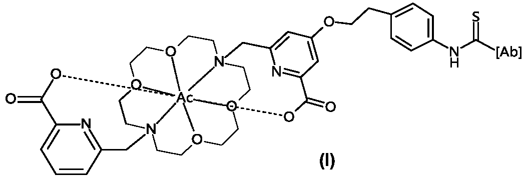 wherein [Ab] is the monoclonal antibody Telisotuzumab or an antigen-binding fragment thereof comprising at least the three CDR heavy chain sequences according to SEQ ID NO. 25, SEQ ID NO. 26 and SEQ ID NO. 27 and the three CDR light chain sequences according to SEQ ID NO. 28, SEQ ID NO. 29 and SEQ ID NO. 30.

In a further embodiment, the present invention relates to a tissue-targeting compound of formula (I) 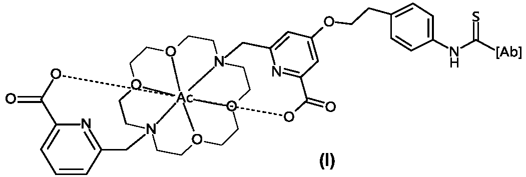 wherein [Ab] is a monoclonal antibody or an antigen-binding fragment thereof having binding affinity for FolRa, preferably wherein the monoclonal antibody is Farletuzumab or an antigen-binding fragment thereof.

In a further embodiment, the present invention relates to a tissue-targeting compound of formula (I) 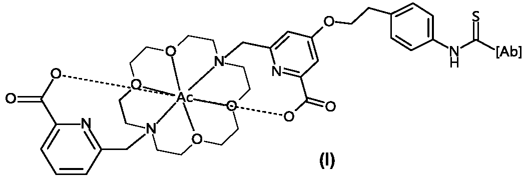 wherein [Ab] is a monoclonal antibody or an antigen-binding fragment thereof having binding affinity for FolRa, preferably wherein the monoclonal antibody is Farletuzumab or an antigen-binding fragment thereof, wherein the monoclonal antibody or an antigen-binding fragment thereof having binding affinity for FolRa is bound to the rest of the molecule via a terminal amino group of a Lysine residue of the monoclonal antibody or antigen-binding fragment thereof.

In a further embodiment, the present invention relates to a tissue-targeting compound of formula (I) 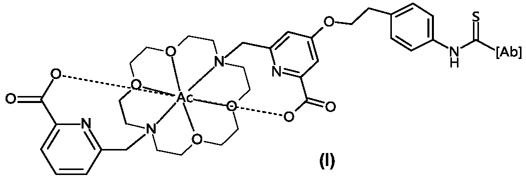 wherein [Ab] is the monoclonal antibody Farletuzumab or an antigen-binding fragment thereof comprising at least the three CDR heavy chain sequences according to SEQ ID NO. 31, SEQ ID NO. 32 and SEQ ID NO. 33 and the three CDR light chain sequences according to SEQ ID NO. 34, SEQ ID NO. 35 and SEQ ID NO. 36.

In a further embodiment, the present invention relates to a tissue-targeting compound of formula (I) 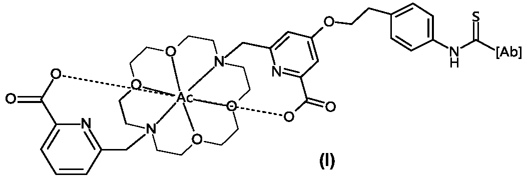 wherein [Ab] is a monoclonal antibody or an antigen-binding fragment thereof having binding affinity for FolRa, preferably wherein the monoclonal antibody is Mirvetuximab or an antigen-binding fragment thereof.

In a further embodiment, the present invention relates to a tissue-targeting compound of formula (I) 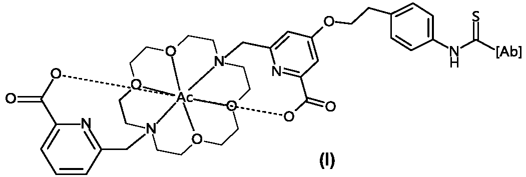 wherein [Ab] is a monoclonal antibody or an antigen-binding fragment thereof having binding affinity for FolRa, preferably wherein the monoclonal antibody is Mirvetuximab or an antigen-binding fragment thereof, wherein the monoclonal antibody or an antigen-binding fragment thereof having binding affinity for FolRa is bound to the rest of the molecule via a terminal amino group of a Lysine residue of the monoclonal antibody or antigen-binding fragment thereof.

In a further embodiment, the present invention relates to a tissue-targeting compound of formula (I) 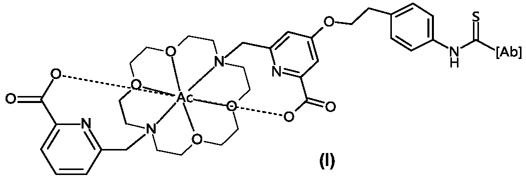 wherein [Ab] is the monoclonal antibody Mirvetuximab or an antigen-binding fragment thereof comprising at least the three CDR heavy chain sequences according to SEQ ID NO. 37, SEQ ID NO. 38 and SEQ ID NO. 39 and the three CDR light chain sequences according to SEQ ID NO. 40, SEQ ID NO. 41 and SEQ ID NO. 42.

In a further embodiment, the present invention relates to a tissue-targeting compound of formula (I) 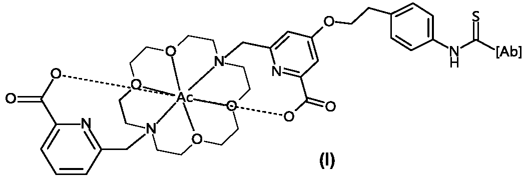 wherein [Ab] is a monoclonal antibody or an antigen-binding fragment thereof having binding affinity for DLL3, preferably wherein the monoclonal antibody is Rovalpituzumab or an antigen-binding fragment thereof.

In a further embodiment, the present invention relates to a tissue-targeting compound of formula (I) 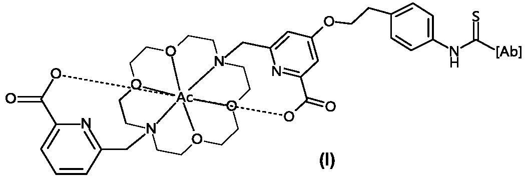 wherein [Ab] is a monoclonal antibody or an antigen-binding fragment thereof having binding affinity for DLL3, preferably wherein the monoclonal antibody is Rovalpituzumab or an antigen-binding fragment thereof, wherein the monoclonal antibody or an antigen-binding fragment thereof having binding affinity for DLL3 is bound to the rest of the molecule via a terminal amino group of a Lysine residue of the monoclonal antibody or antigen-binding fragment thereof.

In a further embodiment, the present invention relates to a tissue-targeting compound of formula (I) 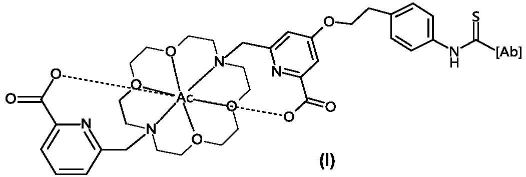 wherein [Ab] is the monoclonal antibody Rovalpituzumab or an antigen-binding fragment thereof comprising at least the three CDR heavy chain sequences according to SEQ ID NO. 43, SEQ ID NO. 44 and SEQ ID NO. 45 and the three CDR light chain sequences according to SEQ ID NO. 46, SEQ ID NO. 47 and SEQ ID NO. 48.

In a further embodiment, the present invention relates to a tissue-targeting compound of formula (I) 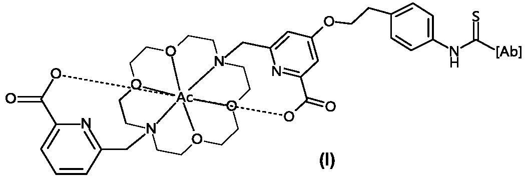 wherein [Ab] is a monoclonal antibody or an antigen-binding fragment thereof having binding affinity for Nectin-4, preferably wherein the monoclonal antibody is Enfortumab or an antigen-binding fragment thereof.

In a further embodiment, the present invention relates to a tissue-targeting compound of formula (I) 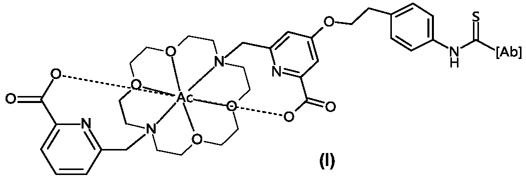 wherein [Ab] is a monoclonal antibody or an antigen-binding fragment thereof having binding affinity for Nectin-4, preferably wherein the monoclonal antibody is Enfortumab or an antigen-binding fragment thereof, wherein the monoclonal antibody or an antigen-binding fragment thereof having binding affinity for Nectin-4 is bound to the rest of the molecule via a terminal amino group of a Lysine residue of the monoclonal antibody or antigen-binding fragment thereof.

In a further embodiment, the present invention relates to a tissue-targeting compound of formula (I) 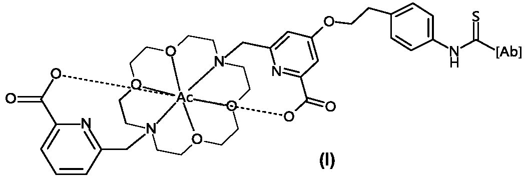 wherein [Ab] is the monoclonal antibody Enfortumab or an antigen-binding fragment thereof comprising at least the three CDR heavy chain sequences according to SEQ ID NO. 49, SEQ ID NO. 50 and SEQ ID NO. 51 and the three CDR light chain sequences according to SEQ ID NO. 52, SEQ ID NO. 53 and SEQ ID NO. 54.

In a further embodiment, the present invention relates to a tissue-targeting compound of formula (I) 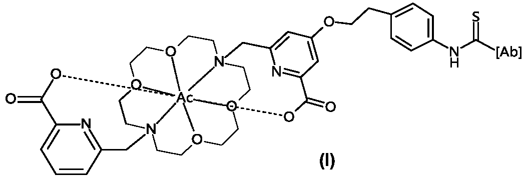 wherein [Ab] is a monoclonal antibody or an antigen-binding fragment thereof having binding affinity for STEAP1, preferably wherein the monoclonal antibody is Vandortuzumab or an antigen-binding fragment thereof.

In a further embodiment, the present invention relates to a tissue-targeting compound of formula (I) 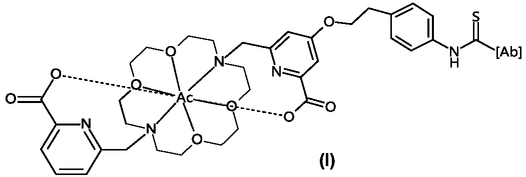 wherein [Ab] is a monoclonal antibody or an antigen-binding fragment thereof having binding affinity for STEAP1, preferably wherein the monoclonal antibody is Vandortuzumab or an antigen-binding fragment thereof, wherein the monoclonal antibody or an antigen-binding fragment thereof having binding affinity for STEAP1 is bound to the rest of the molecule via a terminal amino group of a Lysine residue of the monoclonal antibody or antigen-binding fragment thereof.

In a further embodiment, the present invention relates to a tissue-targeting compound of formula (I) 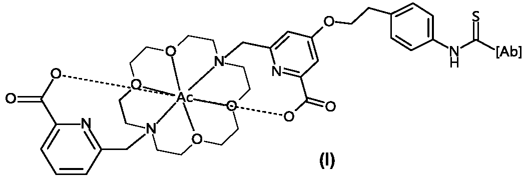 wherein [Ab] is the monoclonal antibody Vandortuzumab or an antigen-binding fragment thereof comprising at least the three CDR heavy chain sequences according to SEQ ID NO. 55, SEQ ID NO. 56 and SEQ ID NO. 57 and the three CDR light chain sequences according to SEQ ID NO. 58, SEQ ID NO. 59 and SEQ ID NO. 60.

In a further embodiment, the present invention relates to a tissue-targeting compound of formula (I) 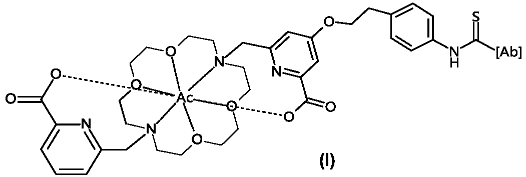 wherein [Ab] is a monoclonal antibody or an antigen-binding fragment thereof having binding affinity for GPC3, preferably wherein the monoclonal antibody is Codrituzumab or an antigen-binding fragment thereof.

In a further embodiment, the present invention relates to a tissue-targeting compound of formula (I) 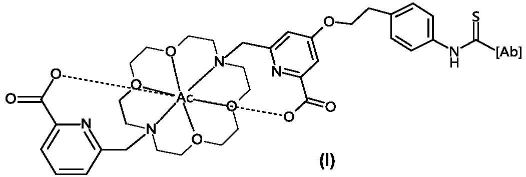 wherein [Ab] is a monoclonal antibody or an antigen-binding fragment thereof having binding affinity for GPC3, preferably wherein the monoclonal antibody is Codrituzumab or an antigen-binding fragment thereof, wherein the monoclonal antibody or an antigen-binding fragment thereof having binding affinity for GPC3 is bound to the rest of the molecule via a terminal amino group of a Lysine residue of the monoclonal antibody or antigen-binding fragment thereof.

In a further embodiment, the present invention relates to a tissue-targeting compound of formula (I) 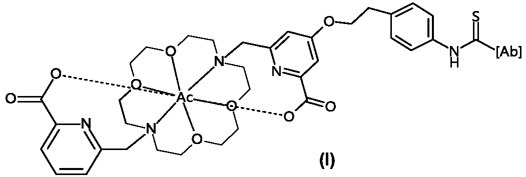 wherein [Ab] is the monoclonal antibody Codrituzumab or an antigen-binding fragment thereof comprising at least the three CDR heavy chain sequences according to SEQ ID NO. 61, SEQ ID NO. 62 and SEQ ID NO. 63 and the three CDR light chain sequences according to SEQ ID NO. 5864 SEQ ID NO. 65 and SEQ ID NO. 66.

The tissue-targeting compounds of formula (I) of the present invention comprise a chelating moiety capable of binding actinium. Preferably, the chelating moiety comprises an 18-membered chelating moiety. Preferably, the chelating moiety comprises the macrocyclic chelator N,N'-bis[(6-carboxy-2-pyridil)methyl]-4,13-diaza-18-crown-6 (macropa).

The macrocyclic chelator macropa has been described, for example, in Thiele et al., Angew. Chem. Int. Ed. 2017, 56(46), 14712 and references therein. Macropa chelators and derivatives thereof have also been described in WO2018/183906 and WO2020/106886 and references therein.

The actinium-complexating chelating moiety preferably terminates in an isothiocyanate or carboxylic acid moiety in order to facilitate its coupling with [Ab], i.e., the monoclonal antibody or an antigen-binding fragment thereof. When the macropa chelator is used, one of the pyridine rings of the macropa chelator is typically substituted so that it can be coupled to the [Ab], i.e., the monoclonal antibody or an antigen-binding fragment thereof. This can be a simple functional group (e.g., a carboxylic acid -COOH) or a more complex chemical structure, as long as it is capable of being coupled to [Ab], i.e., the monoclonal antibody or an antigen-binding fragment thereof. In particular, the substituent on the pyridine ring of the macropa chelator preferably terminates in an isothiocyanate or carboxylic acid moiety.

The [Ab], i.e., the monoclonal antibody or an antigen-binding fragment thereof is preferably coupled to the rest of the molecule forming the tissue-targeting compounds of the present invention through a Lysine residue of the antibody. Thus, the actinium-complexating chelating moiety and the [Ab], i.e., the monoclonal antibody or the antigen-binding fragment thereof can be coupled via an amide or thiourea moiety. When using the preferred macrocyclic chelator macropa, a terminal isothiocyanate or carboxylic acid moiety is coupled with a terminal amino group of a Lysine residue in the [Ab], i.e., the monoclonal antibody or an antigen-binding fragment thereof, leading to the formation of a thiourea or an amide group, respectively. Thus, the preferred actinium-complexating chelating moiety comprising a macropa chelator is attached to the [Ab], i.e., the monoclonal antibody or an antigen-binding fragment thereof via a thiourea or an amide bond derived from a terminal amino group of a Lysine residue of said anti-PSMA antibody or an antigen-binding fragment thereof.

Preferred antibodies or antigen-binding fragment thereof in the compounds of the present invention have binding affinity for HER2, GPC3, CEACAM5, TROP2, GUCY2C, cMet, FolRa, DLL3, Nectin-4 or STEAP1. Specific binding fragments such as Fab, Fab', F(ab')2 and single-chain specific binding antibodies are typical fragments. In particular, for HER2 it is preferred that the monoclonal antibody is Trastuzumab or an antigen-binding fragment thereof. In particular, for CEACAM5 it is preferred that the monoclonal antibody is Labetuzumab or an antigen-binding fragment thereof. In particular, for TROP2 it is preferred that the monoclonal antibody is Sacituzumab or an antigen-binding fragment thereof. In particular, for GUCY2C it is preferred that the monoclonal antibody is Indusatumab or an antigen-binding fragment thereof. In particular, for cMet it is preferred that the monoclonal antibody is Telisotuzumab or an antigen-binding fragment thereof. In particular, for FolRa it is preferred that the monoclonal antibody is Farletuzumab or an antigen-binding fragment thereof. In particular, for FolRa it is preferred that the monoclonal antibody is Mirvetuximab or an antigen-binding fragment thereof. In particular, for DLL3 it is preferred that the monoclonal antibody is Rovalpituzumab or an antigen-binding fragment thereof. In particular, for Nectin-4 it is preferred that the monoclonal antibody is Enfortumab or an antigen-binding fragment thereof. In particular, for STEAP1 it is preferred that the monoclonal antibody is Vandortuzumab or an antigen-binding fragment thereof.

Trastuzumab (CAS Registry Number 180288-69-1, INN published in WHO Recommended List 40, WHO Drug Information Vol 12, No 2, 1998) is a monoclonal antibody having binding affinity for HER2. Its preparation, isolation and purification has been described in e.g., WO2011015920A2.

Further, Trastuzumab comprises at least the three CDR heavy chain sequences according to SEQ ID NO. 1, SEQ ID NO. 2 and SEQ ID NO. 3 and the three CDR light chain sequences according to SEQ ID NO. 4, SEQ ID NO. 5 and SEQ ID NO. 6:
Trastuzumab (HER2) CDR heavy chain sequences
   SEQ ID NO. 1: DTYIH
   SEQ ID NO. 2: RIYPTNGYTRYADSVKG
   SEQ ID NO. 3: WGGDGFYAMDY
Trastuzumab (HER2) CDR light chain sequences
   SEQ ID NO. 4: RASQDVNTAVA
   SEQ ID NO. 5: SASFLYS
   SEQ ID NO. 6: QQHYTTPPT

Codrituzumab (CAS Registry Number 1365267-33-9, INN published in WHO Recommended List 71, WHO Drug Information, Vol. 28, No. 1, 2014), is a humanized monoclonal anti-GPC3 antibody. Its preparation, isolation and purification has been described in e.g., US7,919,086. Codrituzumab comprises a heavy chain protein sequence according to SEQ ID NO. 57 and a light chain protein sequence according to SEQ ID NO.58. Further, Codrituzumab comprises at least the three CDR heavy chain sequences according to SEQ ID NO. 42, SEQ ID NO. 43 and SEQ ID NO. 44 and the three CDR light chain sequences according to SEQ ID NO. 46, SEQ ID NO. 47 and SEQ ID NO. 48:
Codrituzumab CDR Heavy Chain Sequences
   SEQ ID NO. 42: DYEMH
   SEQ ID NO. 43: ALDPKTGDTAYSQKFKG
   SEQ ID NO. 44: FYSYTY
Codrituzumab CDR Light Chain Sequences
   SEQ ID NO. 46: RSSQSLVHSNRNTYLH
   SEQ ID NO. 47: KVSNRFS
   SEQ ID NO. 48: SQNTHVPPT

Labetuzumab (CAS Registry Number 219649-07-7, INN published in WHO Recommended List 47, WHO Drug Information, Vol. 16, No-1, 2002) is a monoclonal antibody having binding affinity for CEACAM5. Its preparation, isolation and purification has been described in e.g., Juweid et al., 1997, Prospects of Radioimmunotherapy in Epithelial Ovarian Cancer: Results with Iodine-131-Labeled Murine and Humanized MN-14 Anti-carcinoembryonic Antigen Monoclonal Antibodies, Gynecologic Oncology, Volume 67, Issue 3, Pages 259-271, ISSN 0090-8258, https://doi.org/10.1006/gyno.1997.4870.

Further, Labetuzumab comprises at least the three CDR heavy chain sequences according to SEQ ID NO. 7, SEQ ID NO. 8 and SEQ ID NO. 9 and the three CDR light chain sequences according to SEQ ID NO. 10, SEQ ID NO. 11 and SEQ ID NO. 12:
Labetuzumab (CEACAM5) CDR heavy chain sequences
   SEQ ID NO. 7: TYWMS
   SEQ ID NO. 8: EIHPDSSTINYAPSLKD
   SEQ ID NO. 9: LYFGFPWFAY
Labetuzumab (CEACAM5) CDR light chain sequences
   SEQ ID NO. 10: KASQDVGTSVA
   SEQ ID NO. 11: WTSTRHT
   SEQ ID NO. 12: QQYSLYRS

Sacituzumab (CAS Registry Number 1796566-95-4, INN published in WHO Proposed List 129, WHO Drug Information, Vol. 37, No. 2, 2023 as Sacituzumab Govitecan is a monoclonal antibody having binding affinity for TROP2. Its preparation, isolation and purification has been described in e.g., EP1483295B1.

Further, Sacituzumab comprises at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18:
Sacituzumab TROP2) CDR heavy chain sequences
   SEQ ID NO. 13: NYGMN
   SEQ ID NO. 14: WINTYTGEPTYTDDFKG
   SEQ ID NO. 15: GGFGSSYWYFDV
Sacituzumab TROP2) CDR light chain sequences
   SEQ ID NO. 16: KASQDVSIAVA
   SEQ ID NO. 17: SASYRYT
   SEQ ID NO. 18: QQHYITPLT

Indusatumab (CAS Registry Number 1514889-12-3, INN published in WHO Recommended List 112, WHO Drug Information, Vol. 28, No-4, 2014) is a monoclonal antibody having binding affinity for GUCY2C. Its preparation, isolation and purification has been described in e.g., Gallery et al., 2018, A monomethyl auristatin E-conjugated antibody to guanylyl cyclase C is cytotoxic to target-expressing cells in vitro and in vivo, PLOS ONE, Volume 13, Issue 1, e0191046, https://doi.org/10.1371/journal.pone.0191046.

Further, Indusatumab comprises at least the three CDR heavy chain sequences according to SEQ ID NO. 19, SEQ ID NO. 20 and SEQ ID NO. 21 and the three CDR light chain sequences according to SEQ ID NO. 22, SEQ ID NO. 23 and SEQ ID NO. 24:
Indusatumab (5F9, GUCY2C) CDR heavy chain sequences
   SEQ ID NO. 19: GYYWS
   SEQ ID NO. 20: EINHRGNTNDNPSLKS
   SEQ ID NO. 21: ERGYTYGNFDH
Indusatumab (5F9, GUCY2C) CDR light chain sequences
   SEQ ID NO. 22: RASQSVSRNLA
   SEQ ID NO. 23: GASTRAT
   SEQ ID NO. 24: QQYKTWPRT

Telisotuzumab (CAS Registry Number CAS 1781223-80-0, INN published in WHO Proposed List 155, WHO Drug Information, Vol. 30, No. 2, 2016) is a monoclonal antibody having binding affinity for cMet. Its preparation, isolation and purification has been described in e.g., J. Wang u. a., "Anti-c-Met monoclonal antibody ABT-700 breaks oncogene addiction in tumors with MET amplification", BMC Cancer, Bd. 16, Nr. 1, S. 105, Dez. 2016, doi: 10.1186/s12885-016-2138-z.

Further, Telisotuzumab comprises at least the three CDR heavy chain sequences according to SEQ ID NO. 25, SEQ ID NO. 26 and SEQ ID NO. 27 and the three CDR light chain sequences according to SEQ ID NO. 28, SEQ ID NO. 29 and SEQ ID NO. 30:
Telisotuzumab (cMet) CDR heavy chain sequences
   SEQ ID NO. 25: AYTMH
   SEQ ID NO. 26: WIKPNNGLANYAQKFQG
   SEQ ID NO. 27: SEITTEFDY
Telisotuzumab (cMet) CDR light chain sequences
   SEQ ID NO. 28: KSSESVDSYANSFLH
   SEQ ID NO. 29: RASTRES
   SEQ ID NO. 30: QQSKEDPLT

Farletuzumab (CAS Registry Number 896723-44-7, INN published in WHO Proposed List 100, WHO Drug Information, Vol. 22, No. 4, 2008) is a monoclonal antibody having binding affinity for FolRa. Its preparation, isolation and purification has been described in Ebel et al. Cancer Immunity 2007 Mar 9;7:6. PMID: 17346028; PMCID: PMC2935753).

Further, Farletuzumab comprises at least the three CDR heavy chain sequences according to SEQ ID NO. 31, SEQ ID NO. 32 and SEQ ID NO. 33 and the three CDR light chain sequences according to SEQ ID NO. 34, SEQ ID NO. 35 and SEQ ID NO. 36:
Farletuzumab (FolRa) CDR heavy chain sequences
   SEQ ID NO. 31: GYGLS
   SEQ ID NO. 32: MISSGGSYTYYADSVKG
   SEQ ID NO. 33: HGDDPAWFAY
Farletuzumab (FolRa) CDR light chain sequences
   SEQ ID NO. 34: SVSSSISSNNLH
   SEQ ID NO. 35: GTSNLAS
   SEQ ID NO. 36: QQWSSYPYMYT

Mirvetuximab (CAS Registry Number 1453084-36-0, INN published in WHO Proposed List 114, WHO Drug Information, Vol. 29, No. 4, 2015) is a monoclonal antibody having binding affinity for FolRa. Its preparation, isolation and purification has been described in e.g., Ab et al Molecular Cancer Therapeutics 2015 (PMID: 25904506).

Further, Mirvetuximab comprises at least the three CDR heavy chain sequences according to SEQ ID NO. 37, SEQ ID NO. 38 and SEQ ID NO. 39 and the three CDR light chain sequences according to SEQ ID NO. 40, SEQ ID NO. 41 and SEQ ID NO. 42:
Mirvetuximab (FolRa) CDR heavy chain sequences
   SEQ ID NO. 37: GYFMN
   SEQ ID NO. 38: RIHPYDGDTFYNQKFQG
   SEQ ID NO. 39: YDGSRAMDY
Mirvetuximab (FolRa) CDR light chain sequences
   SEQ ID NO. 40: KASQSVSFAGTSLMH
   SEQ ID NO. 41: RASNLEA
   SEQ ID NO. 42: QQSREYPYT

Rovalpituzumab (CAS Registry Number 1613313-01-1, INN (Rovalpituzumab Tesirine: 10141) published in WHO Proposed List 113, WHO Drug Information, Vol. 29, No. 2, 2015) is a monoclonal antibody having binding affinity for DLL3. Its preparation, isolation and purification has been described in e.g., Lashari BH, Vallatharasu Y, Kolandra L, Hamid M, Uprety D. Rovalpituzumab Tesirine: A Novel DLL3-Targeting Antibody-Drug Conjugate. Drugs R D. 2018 Dec;18(4):255-258. doi: 10.1007/s40268-018-0247-7. PMID: 30232719; PMCID: PMC6277321.

Further, Rovalpituzumab comprises at least the three CDR heavy chain sequences according to SEQ ID NO. 43, SEQ ID NO. 44 and SEQ ID NO. 45 and the three CDR light chain sequences according to SEQ ID NO. 46, SEQ ID NO. 47 and SEQ ID NO. 48:
Rovalpituzumab (DLL3) CDR heavy chain sequences
   SEQ ID NO. 43: NYGMN
   SEQ ID NO. 44: WINTYTGEPTYADDFKG
   SEQ ID NO. 45: IGDSSPSDY
Rovalpituzumab (DLL3) CDR light chain sequences
   SEQ ID NO. 46: KASQSVSNDVV
   SEQ ID NO. 47: YASNRYT
   SEQ ID NO. 48: QQDYTSPWT

Enfortumab (CAS Registry Number 1448664-46-7, INN 9821 published in WHO Recommended List 71. WHO Drug Information, Vol. 28, No. 1, 2014 as enfortumab vedotin) is a monoclonal antibody having binding affinity for Nectin-4. Its preparation, isolation and purification has been described in e.g., Halford Z, Anderson MK, Clark MD. Enfortumab Vedotin-ejfv: A First-in-Class Anti-Nectin-4 Antibody-Drug Conjugate for the Management of Urothelial Carcinoma. Ann Pharmacother. 2021 Jun;55(6):772-782. doi: 10.1177/1060028020960402. Epub 2020 Sep 18. PMID: 32945172.

Further, Enfortumab comprises at least the three CDR heavy chain sequences according to SEQ ID NO. 49, SEQ ID NO. 50 and SEQ ID NO. 51 and the three CDR light chain sequences according to SEQ ID NO. 52, SEQ ID NO. 53 and SEQ ID NO. 54:
Enfortumab (Nectin-4) CDR heavy chain sequences
   SEQ ID NO. 49: SYNMN
   SEQ ID NO. 50: YISSSSSTIYYADSVKG
   SEQ ID NO. 51: AYYYGMDV
Enfortumab (Nectin-4) CDR light chain sequences
   SEQ ID NO. 52: RASQGISGWLA
   SEQ ID NO. 53: AASTLQS
   SEQ ID NO. 54: QQANSFPPT

Vandortuzumab (CAS Registry Number 1557989-52-2, INN published in WHO Recommended List 74, WHO Drug Information, Vol. 29, No. 3, 2015 as vandortuzumab vedotin) is a monoclonal antibody having binding affinity for STEAP1. Its preparation, isolation and purification has been described in e.g., Danila DC, et al., Phase I Study of DSTP3086S, an Antibody-Drug Conjugate Targeting Six-Transmembrane Epithelial Antigen of Prostate 1, in Metastatic Castration-Resistant Prostate Cancer. J Clin Oncol. 2019 Dec 20;37(36):3518-3527. doi: 10.1200/JCO.19.00646. Epub 2019 Nov 5. PMID: 31689155; PMCID: PMC7351321.

Further, Vandortuzumab comprises at least the three CDR heavy chain sequences according to SEQ ID NO. 55, SEQ ID NO. 56 and SEQ ID NO. 57 and the three CDR light chain sequences according to SEQ ID NO. 58, SEQ ID NO. 59 and SEQ ID NO. 60:
Vandortuzumab (STEAP1) CDR heavy chain sequences
   SEQ ID NO. 55: SDYAWN
   SEQ ID NO. 56: YISNSGSTSYNPSLKS
   SEQ ID NO. 57: ERNYDYDDYYYAMDY
Vandortuzumab (STEAP1) CDR light chain sequences
   SEQ ID NO. 58: KSSQSLLYRSNQKNYLA
   SEQ ID NO. 59: WASTRES
   SEQ ID NO. 60: QQYYNYPRT

### Combinations

In a further aspect, the present invention relates to combinations, preferably pharmaceutical combinations comprising a tissue-targeting compound of formula (I) as described above and a further pharmaceutical agent. Said combinations are useful in therapy, preferably in treating hyperproliferative diseases such as cancer.

Combinations comprising effective amounts of a tissue-targeting compound of formula (I) as described above and a further pharmaceutical agent achieve a greater therapeutic efficacy than when either compound is used alone.

The relative ratios of each compound in the combination can also be selected based on their respective mechanisms of action and the disease biology. The relative ratios of each compound can vary widely.

The release of one or more agents of the combination can also be controlled, where appropriate, to provide the desired therapeutic activity when in a single dosage form, combination pack, kit or when in separate independent dosage forms.

Generally, the combinations of the present invention will serve to:
(1) yield better efficacy in reducing the growth of a tumor or even eliminate the tumor as compared to administration of either agent alone,
(2) provide for the administration of lesser amounts of the administered targeted compounds and/or chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time until tumor progression, and/or
(8) yield efficacy and tolerability results at least as good as those of the agents used alone, compared to known instances where other cancer agent combinations produce inhibitory effects.

The tissue-targeting compound of formula (I) is a compound of the present invention as described herein.

### Synthesis

In a further aspect, the present invention relates to a process for the preparation of a tissue-targeting compound of formula (I) 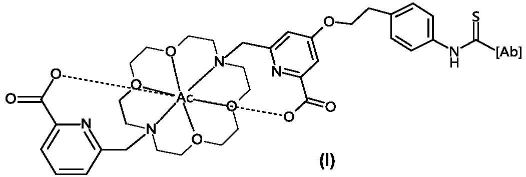
wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1,
said method comprising
   (i) coupling a chelating moiety of formula (II) 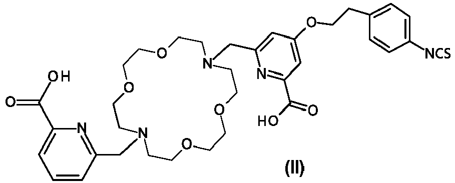 with a monoclonal antibody or an antigen-binding fragment thereof having binding affinity for HER2, GPC3, CEACAM5, TROP2, GUCY2C, cMet, FolRa, DLL3, Nectin-4 or STEAP1, and
   (ii) contacting the resulting product with an aqueous solution comprising an ion of at least one alpha-emitting actinium isotope.

In a further embodiment, the present invention relates to a process for the preparation of a tissue-targeting compound of formula (I) 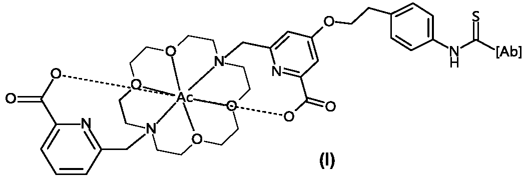
wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1,
said method comprising
   (i) coupling a chelating moiety of formula (II) 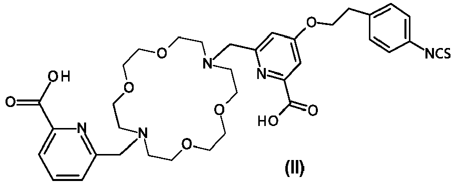 with a monoclonal antibody or an antigen-binding fragment thereof having binding affinity for HER2, GPC3, CEACAM5, TROP2, GUCY2C, cMet, FolRa, DLL3, Nectin-4 or STEAP1 via a Lysine residue of the monoclonal antibody or antigen-binding fragment thereof, and
   (ii) contacting the resulting product with an aqueous solution comprising an ion of at least one alpha-emitting actinium isotope.

In a further embodiment, the present invention relates to a process for the preparation of a tissue-targeting compound of formula (I) 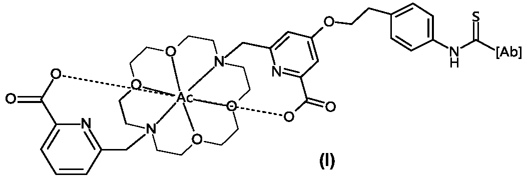 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1, preferably wherein the monoclonal antibody is selected from Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof and Vandortuzumab or an antigen-binding fragment thereof,
said method comprising
(i) coupling a chelating moiety of formula (II) 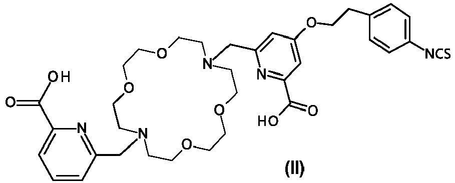 with a monoclonal antibody or an antigen-binding fragment thereof having binding affinity for HER2, GPC3, CEACAM5, TROP2, GUCY2C, cMet, FolRa, DLL3, Nectin-4 or STEAP1, preferably wherein the monoclonal antibody is selected from Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof and Vandortuzumab or an antigen-binding fragment thereof via a Lysine residue of the antibody or antigen-binding fragment thereof, and
(ii) contacting the resulting product with an aqueous solution comprising an ion of at least one alpha-emitting actinium isotope.

In a further embodiment, the present invention relates to a process for the preparation of a tissue-targeting compound of formula (I) 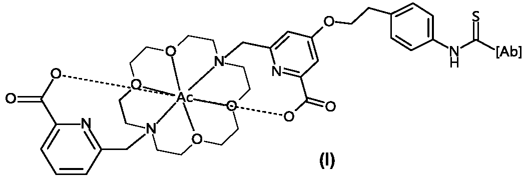 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1, preferably wherein the monoclonal antibody is selected from Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof and Vandortuzumab or an antigen-binding fragment thereof,
said method comprising
(i) coupling a chelating moiety of formula (II) 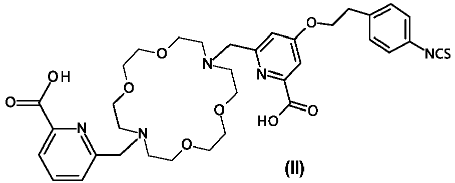 with a monoclonal antibody or an antigen-binding fragment thereof having binding affinity for HER2, GPC3, CEACAM5, TROP2, GUCY2C, cMet, FolRa, DLL3, Nectin-4 or STEAP1, preferably wherein the monoclonal antibody is selected from Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof and Vandortuzumab or an antigen-binding fragment thereof via a Lysine residue of the monoclonal antibody or antigen-binding fragment thereof, and
(ii) contacting the resulting product with an aqueous solution comprising an ion of at least one alpha-emitting actinium isotope.

In step (i), a chelating moiety of formula (II) 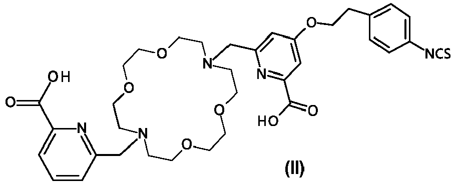 is coupled with a monoclonal antibody or an antigen-binding fragment thereof having binding affinity for for HER2, GPC3, CEACAM5, TROP2, GUCY2C, cMet, FolRa, DLL3, Nectin-4 or STEAP1. Preferably, the monoclonal antibody or antigen-binding fragment thereof is selected from Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof or Vandortuzumab or an antigen-binding fragment thereof.

Preferably, the chelating moiety of formula (II) is coupled to the monoclonal antibody or an antigen-binding fragment thereof having binding affinity for for HER2, GPC3, CEACAM5, TROP2, GUCY2C, cMet, FolRa, DLL3, Nectin-4 or STEAP1 via a Lysine residue of the monoclonal antibody, thus forming a thiourea moiety that links the chelating moiety with the antibody or antigen-binding fragment thereof.

Preferably, the coupling in step (i) is carried out under basic pH, i.e., at a pH higher than 7. More preferably, the coupling in step (i) is carried out at a pH of from 8 to 10, most preferably at a pH of 9.

After step (i), a tissue-targeting chelator of formula (III) is generated. Thus, in further embodiments, the present invention relates to a tissue-targeting chelator of formula (III) 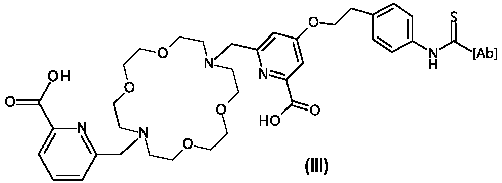 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1.

Preferably, the monoclonal antibody or antigen-binding fragment thereof having binding affinity for HER2, GPC3, CEACAM5, TROP2, GUCY2C, cMet, FolRa, DLL3, Nectin-4 or STEAP1 is selected from Trastuzumab or an antigen-binding fragment thereof, Codrituzumab or an antigen-binding fragment thereof, Labetuzumab or an antigen-binding fragment thereof, Sacituzumab or an antigen-binding fragment thereof, Indusatumab or an antigen-binding fragment thereof, Telisotuzumab or an antigen-binding fragment thereof, Farletuzumab or an antigen-binding fragment thereof, Mirvetuximab or an antigen-binding fragment thereof, Rovalpituzumab or an antigen-binding fragment thereof, Enfortumab or an antigen-binding fragment thereof and Vandortuzumab or an antigen-binding fragment thereof.

In step (ii), the product resulting from the coupling in step (i) is contacted with an aqueous solution comprising an ion of at least one alpha-emitting actinium isotope. Preferably, the alpha-emitting actinium isotope is 225Ac. Preferably, the ion of at least one alpha-emitting actinium isotope is ²²⁵Ac having a triple positive charge, i.e., ²²⁵Ac³⁺. The present invention allows for the execution of step (ii) at room temperature, i.e., at a temperature that is lower than the one required for the preparation of the corresponding DOTA conjugates (i.e. Ac225-DOTA-[Ab]), which require higher temperatures (60°C).

### Uses

In a further aspect, the tissue-targeting compounds of formula (I) of the present invention and/or the combinations comprising tissue-targeting compounds of formula (I) and a further pharmaceutical agent of the present invention may be used for the treatment of disease in a human or in a non-human animal subject. In particular, the tissue-targeting compounds of formula (I) of the present invention and/or the combinations comprising tissue-targeting compounds of formula (I) and a further pharmaceutical agent of the present invention may be used for the treatment of hyperplastic and/or neoplastic diseases, such as cancer. There are a number of targets which are known to be associated with hyperplastic and neoplastic disease. These include certain receptors, cell surface proteins, transmembrane proteins and proteins/peptides found in the extracellular matrix in the vicinity of diseased cells. Examples of cell-surface receptors and antigens which may be associated with neoplastic disease include but are not limited to CD22, CD33, FGFR2 (CD332), PSMA, HER2, GPC3 and Mesothelin. In one preferred embodiment, the tissue-targeting compounds of formula (I) of the present invention and/or the combinations comprising tissue-targeting compounds of formula (I) of the present invention comprise a tissue-targeting moiety comprising a monoclonal antibody having specificity and/or binding affinity for HER2, GPC3, CEACAM5, TROP2, GUCY2C, cMet, FolRa, DLL3, Nectin-4 or STEAP1. This may be reflected, for example by having 50 or more times greater binding affinity for cells expressing HER2, GPC3, CEACAM5, TROP2, GUCY2C, cMet, FolRa, DLL3, Nectin-4 or STEAP1, than for non-expressing cells (preferably at least 100 time greater, more preferably at least 300 times greater).

The various aspects of the invention as described herein relate to treatment of disease, particularly for the selective targeting of diseased tissue, as well as relating to complexes, conjugates, medicaments, formulation, kits etc. useful in such methods. In all aspects, the diseased tissue may reside at a single site in the body (for example in the case of a localized solid tumor) or may reside at a plurality of sites (for example where several joints are affected in arthritis or in the case of a distributed or metastasized cancerous disease).

The diseased tissue to be targeted may be at a soft tissue site, at a calcified tissue site or a plurality of sites which may all be in soft tissue, all in calcified tissue or may include at least one soft tissue site and/or at least one calcified tissue site. In one embodiment, at least one soft tissue site is targeted. The sites of targeting and the sites of origin of the disease may be the same, but alternatively may be different (such as where metastatic sites are specifically targeted). Where more than one site is involved, this may include the site of origin or may be a plurality of secondary sites.

The term "soft tissue" is used herein to indicate tissues which do not have a "hard" mineralized matrix. In particular, soft tissues as used herein may be any tissues that are not skeletal tissues. Correspondingly, "soft tissue disease" as used herein indicates a disease occurring in a "soft tissue" as used herein. The invention is particularly suitable for the treatment of cancers and "soft tissue disease" thus encompasses carcinomas, sarcomas, myelomas, leukemias, lymphomas and mixed type cancers occurring in any "soft" (i.e. non-mineralized) tissue, as well as other non-cancerous diseases of such tissue. Cancerous "soft tissue disease" includes solid tumors occurring in soft tissues as well as metastatic and micro-metastatic tumors. Indeed, the soft tissue disease may comprise a primary solid tumor of soft tissue and at least one metastatic tumor of soft tissue in the same patient. Alternatively, the "soft tissue disease" may consist of only a primary tumor or only metastases with the primary tumor being a skeletal disease. Particularly suitable for treatment and/or targeting in all appropriate aspects of the invention are hematological neoplasms and especially neoplastic diseases of lymphoid cells, such as lymphomas and lymphoid leukemias, including Non-Hodgkin's Lymphoma, B-cell neoplasms of B-cell lymphomas. Similarly, any neoplastic diseases of bone marrow, spine (especially spinal cord) lymph nodes and/or blood cells are suitable for treatment and/or targeting in all appropriate aspects of the invention.

Some examples of B-cell diseases (e.g. neoplasms) that are suitable for treatment and/or targeting in appropriate aspects of the present invention include:
Chronic lymphocytic leukemia/Small lymphocytic lymphoma, B-cell prolymphocytic leukemia, Lymphoplasmacytic lymphoma (such as Waldenström macroglobulinemia), Splenic marginal zone lymphoma, Plasma cell neoplasms (e.g. Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma and Burkitt lymphoma/leukemia.

The tissue-targeting compounds of formula (I) of the present invention and/or the combinations comprising tissue-targeting compounds of formula (I) and a further pharmaceutical agent of the present invention preferably comprise a tissue-targeting moiety comprising a monoclonal antibody or an antigen-binding fragment thereof having binding affinity for HER2, GPC3, CEACAM5, TROP2, GUCY2C, cMet, FolRa, DLL3, Nectin-4 or STEAP1.

Examples of diseases and/or neoplasms suitable for treatment using the preferred tissue-targeting compounds of formula (I) of the present invention and/or the combinations comprising tissue-targeting compounds of formula (I) of the present invention include prostate cancer, salivary gland cancer, glioblastoma, brain cancer, thyroid cancer, hepatocellular carcinoma and clear cell renal carcinoma. Preferably, examples of diseases and/or neoplasms suitable for treatment using the preferred tissue-targeting compounds of formula (I) of the present invention and/or the combinations comprising tissue-targeting compounds of formula (I) of the present invention include bladder, brain, breast, colon, liver, lung, pancreatic, prostate, HNSCC and stomach cancers, which are currently among the most prevalent types of cancer.

In some embodiments, the present invention includes a method of using a tissue-targeting compound of formula (I) and/or a combination comprising a tissue-targeting compound of formula (I) and a further pharmaceutical agent of the present invention for the treatment of diseases, more preferably hyperproliferative diseases, more particularly wherein the hyperproliferative disease is cancer, and yet even more particularly wherein the cancer disease is bladder, brain, breast, colon, liver, lung, pancreatic, prostate, HNSCC or stomach cancer. In some embodiments, the present invention includes a method of using a tissue-targeting compound of formula (I) and/or a combination comprising a tissue-targeting compound of formula (I) and a further pharmaceutical agent of the present invention for the treatment of diseases, preferably hyperproliferative diseases, more preferably cancer, even more preferably bladder, brain, breast, colon, liver, lung, pancreatic, prostate, HNSCC or stomach cancer.

In some embodiments, the present invention includes a method of treating a hyperproliferative disease, more particularly cancer, more particularly bladder, brain, breast, colon, liver, lung, pancreatic, prostate, HNSCC or stomach cancer, said method comprising administering an effective amount of at least one tissue-targeting compound of formula (I) and/or a combination comprising a tissue-targeting compound of formula (I) and a further pharmaceutical agent of the present invention to a subject in need thereof.

In some embodiments, the present invention provides a tissue-targeting compound of formula (I), as described *supra,* and/or a combination comprising a tissue-targeting compound of formula (I) and a further pharmaceutical agent of the present invention as described *supra,* for use in the treatment and/or prophylaxis of diseases, in particular hyperproliferative disorders, more particularly cancer, even more particularly wherein the cancer disease is bladder, brain, breast, colon, liver, lung, pancreatic, prostate, HNSCC or stomach cancer.

In some embodiments, the present invention includes a tissue-targeting compound of formula (I) and/or a combination comprising a tissue-targeting compound of formula (I) and a further pharmaceutical agent of the present invention for use in a method of inhibiting proliferation of a cell and/or the induction of apoptosis in a cell, comprising contacting the cell with a tissue-targeting compound of formula (I) and/or with a combination comprising a tissue-targeting compound of formula (I) and a further pharmaceutical agent.

In some embodiments, the present invention includes a tissue-targeting compound of formula (I) and/or a combination comprising a tissue-targeting compound of formula (I) and a further pharmaceutical agent for use in a method of treating a hyperproliferative disease, more particularly wherein the hyperproliferative disease is cancer, and yet even more particularly wherein the cancer disease is bladder, brain, breast, colon, liver, lung, pancreatic, prostate, HNSCC or stomach cancer.

In some embodiments, the present invention includes the use of a tissue-targeting compound of formula (I) and/or a combination comprising a tissue-targeting compound of formula (I) and a further pharmaceutical agent for the manufacture of a medicament for the treatment and/or prophylaxis of a hyperproliferative disease.

In some embodiments, the present invention includes the use of a tissue-targeting compound of formula (I) and/or a combination comprising a tissue-targeting compound of formula (I) and a further pharmaceutical agent for the manufacture of a medicament for the treatment of a hyperproliferative disease, particularly cancer and more particularly bladder, brain, breast, colon, liver, lung, pancreatic, prostate, HNSCC or stomach cancer.

### Administration

In the combinations comprising a tissue-targeting compound of formula (I) and a further pharmaceutical agent, the tissue-targeting compound of formula (I) and the further pharmaceutical agent may be administered sequentially in either order, or simultaneously.

The tissue-targeting compounds of formula (I) or the combinations of the present invention comprising a tissue-targeting compound of formula (I) and a further pharmaceutical agent can be administered in any form by any effective route, including, e.g., oral, parenteral, enteral, intravenous, intraperitoneal, topical, transdermal (e.g., using any standard patch), ophthalmic, nasally, local, non-oral, such as aerosol, inhalation, subcutaneous, intramuscular, buccal, sublingual, rectal, vaginal, intra-arterial, and intrathecal, etc. They can be administered alone, or in combination with any ingredient(s), active or inactive. Preferably, the tissue-targeting compounds of formula (I) or the combinations of the present invention are administered intravenously.

The tissue-targeting compounds of formula (I) or the combinations of the present invention comprising a tissue-targeting compound of formula (I) and a further pharmaceutical agent can be converted in a known manner into the usual pharmaceutical formulations, which may be liquid or solid formulations e.g., without limitation normal and enteric coated tablets, capsules, pills, powders, granules, elixirs, tinctures, solutions, suspensions, syrups, solid and liquid aerosols and emulsions.

The relative ratios of each compound in the combinations of the present invention comprising a tissue-targeting compound of formula (I) and a further pharmaceutical agent can also be selected based on their respective mechanisms of action and the disease biology. The relative ratios of each compound can vary widely.

The release of one or more agents of the combinations of the present invention comprising a tissue-targeting compound of formula (I) and a further pharmaceutical agent can also be controlled, where appropriate, to provide the desired therapeutic activity when in a single dosage form, combination pack, kit or when in separate independent dosage forms.

The present invention includes pharmaceutical compositions which are comprised of a pharmaceutically acceptable carrier and a pharmaceutically effective amount of the tissue-targeting compounds of formula (I) or the combinations of the present invention i.e., a tissue-targeting compound of formula (I) as described above and a further pharmaceutical agent. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated.

For oral administration, the tissue-targeting compounds of formula (I) or the combinations of the present invention comprising a tissue-targeting compound of formula (I) and a further pharmaceutical agent can be formulated into solid or liquid preparations such as solid dispersion, capsules, pills, tablets, troches, lozenges, melts, powders, solutions, suspensions, or emulsions, and may be prepared according to methods known to the art for the manufacture of pharmaceutical compositions. The solid unit dosage forms can be a capsule which can be of the ordinary hard- or soft-shelled gelatin type containing, for example, surfactants, lubricants, and inert fillers such as lactose, sucrose, calcium phosphate, and corn starch.

If administered in the form of a tablet, the tissue-targeting compounds of formula (I) or the combinations of the present invention comprising a tissue-targeting compound of formula (I) and a further pharmaceutical agent may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance, tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The tissue-targeting compounds of formula (I) or the combinations of the present invention comprising a tissue-targeting compound of formula (I) and a further pharmaceutical agent may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soybean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more coloring agents; one or more flavoring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavoring and coloring agents.

The tissue-targeting compounds of formula (I) or the combinations of the present invention comprising a tissue-targeting compound of formula (I) and a further pharmaceutical agent may also be administered parenterally, that is, subcutaneously, intravenously, intraocularly, intrasynovially, intramuscularly, or intraperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Preferably, the tissue-targeting compounds of formula (I) or the combinations of the present invention comprising a tissue-targeting compound of formula (I) and a further pharmaceutical agent are administered intravenously and/or orally, either together or separately. Preferably, in the combinations of the present invention comprising a tissue-targeting compound of formula (I) and a further pharmaceutical agent, the tissue-targeting compound of formula (I) is administered intravenously, and the further pharmaceutical agent is administered orally.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The tissue-targeting compounds of formula (I) or the combinations of the present invention comprising a tissue-targeting compound of formula (I) and a further pharmaceutical agent may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

The tissue-targeting compounds of formula (I) or the combinations of the present invention comprising a tissue-targeting compound of formula (I) and a further pharmaceutical agent may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such material is, for example, cocoa butter and polyethylene glycol.

Controlled release formulations for parenteral administration include liposomal, polymeric microsphere and polymeric gel formulations which are known in the art.

The tissue-targeting compounds of formula (I) or the combinations of the present invention comprising a tissue-targeting compound of formula (I) and a further pharmaceutical agent may also be in the form of a solid dispersion. The solid dispersion may be a solid solution, glass solution, glass suspension, amorphous precipitation in a crystalline carrier, eutectic or monotectic, compound or complex formation and combinations thereof.

The tissue-targeting compounds of formula (I) or the combinations of the present invention comprising a tissue-targeting compound of formula (I) and a further pharmaceutical agent can also contain other conventional pharmaceutically acceptable compounding ingredients, generally referred to as carriers or diluents, as necessary or desired. Conventional procedures for preparing such compositions in appropriate dosage forms can be utilized.

Commonly used pharmaceutical ingredients which can be used as appropriate to formulate the composition for its intended route of administration include:
- acidifying agents (examples include but are not limited to acetic acid, citric acid, fumaric acid, hydrochloric acid, nitric acid);
- alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);
- adsorbents (examples include but are not limited to powdered cellulose and activated charcoal);
- aerosol propellants (examples include but are not limited to carbon dioxide, CCl2F2, F2CIC-CCIF2 and CCIF3)
- air displacement agents (examples include but are not limited to nitrogen and argon);
- antifungal preservatives (examples include but are not limited to benzoic acid, butylparaben, ethylparaben, methylparaben, propylparaben, sodium benzoate);
- antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
- antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
- binding materials (examples include but are not limited to block polymers, natural and synthetic rubber, polyacrylates, polyurethanes, silicones, polysiloxanes and styrenebutadiene copolymers);
- buffering agents (examples include but are not limited to potassium metaphosphate, dipotassium phosphate, sodium acetate, sodium citrate anhydrous and sodium citrate dihydrate)
- carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)
- chelating agents (examples include but are not limited to edetate disodium and edetic acid)
- colorants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
- clarifying agents (examples include but are not limited to bentonite);
- emulsifying agents (examples include but are not limited to acacia, cetomacrogol, cetyl alcohol, glyceryl monostearate, lecithin, sorbitan monooleate, polyoxyethylene 50 monostearate);
- encapsulating agents (examples include but are not limited to gelatin and cellulose acetate phthalate)
- flavorants (examples include but are not limited to anise oil, cinnamon oil, cocoa, menthol, orange oil, peppermint oil and vanillin);
- humectants (examples include but are not limited to glycerol, propylene glycol and sorbitol);
- levigating agents (examples include but are not limited to mineral oil and glycerin);
- oils (examples include but are not limited to arachis oil, mineral oil, olive oil, peanut oil, sesame oil and vegetable oil);
- ointment bases (examples include but are not limited to lanolin, hydrophilic ointment, polyethylene glycol ointment, petrolatum, hydrophilic petrolatum, white ointment, yellow ointment, and rose water ointment);
- penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
- plasticizers (examples include but are not limited to diethyl phthalate and glycerol);
- scavenging compounds which reduce and/or prevent radiolysis;
- solvents (examples include but are not limited to ethanol, corn oil, cottonseed oil, glycerol, isopropanol, mineral oil, oleic acid, peanut oil, purified water, water for injection, sterile water for injection and sterile water for irrigation);
- stiffening agents (examples include but are not limited to cetyl alcohol, cetyl esters wax, microcrystalline wax, paraffin, stearyl alcohol, white wax and yellow wax);
- suppository bases (examples include but are not limited to cocoa butter and polyethylene glycols (mixtures));
- surfactants (examples include but are not limited to benzalkonium chloride, nonoxynol 10, oxtoxynol 9, polysorbate 80, sodium lauryl sulfate and sorbitan mono-palmitate);
- suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
- sweetening agents (examples include but are not limited to aspartame, dextrose, glycerol, mannitol, propylene glycol, saccharin sodium, sorbitol and sucrose);
- tablet anti-adherents (examples include but are not limited to magnesium stearate and talc);
- tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
- tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
- tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
- tablet direct compression excipients (examples include but are not limited to dibasic calcium phosphate);
- tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);
- tablet glidants (examples include but are not limited to colloidal silica, corn starch and talc);
- tablet lubricants (examples include but are not limited to calcium stearate, magnesium stearate, mineral oil, stearic acid and zinc stearate);
- tablet/capsule opaquants (examples include but are not limited to titanium dioxide);
- tablet polishing agents (examples include but are not limited to carnauba wax and white wax);
- thickening agents (examples include but are not limited to beeswax, cetyl alcohol and paraffin);
- tonicity agents (examples include but are not limited to dextrose and sodium chloride);
- viscosity increasing agents (examples include but are not limited to alginic acid, bentonite, carbomers, carboxymethylcellulose sodium, methylcellulose, polyvinyl pyrrolidone, sodium alginate and tragacanth); and
- wetting agents (examples include but are not limited to heptadecaethylene oxycetanol, lecithin, sorbitol monooleate, polyoxyethylene sorbitol monooleate, and polyoxyethylene stearate).

One skilled in the art, utilizing the preceding information, can utilize the present invention to its fullest extent.

### DESCRIPTION OF THE FIGURES

Figure 1. Size-exclusion UV chromatogram of Trastuzumab-macropa.
Figure 2. Electrospray mass spectrum of Trastuzumab-macropa (CAR 0.5)
Figure 3. In vitro binding properties of Trastuzumab and Trastuzumab-macropa on HER2-expressing cells lines JIMT-1, MCF-7, T-47D and ZR-75-1.
Figure 4. Binding of Ac-225-macropa-Trastuzumab at 2 kBq/µg to HER2-coated beads.
Figure 5. Internalization of Ac-225-macropa-Trastuzumab at 20 kBq/µg in HER2-expressing JIMT-1 cells.
Figure 6A. Cell toxicity of Ac-225-macropa-Trastuzumab at 20 kBq/µg on HER2-expressing cells line JIMT-1 - Effect of exposure time.
Figure 6B. Inhibition of the cytotoxicity of Ac-225-macropa-Trastuzumab at 20 kBq/µg by 2 hours of pre-incubation with Trastuzumab in JIMT-1 and SKOV-3 cells.
Figure 6C. Cytotoxicity of Ac225-macropa-Trastuzumab at 2, 10 and 20 kBq/µg in HER2-expressing cell lines JIMT-1, MCF-7, T-47D and ZR-75-1.
Figure 7A. Biodistribution of Ac-225-macropa-Trastuzumab (HER2) in the HER2-expressing JIMT-1 tumor model. The results are presented as %injected dose per gram (%ID/g). n=3 per timepoint.
Figure 7B. *In vivo* antitumor efficacy of ²²⁵Ac-Macropa-Trastuzumab vs vehicle and Enhertu (fam-trastuzumab deruxtecan-nxki) in the ZR-75-1 breast cancer model on mice. Tumor bearing animals received ²²⁵Ac-Macropa-Trastuzumab (125, or 250 kBq/kg; total antibody dose of 0.75 mg/kg; treatments on day 0 and day 28; i.v.) or Enhertu (2.7, or 5.4 mg/kg;
treatments on day 0 and day 21; i.v.), (n=12 per group). Statistical analyses were performed using one-way ANOVA followed by Dunnett's test. ***, P<0.001 (day 73).
Figure 8. Size-exclusion UV chromatogram of Codrituzumab-macropa.
Figure 9. Electrospray mass spectrum of Codrituzumab-macropa (CAR 0.7).
Figure 10. Electrospray mass spectrum of Codrituzumab-DOTA (CAR 4.6).
Figure 11. In vitro binding of Codrituzumab and Codrituzumab-macropa on GPC3-expressing cell line Hep3B2.
Figure 12. In vitro binding of Codrituzumab and Codrituzumab-DOTA to human recombinant GPC3.
Figure 13. IRF of Ac-225-macropa-Codrituzumab showing binding to GPC3-peptide coated beads.
Figure 14. Cell toxicity of Ac-225-macropa-Codrituzumab in GPC3-expressing cell lines: HepG2 (A) and Hep3B2-1.7 (B) cells.
Figure 15. BioD of Ac-225-macropa-isotype control and Ac-225-macropa-Codrituzumab (GPC3) in the GPC3-low expressing tumor model Huh-7. The results are presented as %injected dose per gram (%ID/g). n=3 per timepoint.
Figure 16. Efficacy of Ac-225-macropa-Codrituzumab (GPC3) vs vehicle and Ac-225-macropa isotype-control in the GPC3-low expressing model Huh-7. The mice were treated with a single dose IV of 500 kBq/kg with a total antibody dose of 0.75 mg/kg, with a repeat dose of 250 kBq/kg on day 25 for the surviving animals. n=7 per group.
Figure 17. Size-exclusion UV chromatogram of Labetuzumab-macropa.
Figure 18. Electrospray mass spectrum of Labetuzumab-macropa (CAR 0.5).
Figure 19. In vitro binding of Labetuzumab and Labetuzumab-macropa on CEACAM5-expressing cell lines HT-55 and MKN-45.
Figure 20. IRF, Binding of Ac-225-macropa-Labetuzumab at 2 kBq/µg to CEACAM5-coated beads.
Figure 21. Internalization of Labetuzumab. CEACAM5 expressing MKN-45 cells were incubated with Labetuzumab, isotyp or an anti-CD71 (transferrin recpetor) control antobdy for up to 24 hours showing internalization for Labetuzumab as well as the anti-CD71 positive control antibody.
Figure 22. BioD of Ac-225-macropa-Labetuzumab (CEACAM5) in the CEACAM5 expressing tumor model HT-55. The results are presented as %injected dose per gram (%ID/g). n=3 per timepoint.
Figure 23. Cell toxicity of Ac-225-macropa-Labetuzumab at 2 kBq/µg on CEACAM5-expressing cells lines HT-55 and MKN-45.
Figure 24. Size-exclusion UV chromatogram of Sacituzumab-macropa.
Figure 25. Electrospray mass spectrum of Sacituzumab-macropa (CAR 0.5).
Figure 26. In vitro binding properties of Sacituzumab and Sacituzumab-macropa on TROP2-expressing cells lines JIMT-1, MCF-7, T-47D and ZR-75-1.
Figure 27. Cell toxicity of Ac-225-macropa-Sacituzumab in TROP2-expressing cell lines: C4-2, JIMT-1, T-47D, NCI-N87 and Calu-3 cells.
Figure 28. Efficacy of two different doses of Ac-225-macropa-Sacituzumab vs Sacituzumab govitecan, vehicle and Ac-225-macropa isotype-control in the TROP2-low expressing model LK-2. The mice were treated with a single dose IV of 100 or 200 kBq/kg with a total antibody dose of 0.75 mg/kg, compared to once weekly IV dosing of Sacituzumab govitecan. n=12 per group.
Figure 29. Size-exclusion UV chromatogram of Indusatumab-macropa.
Figure 30. Electrospray mass spectrum of Indusatumab-macropa (CAR 0.5).
Figure 31. In vitro binding of Indusatamab and Indusatamab -macropa on GUCY2C-expressing cell line CHO-GUCY2C.
Figure 32. Binding of Ac-225-Macropa-Indusatumab to GUCY2C coated beads.
Figure 33. Cell toxicity of Ac-225-macropa-lndusatumab in CHO cells stably expressing GUCY2C.
Figure 34. BioD of Ac-225-macropa-lndusatamab (GUCY2C) in the GUCY2C expressing tumor model T84. The results are presented as %injected dose per gram (%ID/g). n=3 per timepoint.
Figure 35. Size-exclusion UV chromatogram of Telisotuzumab-macropa.
Figure 36. Electrospray mass spectrum of Telisotuzumab-macropa (CAR 0.9).
Figure 37. *In vitro* binding of Telisotuzumab and Telisotuzumab-macropa on cMET-expressing cell lines HT-55 and MKN-45.
Figure 38. Cell toxicity of Ac-225-macropa-Telisotuzumab on cMET expressing cell lines HT-55 and MKN-45.
Figure 39. Size-exclusion UV chromatogram of Farletuzumab-macropa and Farletuzumab-DOTA.
Figure 40. Electrospray mass spectrum of Farletuzumab-macropa (CAR 0.7).
Figure 41. Electrospray mass spectrum of Farletuzumab-DOTA (CAR 5.8).
Figure 42. Cell toxicity of Ac-225-macropa-Farletuzumab on FolRa expressing cell lines.
Figure 43. *In vitro* binding of Farletuzumab, Farletuzumab-macropa and Farletuzumab-DOTA on FOLRa expressing cell lines OUMS-23 and T-47D.
Figure 44. Size-exclusion UV chromatogram of Mirvetuximab-macropa and Mirvetuximab-DOTA.
Figure 45. Electrospray mass spectrum of Mirvetuximab-macropa (CAR 0.9).
Figure 46. Electrospray mass spectrum of Mirvetuximab-DOTA (CAR 5.0).
Figure 47. In vitro binding of Mirvetuximab, Mirvetuximab-macropa and Mirvetuximab-DOTA on FOLRa expressing cell lines OUMS-23 and T-47D.
Figure 48. Cell toxicity of Ac-225-macropa-Mirvetuximab on FolRa expressing cell lines.
Figure 49. Size-exclusion UV chromatogram of Rovalpituzumab-macropa and Rovalpituzumab-DOTA.
Figure 50. Electrospray mass spectrum of Rovalpituzumab-macropa (CAR 0.9).
Figure 51. Electrospray mass spectrum of Rovalpituzumab-DOTA (CAR 7.1).
Figure 52. Binding potency of Rovalpituzumab-macropa, Rovalpituzumab-DOTA and Rovalpituzumab to human recombinant DLL3 antigen.
Figure 53. IRF of Ac-225-macropa-Rovalpituzumab showing binding to DLL3- coated beads.
Figure 54. Cell toxicity of Ac-225-macropa-Rovalpituzumab at 20 kBq/µg in DLL3-expressing cell lines: NCI-H82 and TT cells.
Figure 55. Radiochemical purity defined as the amount of Bismuth-223 bound to pelgi-macropa in comparison to pelgi-DOTA and DOTA-J591, as determined by instant thin-layer chromatography (iTLC).

### EXPERIMENTAL SECTION

Chemical names were generated using the BIOVIA Draw 2019 from Dassault Systèmes. In some cases, generally accepted names of commercially available reagents were used in place of BIOVIA Draw generated names.

The following Table 1 lists the abbreviations used in this paragraph and in the Examples section as far as they are not explained within the text body. Other abbreviations have their meanings customary *per se* to the skilled person.

**Table 1. Abbreviations**

| The following table lists the abbreviations used herein. | |
|---|---|
| ²²⁵Ac | Actinium-225 |
| ABTS | 2,2'-azino-bis (3-ethylbenzothiazoline-6-sulphonic acid |
| Ac-225 | Actinium-225 |
| ACC | Antibody-chelator conjugate |
| ACN | Acetonitrile |
| ATP | Adenosine triphosphate |
| Bq | Becquerel |
| BSA | Bovine serum albumin |
| CAR | Chelator-to-antibody ratio |
| CEACAM5 | Carcinoembryonic antigen-related cell adhesion molecule 5 |
| c-MET | Mesenchymal-epithelial transition factor |
| CRC | Colorectal cancer |
| Deg | Degrees |
| DLL3 | Delta-like ligand 3 |
| DMA | N,N-dimethylacetamide |
| DMEM | Dulbecco's modified Eagle's medium |
| DMSO | Dimethyl sulfoxide |
| DOTA | 1,4,7,10-tetraazacyclododecane-1,4,7,10-tetraacetic acid |
| EC50 | Half maximal effective concentration |
| ELISA | Enzyme-linked immunosorbent assay |
| ESI | Electrospray ionization |
| FBS | Fetal bovine serum |
| FOLRa | folate receptor alpha |
| FPLC | fast protein liquid chromatography |
| G418 | Geneticin |
| GPC3 | Glypican-3 |
| GPI | Glycophosphatidylinositol |
| GUCY2C | Guanylate cyclase 2C |
| h | Hours |
| HCC | Hepatocellular carcinoma |
| HCl | Hydrochloric acid |
| HER2 | Human epidermal growth factor receptor 2 |
| HRP | Horse radish peroxidase |
| HPGe | High purity germanium |
| HPLC | High performance liquid chromatography |
| ID | Injected dose |
| iTLC | Instant thin layer chromatography |
| IRF | Immunoreactive fraction |
| mAb | Monoclonal antibody |
| MEM | Minimum essential medium |
| MEM-NEAA | Non-essential amino acid solution |
| min | Minutes |
| MS | Mass spectrometry |
| NaCl | Sodium chloride |
| nm | Nanometer |
| nmol | Nanomole |
| pelgi | Pelgifatamab, prostate-specific membrane antigen IgG1 antibody |
| PBS | Phosphate buffered saline |
| PI | Propidium iodide |
| PSMA | Prostate-specific membrane antigen |
| RT | Room temperature |
| RAC | Radioactive concentration |
| RCC | Renal cell carcinoma |
| RCP | Radiochemical purity |
| RPMI | Roswell Park Memorial Institute 1640 medium |
| SEC | Size exclusion chromatography |
| TAC | Targeted actinium conjugate (Ac-225 labelled ACC) |
| TFA | Trifluoroacetic acid |
| TOF | Time of flight |
| TROP2 | Trophoblast cell surface antigen 2 |
| UPLC | Ultra performance liquid chromatography |
| UV | Ultraviolet |

All reagents, for which the synthesis is not described in the experimental part, are either commercially available, or known compounds or may be formed from known compounds by known methods by a person skilled in the art.

The various aspects of the invention described in this application are illustrated by the following examples which are not meant to limit the invention in any way.

The example testing experiments described herein serve to illustrate the present invention and the invention is not limited to the examples given.

### Antibodies

All antibodies were expressed in HEK293 cells using standard transient transfection procedures and purified from the cell culture supernatant via Protein-A and size exclusion chromatography (reference e.g. Durocher et al., Nucleic Acids Res. 2002 Jan 15;30(2):E9).

### Conjugation of Macropa to the antibodies (ACCs)

6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid (Macropa-NCS-2; abbreviated as "Macropa" throughout the document) or p-SCN-Bn-DOTA (Macrocyclics B205) were dissolved in DMA to 10 mg/ml. The antibodies were dissolved in PBS to 10 mg/mL and the pH was adjusted to 9 with 1M carbonate buffer. Chelator and antibody were mixed and incubated on a thermomixer shaking at 350 rpm at RT for two hours. Molar ratio of chelator and antibody was selected depending on the desired Chelator to antibody ratio (CAR). Products were purified by FPLC (column: HiLoad 16/600 Superdex 200 pg column; flow: 1 mL/min; in 0.1 M Acetate buffer (pH 5); detection: UV 280/254 nm). Concentration and monomeric purity were determined by SEC-UV HPLC (as described below). Chelator-to-antibody ratio (CAR) was determined by SEC-MS using positive electrospray ionization (as described below) and the following equation: CAR=[(A*An)/A]0, A=Intensity of all signals of the maximum entropy envelope, An=signals of the maximum entropy envelope containing chelators.

### ACC characterization by size exclusion chromatography and mass spectrometry

CAR of the ACCs was determined by SEC-MS (Water Acquity HPLC connected to Waters XEVO TOF; running buffer: 50/50/0.1 (v/v/v) ACN/Water/TFA; flow rate: 0.06 mL/min, column for on-line desalting: Waters Acquity BEH SEC, 1.7 µm, 2.1 × 150 mm; ESI+ mode) by using MS peak heights in percentage of major peak height for the components mAb, mAb + 1 chelator, mAb + 2 chelators, mAb + 3 chelators etc. and using the formula CAR = Sum(n*An)/Sum An, where n equals the number of chelators and An equals the intensity of the antibody conjugate with n chelators. Purity of the ACC were determined by SEC-UV (Agilent 1260 Infinity HPLC system, running buffer: 10% (v/v) DMSO in PBS and 300 mM NaCl; flow rate: 0.3 mL/min, column: Waters Acquity BEH SEC, 1.7 µm, 4.6 × 300 mm, detection: UV at 280 nm). The method separates higher aggregates, dimers and fragments from the ACC monomer.

### Flow cytometry

The in vitro binding properties of antibody-chelator conjugates were assessed in various relevant cell lines and compared to their corresponding unconjugated antibody. Briefly, samples were diluted in buffer (PBS/3% FBS) and added to relevant cell lines. After completion of the binding step (60 min at 4°C), the cells were washed and incubated with secondary antibody conjugated with phycoerythrin to detect antibodies containing a human IgG Fc region. After 60 min at 4°C, the cells were washed, and fluorescence on the cells was determined by flow cytometry using Guava EasyCyte 8HT (Luminex).

### Radiolabelling of Targeted Actinium-225 Conjugates (TACs)

Actinium-225 (Ac-225) was added to antibody-chelator conjugates in 0.1 M Acetate buffer (pH 5) at a specific activity of 2-20 MBq/mg and a radioactive concentration of 1-2.5 kBq/µL. The Ac-225-Macropa conjugates were incubated for one hour at room temperature followed by determination of radiochemical purity (RCP) by iTLC, while Ac-225-DOTA conjugates were incubated for one hour at 60 °C.

### Instant thin-layer chromatography (iTLC):

An iTLC was preformed to measure radiochemical purity (RCP) of the Ac-225-Macropa and Ac-225-DOTA conjugate solutions. In brief, an iTLC paper impregnated with a silica gel plate was cut to obtain 11 cm × 1 cm strips. Application point (1 cm), cut point (4 cm) and end of run point (10 cm) was marked and 2 µl of TAC was applied on to iTLC strips. The strips were developed in 3 mL mobile phase (0.1 M Citrate in 0.9%(v/v) NaCl pH 5.5) in LSC vials. Free Ac-225 migrated on the strip with the solvent while complexed Ac-225 stayed at the application point. When the liquid front had reached the marked end point, the strips were removed from the vials and placed on an aluminum foil to dry. The strips were cut in two at 4 cm: the shorter "application" half (A) and the longer "front" half (F). Each part was carefully folded and placed at the bottom of scintillation vials. After 6 hours secular equilibrium was obtained between Ac-225 and daughters, the application part was counted for 60 sec and front part counted for 120 sec in a high purity Germanium detector (Ortec). The radiochemical purity (% RCP) of the samples (defined as [(radioactivity of application section)/(total radioactivity on strip)] × 100) was calculated. RCP was determined for all TAC samples and is shown in Table 2.

**Table 2: Radiochemical purity (% RCP) of samples included in this invention.**

| TAC samples | % RCP |
|---|---|
| Ac-225-macropa-Trastuzumab | 100 (n=9) |
| Ac-225-macropa-Codrituzumab | 99.9 (n=1) |
| Ac-225-macropa-Labetuzumab | 99.9 (n=2) |
| Ac-225-macropa-Sacituzumab | 99.7 (n=2) |
| Ac-225-macropa-Indusazumab | 99.9 (n=2) |
| Ac-225-macropa-Telisotuzumab | 99.7 (n=2) |
| Ac-225-macropa-Farletuzumab | 99.9 (n=2) |
| Ac-225-macropa-Mirvetuzumab | 99.9 (n=2) |
| Ac-225-macropa-Rovalpituzumab | 99.9 (n=2) |
| Ac-225-DOTA-Farletuzumab | 60.6 (n=2) |
| Ac-225-DOTA-Mirvetuzumab | 67.2 (n=2) |

### Immunoreactive fraction (IRF)

Shortly after completion of the radiolabeling, the binding was assessed by measuring the fraction of radiolabeled antibody-chelator conjugate bound to relevant antigen coated magnetic beads (Thermo Fisher Scientific). In short, beads were placed in 2 mL Eppendorf tubes in 90 µL buffer (PBS/0.1% Tween 20/1% BSA). The radiolabeled compounds were diluted to 5 Bq/µL in buffer and 50 Bq were added to each bead-containing tube. The binding lasted for 60 min while shaking at 750 rpm at room temperature. After completion of the binding step, magnet (DynaMag-2) was used to separate the beads from half of the supernatant. The radio activities in the supernatant and in the beads of each sample were measured using a high purity Germanium detector (Ortec). The activities were measured after at least 6 hours, to allow daughter nuclides to be in equilibrium with Ac-225. Total binding was defined as [(activity in beads - activity in supernatant)/ (total activity)] × 100.

### Cell cytotoxicity

In vitro cytotoxicity of Ac-225-ACC was determined in cell lines expressing different levels of relevant target receptors. Cells were seeded at an appropriate cell density in relevant cell media. One day after seeding, the radiolabeled compounds at the specific activity of 2-20 kBq/µg and radioactive concentration of 50-100 kBq/ml were titrated in parallel with a radiolabeled isotype control. Cells were incubated for 5-6 days. Cell viability was determined by cell counting using Hoechst and PI staining and analyzed on Operetta CLS High Content Analysis System (Perkin Elmer) or by ATP measurements using CellTiterGlo (Promega).

### ELISA

The relevant antigen was immobilized on ELISA plate by absorption. The plate was washed (PBS with 0.1% Tween 20) and incubated with blocking buffer (PBS + 3% BSA) for 2 h ± 1 h to reduce unspecific binding. Relevant antibodies and ACCs diluted in assay buffer (PBS with 0.05% Tween 20 + 1.5% BSA). The blocking buffer was discarded, and the antibodies and ACCs and controls were applied in triplicates to the plate according to a plate set-up defined in each protocol. After incubation for 1 h ± 30 min at RT, the plate was washed. A horse radish peroxidase (HRP)-conjugated secondary antibody binding specifically to the primary mAb (anti-human IgG-HRP) was added to the plate. After incubation for 1 hour ± 30 min at RT, the plate was washed. A chromogenic substrate (2,2'-azino-bis (3-ethylbenzothiazoline-6-sulphonic acid) (ABTS)), for HRP leading to the formation of a green color was added to the plate and used for detection. The absorbance of the colored reaction product was measured with an absorbance plate reader, e.g. EnVision 2103 Multilabel Reader from Perkin Elmer, or iMark Microplate Absorbance Reader from Bio-Rad.

Absorbance against the log of the antibody concentration was plotted on the GraphPad Prism software. A dose stimulation fit model with "log(agonist) vs response variable slope (four/three parameters)" was used to calculate the concentration necessary to reach 50% of the maximum Absorbance (EC50).

### Tissue-targeting compounds comprising Trastuzumab

Human epidermal growth factor receptor 2 (HER2) is a transmembrane tyrosine kinase receptor that influences cell growth, division, and repair; with increased expression, it can promote tumorigenesis. HER2 is frequently overexpressed or amplified and occasionally mutated in a variety of human cancers and is a well-established therapeutic target in breast and gastric cancers.

### ACC characterization by size exclusion chromatography and mass spectrometry of Trastuzumab-macropa

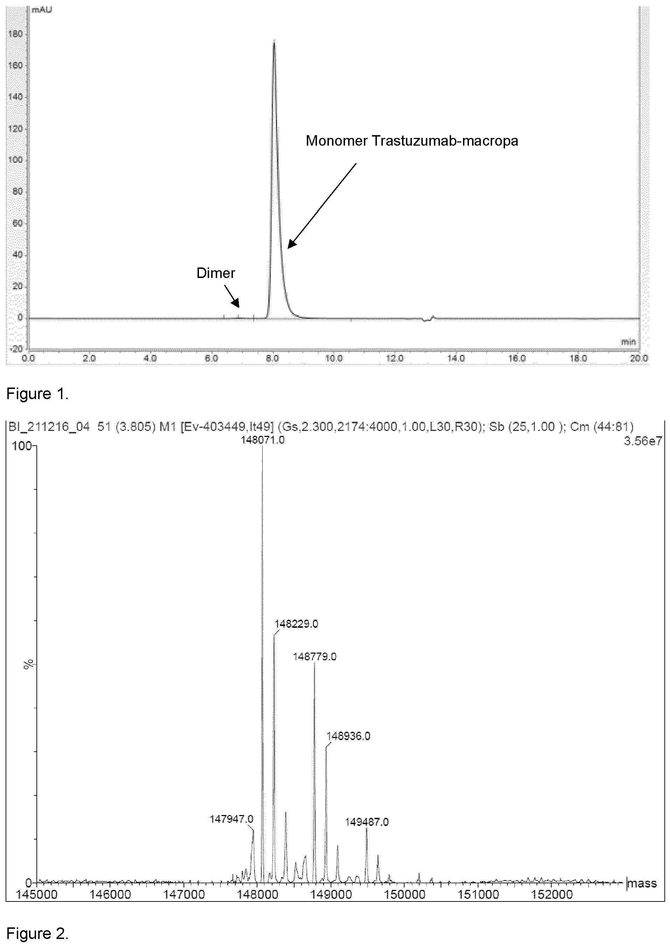

Purity (99.8%) and CAR (0.5) of Trastuzumab-macropa were determined by SEC-UV (Figure 1) and SEC-MS (Figure 2), respectively.

### Flow cytometry

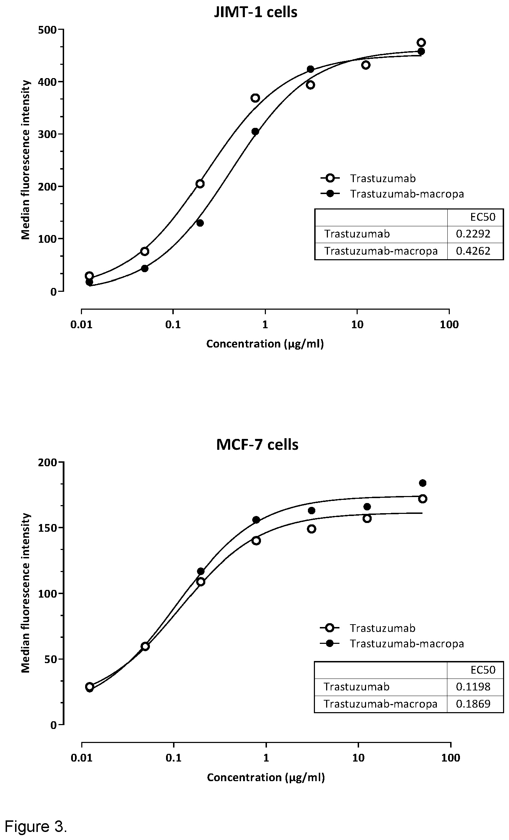

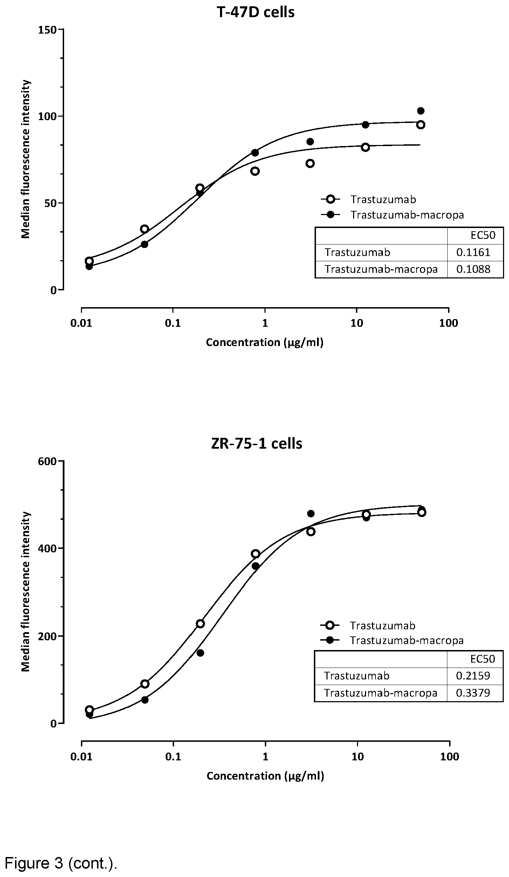

Cell surface binding of Trastuzumab-macropa was compared to unconjugated Trastuzumab and was investigated by flow cytometry using JIMT-1, MCF-7, T-47D and ZR-75-1 cells expressing HER2 (Figure 3). Trastuzumab macropa showed comparable EC50 as unconjugated Trastuzumab, indicating that binding capacity of the Trastuzumab-macropa was maintained after conjugation.

### Immunoreactive fraction (IRF)

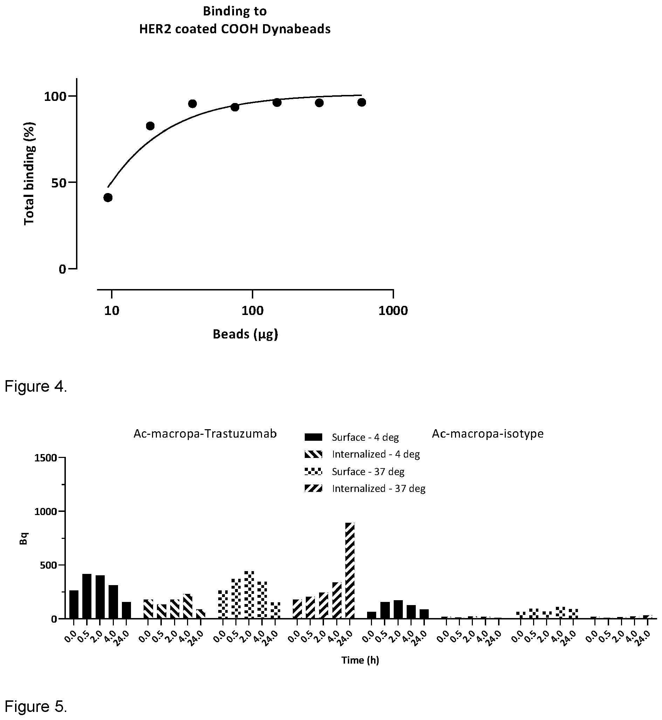

Binding of Ac-225-macropa-Trastuzumab to HER2-coated M-270-carboxy Dynabeads was determined by immunoreactive fraction (IRF as described above) assay (Figure 4). The maximum binding of Ac-225-macropa-Trastuzumab was achieved with 100 µg of HER2-coated beads.

### Internalization

JIMT-1 cells were at an appropriate cell density in DMEM + 10% FBS. One day after seeding, the cells were exposed to 10 kBq/ml of Ac-225-macropa-Trastuzumab and Ac-225-macropa-isotype both at a specific activity of 20 kBq/µg. The binding step was performed by incubation for 3 hours at 4 deg. At T0, some cells were placed at 37 deg while the others were kept at 4 deg. At each time point indicated in Figure 5, the cell culture medium containing radioligand was removed and replaced with ice-cold stripping buffer (RPMI-HCl pH 2.5). After a 5 min incubation, the stripping buffer was removed and collected as "surface fraction". The cells were then washed once with fresh RPMI which was pooled with the surface fraction. The cells were harvested using a cell scraper and collected in fresh RPMI as the "internalized fraction". The radio activities in the fractions of all exposures, time points and temperatures were measured using a high purity Germanium detector (Ortec). Figure 5 shows that Ac225-macropa-Trastuzumab was specifically binding to the surface of JIMT-1 cells and internalized slightly upon incubation of the cells at 37 deg.

### Cell cytotoxicity

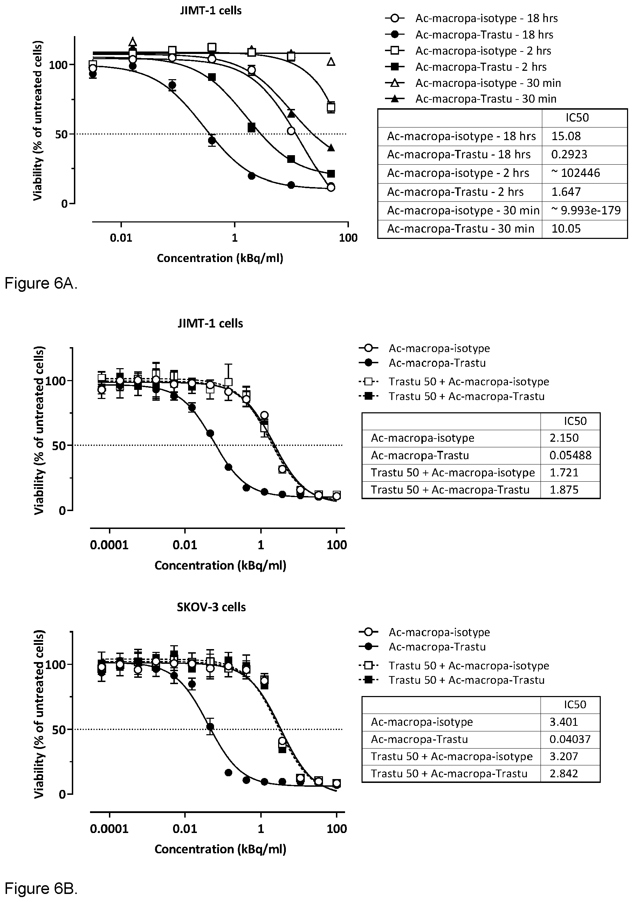

In-vitro cytotoxicity of Ac-225-macropa-Trastuzumab was determined in JIMT-1 cells. Cells were seeded at an appropriate cell density in DMEM + 10% FBS. One day after seeding, Ac-225-macropa-Trastuzumab at a specific activity of 20 kBq/µg was titrated from 50 kBq/ml in parallel with a relevant negative isotype control which was radiolabeled in the same conditions. After 30 min, 2 hrs and 18 hrs of exposure the medium was removed, each well was carefully added 200 µl of fresh medium which was removed as well. Finally, fresh medium was added, and the plates were further placed in the incubator until day 6. At day 6 cell viability was determined using CellTiterGlo (Promega). A strong and specific cytotoxicity was observed, already achieved after 30 min of exposure to the radioligand and increasing with longer exposure times (Figure 6A).

The inhibiting effect of Trastuzumab on the cytotoxicity induced by Ac-225-macropa-Trastuzumab was determined in JIMT-1 and SKOV-3 cells. Cells were seeded at an appropriate density in DMEM + 10% FBS. One day after seeding, 50 µg/ml of Trastuzumab were added to the cells. 2 hours later, Ac-225-macropa-Trastuzumab at a specific activity of 20 kBq/µg was titrated from 100 kBq/ml in parallel with a relevant negative isotype control which was radiolabeled in the same conditions. After 6 days cell viability was determined using CellTiterGlo (Promega). A strong cytotoxicity was achieved which was totally inhibited in the presence of cold Trastuzumab, indicating specificity (Figure 6B).

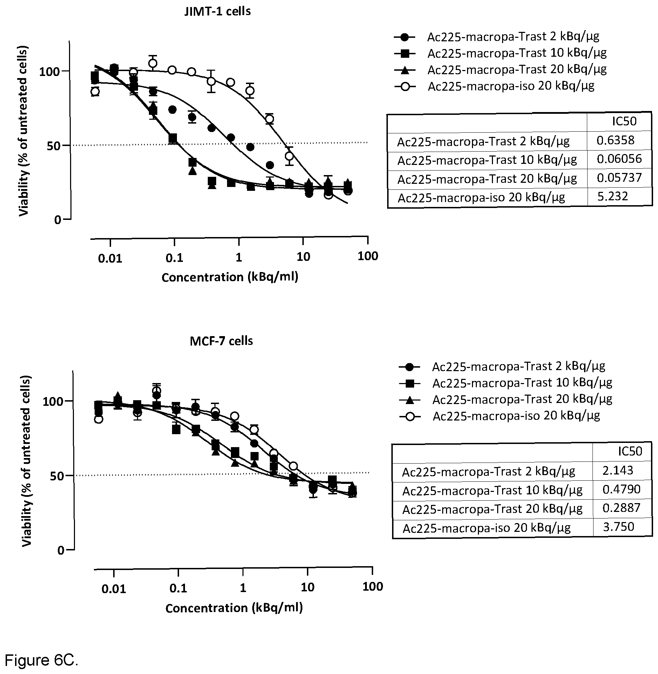

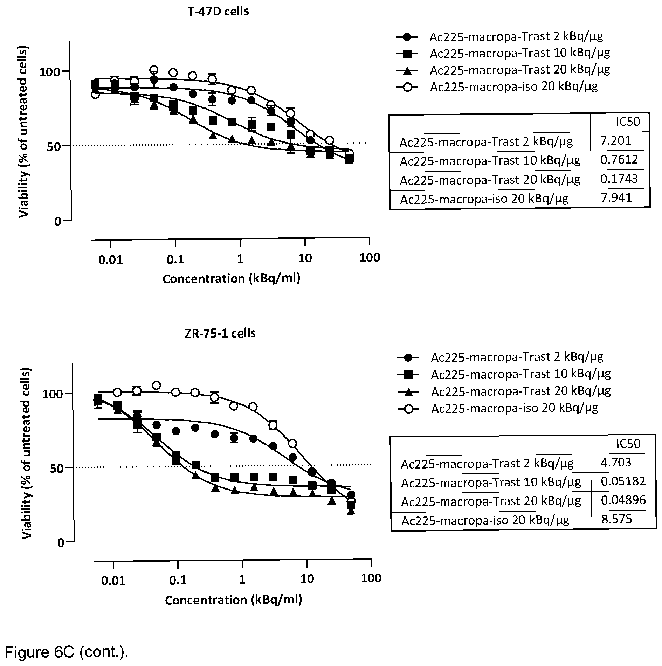

Finally, cytotoxicity of Ac225-macropa-Trastuzumab at specific activities of 2, 10 and 20 kBq/µg were investigated in cell lines with various expression of HER2. The cells were exposed as described above to a titration of the radioligands as well as a relevant isotype control radiolabeled at 20 kBq/µg. After 6 days cell viability was determined using CellTiterGlo. In all cell lines the cytotoxicity induced by Ac225-macropa-Trastuzumab had increasing specificity with increasing specific activity (Figure 6C).

### In vivo Biodistribution study of Ac-225-macropa Trastuzumab

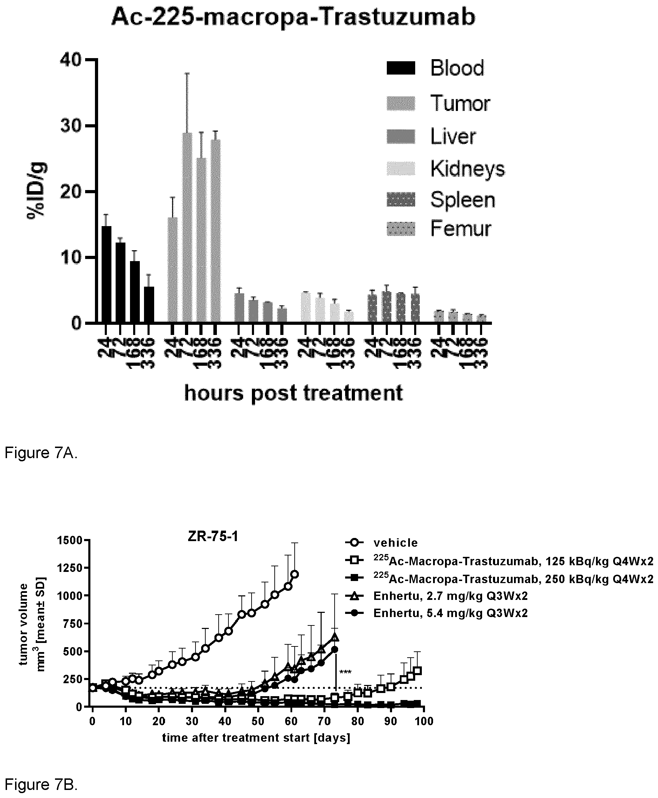

Biodistribution of Ac-225-macropa-Trastuzumab was investigated in the HER2-expressing breast carcinoma xenograft model JIMT-1. Female RJ: NRMI-Foxn1nu/nu mice were inoculated with 1 × 10⁶ JIMT-1 cells on the right flank. Animals were pre-treated with 200 µg/mouse unspecific IgG2a antibody 24 h prior to hot dosing. Animals were treated with 500 kBq/kg Ac-225-macropa-Trastuzumab IV at a total protein dose of 5,1 nmol/kg. Animals were sacrificed 24, 72, 168, and 336 hours post treatment and Ac-225 in blood, tumor, liver, kidney, spleen and femur was determined. Ac-225-macropa-Trastuzumab-treated animals had an average of 5.6 %ID/g in blood at 336 h, with high tumor uptake and retention over 336 h (Figure 7A). The uptake in liver, spleen, and kidneys was less than 5 %ID/g for all timepoints, and less than 2 %ID/g in femur (Figure 7A).

### In vivo antitumor efficacy of Ac-225-macropa Trastuzumab

The antitumor efficacy of ²²⁵Ac-Macropa-Trastuzumab was assessed in the ZR-75-1 breast cancer model. Female NMRI nu/nu mice were implanted s.c. with estradiol pellets and were inoculated subcutaneously with 5×10⁶ ZR-75-1 cells 1-2 days later.

Animals were randomized into treatment groups at an average tumor size of 169 mm³ (n=12) and treatment was started. Animals received either vehicle, ²²⁵Ac-Macropa-Trastuzumab (125, or 250 kBq/kg; total antibody dose of 0.75 mg/kg; treatments on day 0 and day 28; i.v.) or Enhertu (2.7, or 5.4 mg/kg; treatments on day 0 and day 21; i.v.).

Treatment with ²²⁵Ac-Macropa-Trastuzumab resulted in very strong and durable anti-tumor efficacy with complete remissions until end of the study in the higher dose group (250 kBq/kg), whereas Enhertu-treated groups showed onset of tumor regrowth at -day 50 of the study.

Statistical analyses were performed using one-way ANOVA followed by Dunnett's test. ***, =0.001 (day 73).

Figure 7B shows the *in vivo* antitumor efficacy of ²²⁵Ac-Macropa-Trastuzumab vs vehicle and Enhertu (fam-trastuzumab deruxtecan-nxki) in the ZR-75-1 breast cancer model on mice. Tumor bearing animals received ²²⁵Ac-Macropa-Trastuzumab (125, or 250 kBq/kg; total antibody dose of 0.75 mg/kg; treatments on day 0 and day 28; i.v.) or Enhertu (2.7, or 5.4 mg/kg; treatments on day 0 and day 21; i.v.), (n=12 per group). Statistical analyses were performed using one-way ANOVA followed by Dunnett's test. ***, P<0.001 (day 73).

### Tissue-targeting compounds comprising Codrituzumab

Glypican-3 (GPC3) is a surface heparan sulfate proteoglycan expressed in approximately 80% of hepatocellular carcinomas (HCC) and is associated with poorer prognoses. It is postulated that GPC3 plays a role in the regulation of cell division and growth. GPC3 is a clinically relevant biomarker for imaging and therapeutics, as its expression is HCC tumor-specific and absent from normal and other pathological liver tissues.

### ACC characterization by size exclusion chromatography (SEC) and mass spectrometry

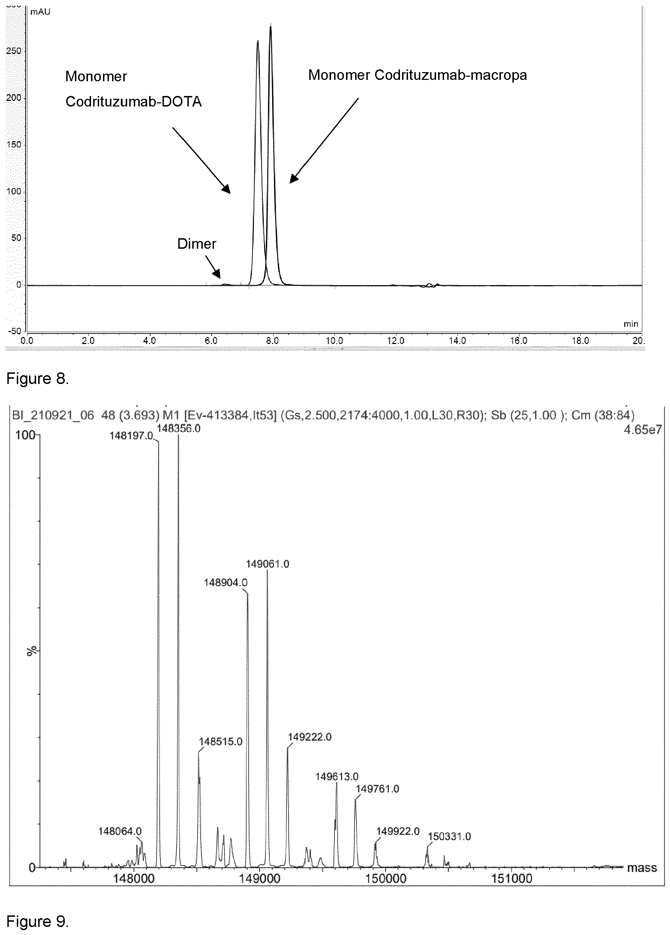

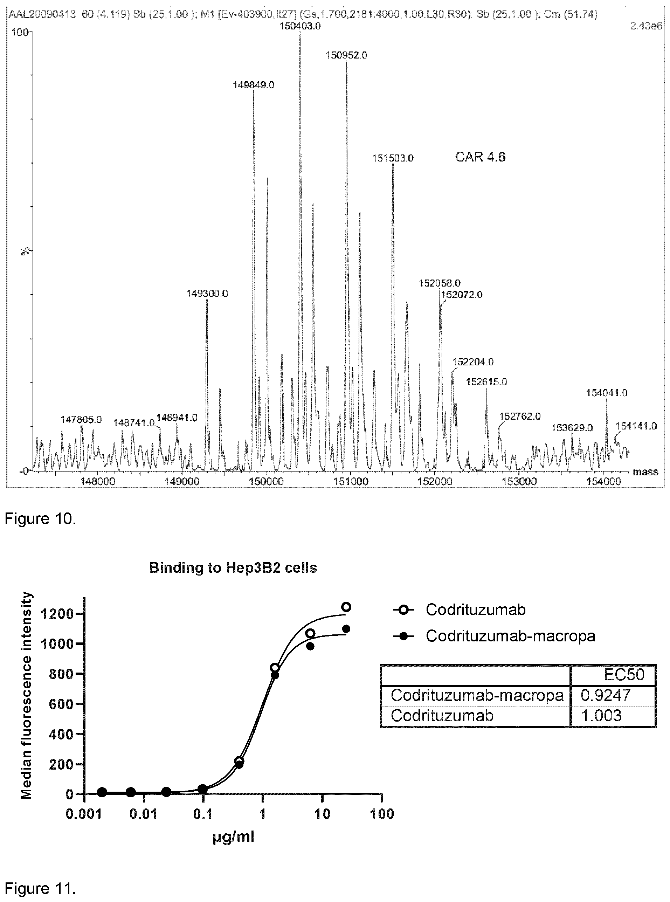

Purity (99.6%) and CAR (0.7) of Codrituzumab-macropa were determined by SEC-UV (Figure 8) and SEC-MS (Figure 9), respectively. Purity (99.4%) and CAR (4.6) of Codrituzumab-DOTA were determined by SEC-UV and SEC-MS (Figure 10), respectively.

### Flow cytometry

Cell surface binding of Codrituzumab-macropa was compared to unconjugated Codrituzumab and was investigated by flow cytometry using Hep3B2 cells expressing human glypican-3 (Figure 11). Codrituzumab-macropa show comparable EC50 as unconjugated Codrituzumab, indicating that binding capacity of the Codrituzumab-macropa is maintained after conjugation.

### ELISA

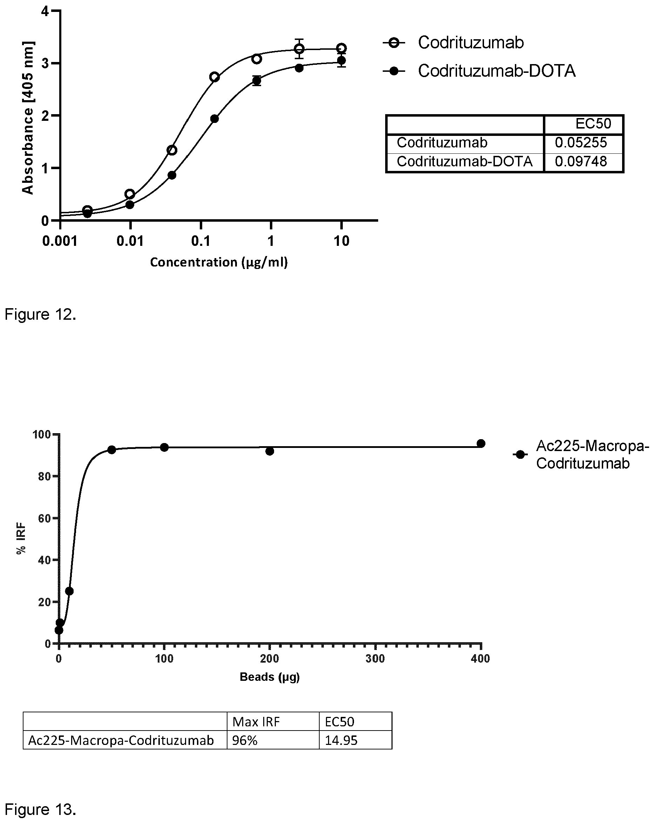

In vitro binding properties of Codrituzumab-DOTA was compared to unconjugated Codrituzumab and investigated by ELISA with plate coated with recombinant human glypican-3 (Figure 12). Codrituzumab-DOTA show higher EC50 than unconjugated Codrituzumab, indicating that binding capacity of the Codrituzumab-DOTA is reduced after conjugation.

### Immunoreactive fraction (IRF)

Shortly after radiolabeling, binding to of Ac225-Macropa-Codrituzumab to GPC3-peptide coated M-270-carboxy Dynabeads was determined by IRF assay (as described above). The maximum binding of Ac-225-macropa-Codrituzumab was achieved with 50 µg of GPC3-coated beads (Figure 13).

### Cell cytotoxicity

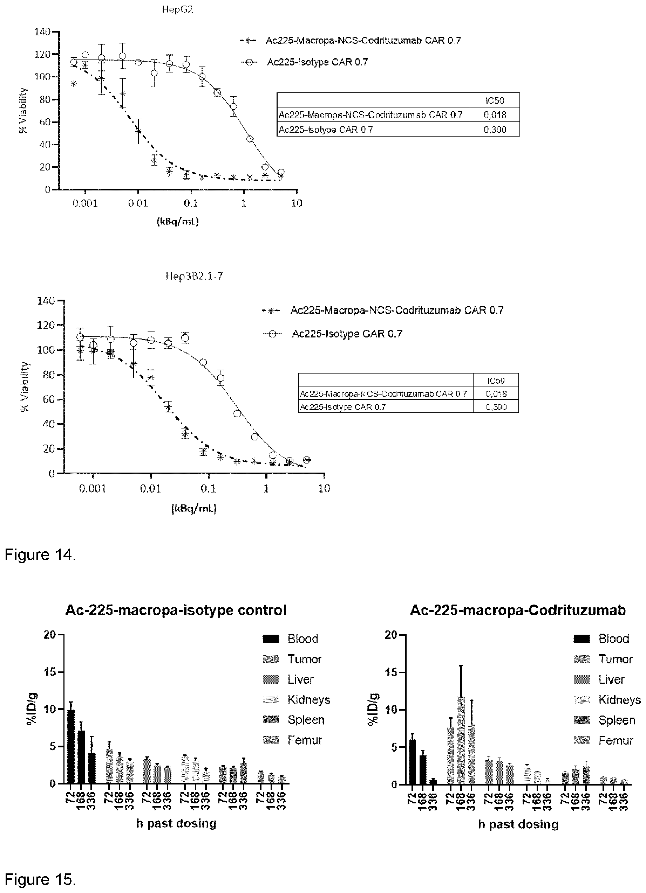

*In vitro* cytotoxicity of Ac-225-macropa-Codrituzumab was determined in Human Hepatocellular Carcinoma (HCC) cell lines expressing different levels of GPC3. HepG2 and Hep3B2.1-7 cells were seeded at an appropriate cell density in the cell media MEM + 10% FBS. HepG2 cells have higher GPC3 expression on the cell surface than Hep3B2.1-7 cells. One day after seeding the compounds at the specific activity of 2 MBq/mg and radioactive concentration of 5 kBq/ml were titrated in parallel with a radiolabeled isotype control. Cells were incubated for 6 days and subsequentially cell viability was determined using Hoechst and PI staining and analyzed on Operetta CLS High Content Analysis System (Perkin Elmer). A strong and specific cytotoxicity was observed in HepG2 and Hep3B2.1-7 cell lines (Figure 14).

### In vivo Biodistribution study comparing Ac-225-macropa-Codrituzumab and Ac-225-isotype-control

Biodistribution of Ac-225-macropa-Codrituzumab compared to Ac-225-macropa-isotype control was investigated in the GPC3 low-expressing hepatoma xenograft model Huh-7. Female RJ: NRMI-Foxn1nu/nu mice were inoculated with 2 × 10⁶ Huh-7 cells on the right flank. Animals were pre-treated with 200 µg/mouse unspecific IgG2a antibody 24 h prior to hot dosing. Animals were treated with 500 kBq/kg Ac-225-macropa-Codrituzumab and Ac-225-macropa-isotype-control IV at a total protein dose of 0,75 mg/kg. Animals were sacrificed 72, 168, and 336 hours post treatment and Ac-225 in blood, tumor, liver, kidney, spleen and femur was determined. Ac-225-macropa-Codrituzumab-treated animals had significantly higher tumor uptake at 168h compared to Ac-225-macropa-isotype-control (Figure 15). Ac-225-macropa-isotype-control had higher and longer blood circulation time than Ac-225-macropa-Codituzumab, while the normal tissue uptake was comparable for the two Ac-225-labelled antibodies (Figure 15).

### In vivo efficacy study comparing Ac-225-macropa-Codrituzumab and Ac-225-isotype-control

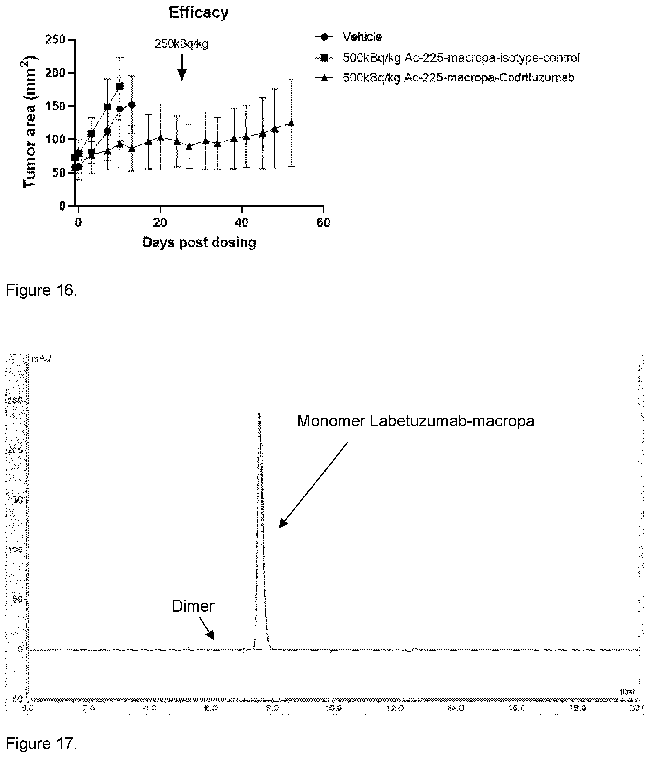

Codrituzumab-macropa and isotype-control-macropa antibody conjugates were labelled with Ac-225. Mice xenografted with Huh-7 hepatoma expressing GPC3 were treated with a single dose IV of 500 kBq/kg with a total antibody dose of 0.75 mg/kg, with a repeat dose of 250 kBq/kg on day 25 for the surviving animals. As presented in Figure 16, significantly higher anti-tumor activity was observed for Ac-225-macropa-Codrituzumab when compared to Ac-225-macropa-isotype control. No body weight loss was observed during the course of the study.

### Tissue-targeting compounds comprising Labetuzumab (CEACAM5)

CEACAM5, also known as CEA or carcinoembryonic antigen, is a cell surface glycoprotein that has gained significant attention in the field of cancer research. CEACAM5 is frequently overexpressed in various cancers, including colorectal, lung, breast, and pancreatic cancers. Its expression has been linked to the prognosis of certain cancers. Studies have shown that higher levels of CEACAM5 are associated with more aggressive tumor behavior, including increased invasiveness and metastasis. Due to its presence on the surface of tumor cells, CEACAM5 is an attractive target for antibody-based therapies. One advantage of targeting CEACAM5 is its relatively restricted expression in healthy tissues, predominantly limited to gastrointestinal epithelial cells during fetal development.

### ACC characterization by size exclusion chromatography and mass spectrometry

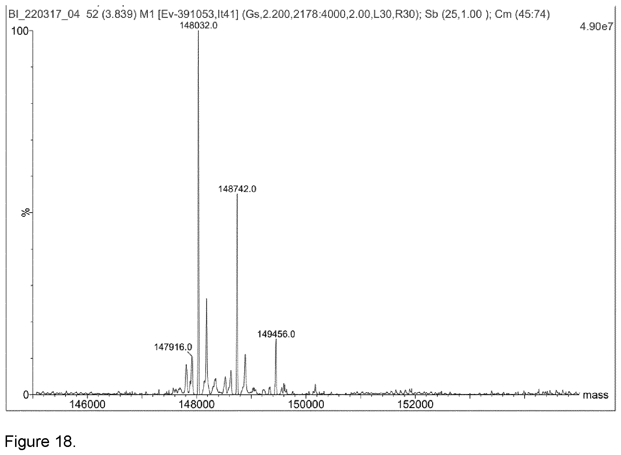

Purity (99.7%) and CAR (0.5) of Labetuzumab-macropa were determined by SEC-UV (Figure 17) and SEC-MS (Figure 18), respectively.

### Flow cytometry

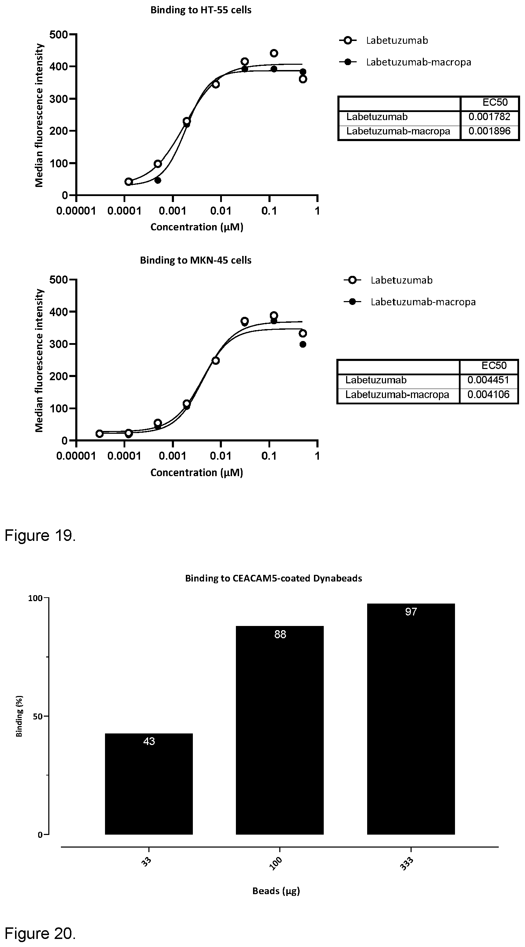

Cell surface binding of Labetuzumab-macropa was compared to unconjugated Labetuzumab and investigated by flow cytometry using MKN-45 and HT-55 cells expressing human CEACAM5 (Figure 19). Labetuzumab-macropa showed comparable EC50 as unconjugated Labetuzumab, indicating that binding capacity of the Labetuzumab-macropa was maintained after conjugation.

### Immunoreactive fraction (IRF)

Shortly after completion of the radiolabeling, the binding of each sample to CEACAM5-coated M270-carboxy Dynabeads was determined. The IRF was 43%, 88% and 97% for Ac-225-macropa-Labetuzumab when using 33 µg, 100 µg and 333 µg beads, respectively (Figure 20).

### Internalization

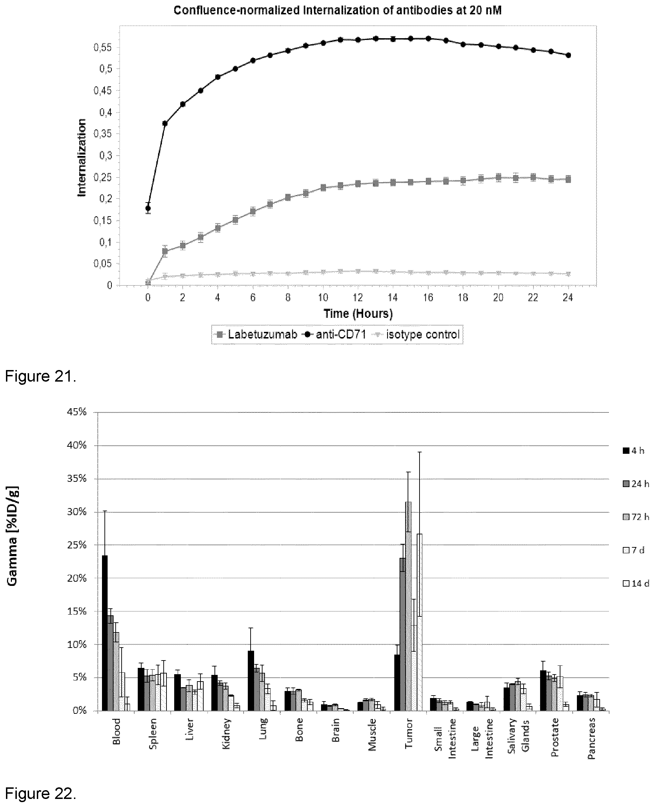

Antibodies are incubated with 2-fold molar excess of "Incucyte^{®} Human Fabfluor-pH Red Antibody Labeling Reagent (Sartorius CatNo #4722)" for 20 min at room temperature. Then, pre-incubated samples are added to seeded cells (20K c/w, 96-w plate, o/n incubation at 37oC in a 5% CO2-cell culture incubator) to a final concentration of 20 nM in duplicates. The plate is further incubated in the Incucyte^{®} S3 (37oC in a 5% CO2), and phase & red channel are imaged for 24 h in 1-h intervals with 2 images/well @20× magnification. The internalization is calculated as "confluence-normalized red fluorescence" (Figure 21).

### In vivo Biodistribution study of Ac-225-macropa Labetuzumab

Biodistribution of Ac-225-macropa-Labetuzumab was investigated in the CEACAM5-expressing colon carcinoma xenograft model HT-55. Female nude mice were inoculated with 1 × 10⁶ HT-55 cells on the right flank. Animals were pre-treated with 200 µg/mouse unspecific IgG2a antibody 24 h prior to hot dosing. Animals were treated with 250 kBq/kg Ac-225-macropa-Labetuzumab IV at a total protein dose of 0.43 mg/kg. Animals were sacrificed 4 hours, 1, 3, 7, and 14 days post treatment and Ac-225 in blood, tumor, liver, kidney, spleen, brain, muscle, small & large intestine, salivary glands, uterus & ovary, pancreas as well as femur was determined (Figure 22).

### Cell cytotoxicity

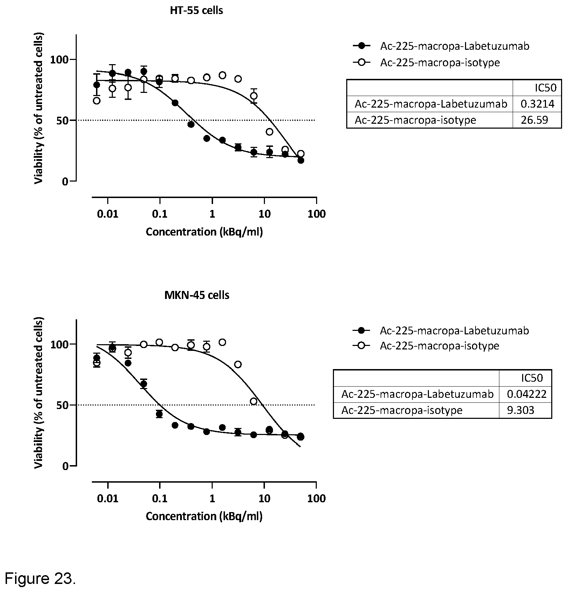

In-vitro cytotoxicity of Ac-225-macropa-Labetuzumab was measured in cell lines expressing different levels of CEACAM5. HT-55 and MKN-45 cells were seeded at an appropriate cell density in the following cell media respectively: MEM + 20% FBS + MEM-NEAA; RPMI + 20% FBS. One day after seeding, Ac-225-macropa-Labetuzumab at a specific activity of 2 kBq/µg was titrated in parallel with a relevant negative isotype control which was radiolabeled in the same conditions. Cells were treated for 6 days and subsequentially cell viability was determined using CellTiterGlo (Promega). Strong and specific cytotoxicity was observed in both cell lines (Figure 23).

### Tissue-targeting compounds comprising Sacituzumab (TROP2)

Trophoblast cell surface antigen 2 (TROP2) is overexpressed in different tumor tissues (breast, cervix, colorectal, esophagus, gastric and lung cancer) and plays an important role in tumor cell proliferation, apoptosis, and invasion, thereby impacting the prognosis and treatment of cancer patients. As a result, TROP2 has become an attractive therapeutic target in cancer treatment.

### ACC characterization by size exclusion chromatography and mass spectrometry

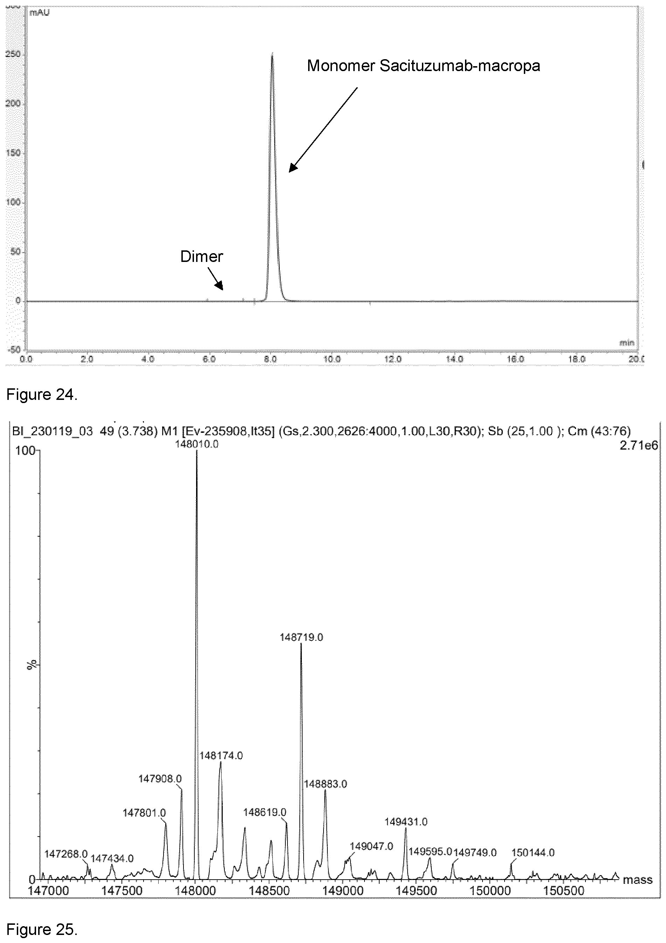

Purity (99.7%) and CAR (0.5) of Sacituzumab-macropa were determined by SEC-UV (Figure 24) and SEC-MS (Figure 25), respectively.

### Flow cytometry

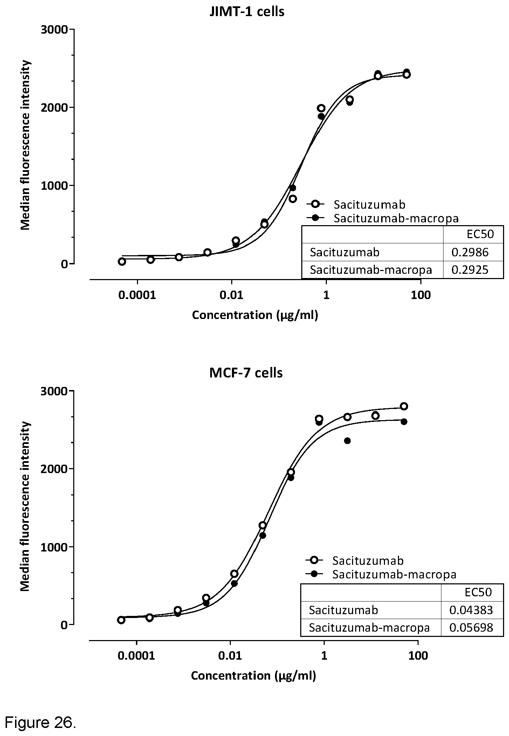

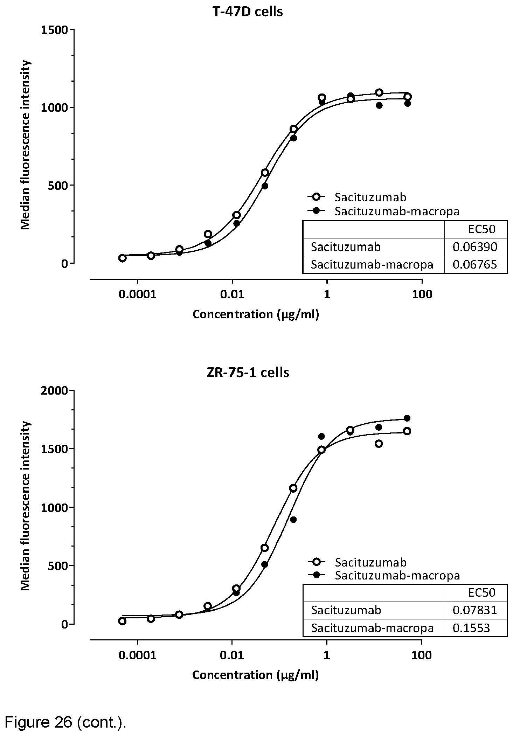

Cell surface binding of Sacituzumab-macropa was compared to unconjugated Sacituzumab and was investigated by flow cytometry using JIMT-1, MCF-7, T-47D and ZR-75-1 cells expressing human TROP2 (Figure 26). Sacituzumab-macropa showed comparable EC50 as unconjugated Sacituzumab, indicating that binding capacity of the Sacituzumab-macropa was maintained after conjugation.

### Cell cytotoxicity

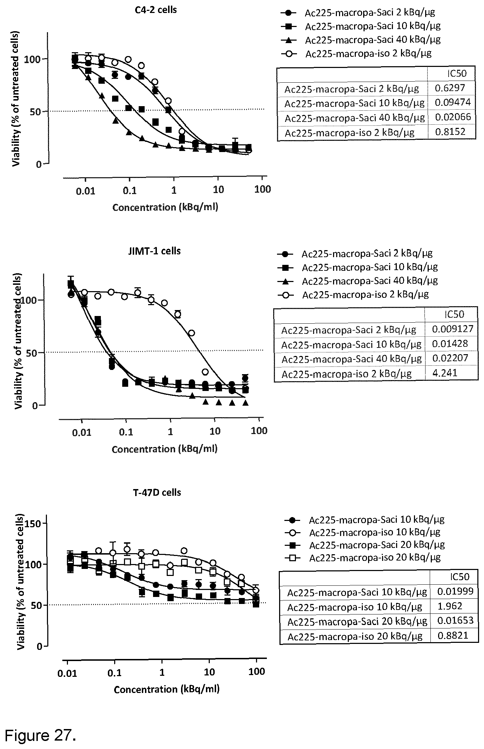

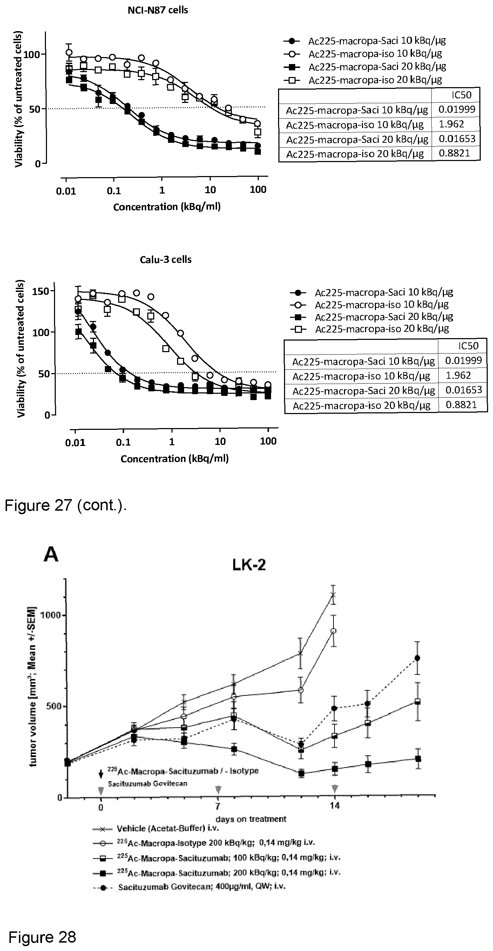

In-vitro cytotoxicity of Ac-225-macropa-Sacituzumab was determined in cell lines expressing different levels of TROP2. C4-2, JIMT-1, T-47D, NCI-N87 and Calu-3 were seeded at an appropriate cell density in the following cell media respectively: RPMI + 10% FBS + 1x MEM-NEAA + 1x HEPES + 1x Na Pyruvate; DMEM + 10% FBS; RPMI + 10% FBS + 10 µg/ml insulin; RPMI + 10% FBS; RPMI + 10% FBS. One day after seeding, Ac-225-macropa-Sacituzumab at a specific activity of 2 to 40 kBq/µg was titrated in parallel with a relevant negative isotype control which was radiolabeled in the same conditions. Cells were treated for 6 days and subsequentially cell viability was determined using CellTiterGlo (Promega). A strong and specific cytotoxicity was observed in all cell lines (Figure 27).

### In vivo antitumor efficacy of Ac-225-macropa Sacituzumab

The *In vivo* antitumor efficacy of Ac-225-macropa-Sacituzumab was evaluated in the LK-2 lung cancer model on mice. Female BALB/c nude mice were inoculated subcutaneously with 1×10⁶ LK-2 cells. When tumors reached an average size of ~190mm³, animals were randomized into treatment groups (n=12 per group) and treatment was started.

Animals received either vehicle, Ac-225-macropa-lsotype control (200 kBq/kg, total antibody dose 0.14 mg/kg, i.v., single dose day 0), Ac-225-macropa-Sacituzumab (100 or 200 kBq/kg, total antibody dose 0.14 mg/kg, i.v., single dose day 0), or Trodelvy (sacituzumab govitecan-hziy) (20 mg/kg; i.v., treatment on day 0,7,14).

Treatment with Ac-225-macropa-Sacituzumab resulted in strong tumor growth inhibition at both doses tested. Antitumor activity of Ac-225-macropa-Sacituzumab was significantly improved as compared to Trodelvy.

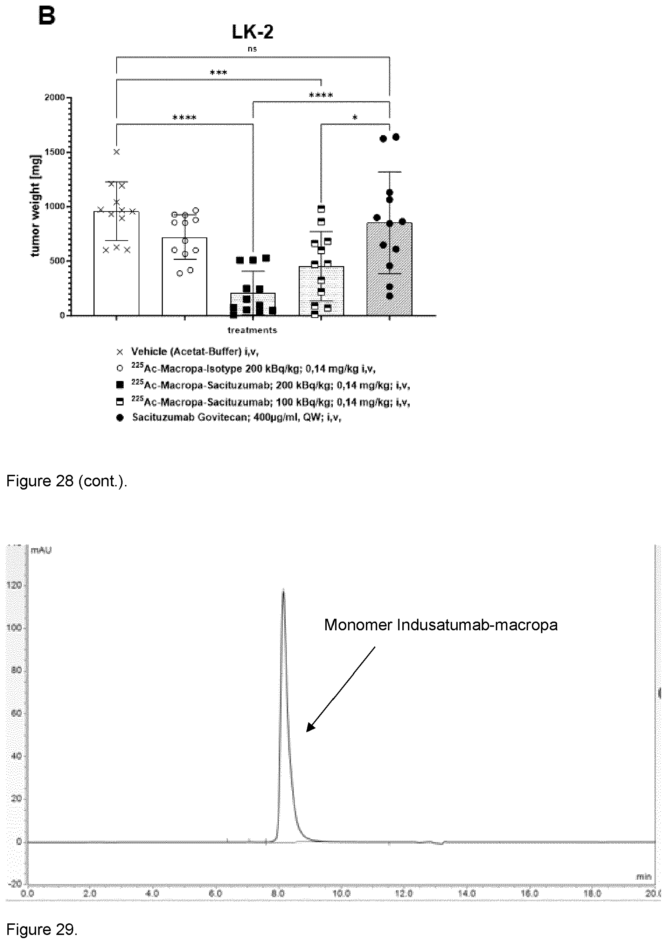

Statistical analyses were performed using one-way ANOVA followed by Sidak's multiple comparison test. (**, *P*=0.0024. ****, *P*<0.001) (Figure 28).

### Tissue-targeting compounds comprising Indusatumab (GUCY2C)

GUCY2C (guanylate cyclase 2C) is primarily expressed in the gastrointestinal tract and is elevated in various types of gastrointestinal cancers, including colorectal, gastric, and pancreatic cancers. This characteristic makes it an attractive target due to its specificity to tumor cells and limited expression in normal tissues. Its expression is associated with more aggressive tumor phenotypes, including increased invasiveness and metastasis, as well as poor patient prognosis.

### ACC characterization by size exclusion chromatography and mass spectrometry

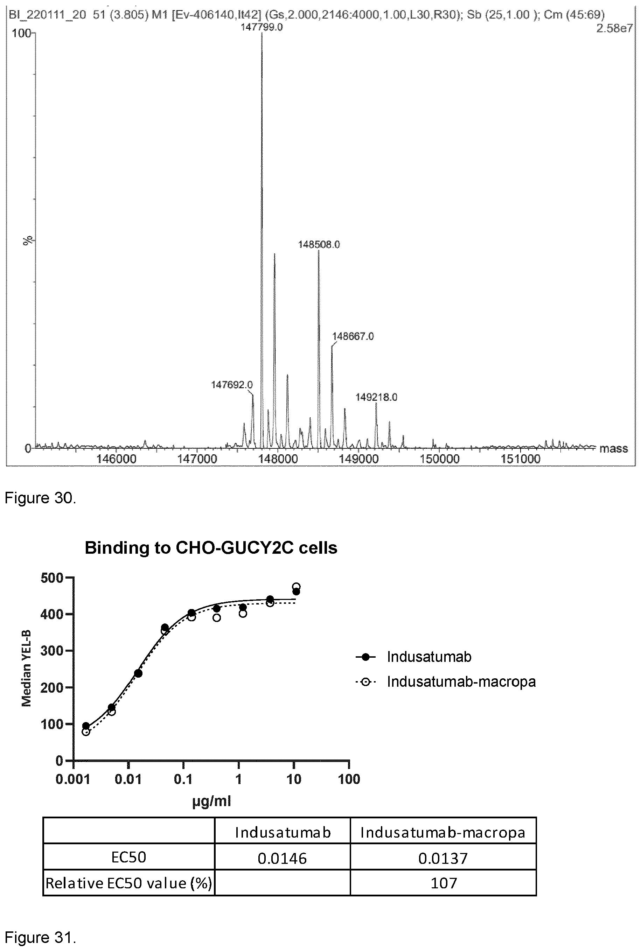

Purity (100%) and CAR (0.5) of Indusatumab-macropa were determined by SEC-UV (Figure 29) and SEC-MS (Figure 30), respectively.

### Flow cytometry

Cell surface binding of Indusatumab-macropa was compared to unconjugated Indusatumab and investigated by flow cytometry using CHO-GUCY2C cells expressing human GUCY2C (Figure 31). Indusatumab-macropa showed comparable EC50 as unconjugated Indusatumab, indicating that binding capacity of the Indusatumab-macropa was maintained after conjugation.

### Immunoreactive fraction (IRF)

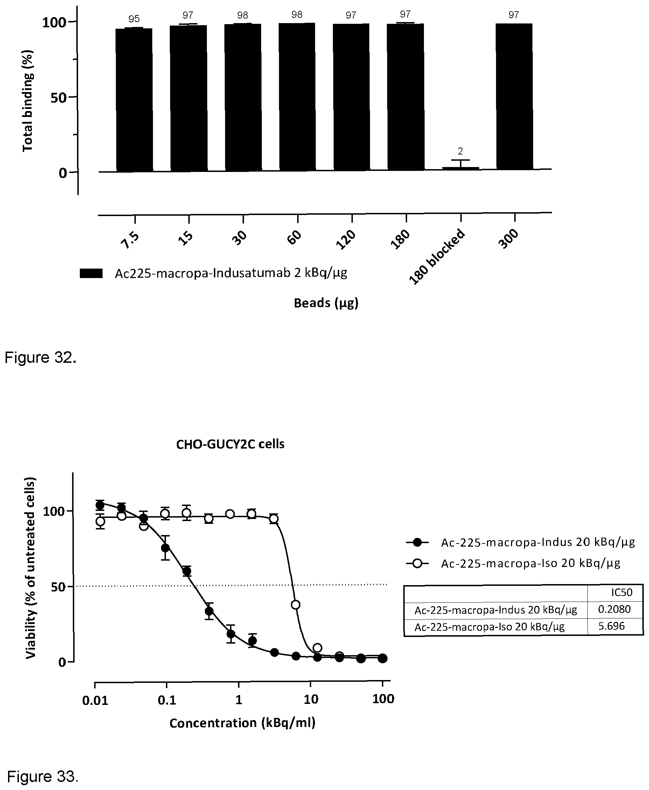

After completion of the radiolabeling, the binding Ac225-macropa-Indusatumab to GUCY2C-coated M270-carboxy Dynabeads was determined by IRF (as describe above). Maximal binding was achieved with as little as 7.5 µg GUCY2C-coated beads. The specificity was demonstrated by the presence of a sample blocked with an excess of naked antibody (Figure 32).

### Cell cytotoxicity

In-vitro cytotoxicity of Ac-225-macropa-lndusatumab was determined in CHO cells stably expressing GUCY2C. The cells were seeded at an appropriate cell density in MEM Alpha + 10% FBS + 1x Glutamax + 0.5 mg/ml G418. One day after seeding, Ac-225-macropa-Indusatumab at a specific activity of 20 kBq/µg was titrated in parallel with a relevant negative isotype control which was radiolabeled in the same conditions. Cells were treated for 6 days and subsequentially cell viability was determined using CellTiterGlo (Promega). A strong and specific cytotoxicity was observed (Figure 33).

### In vivo Biodistribution study of Ac-225-macropa

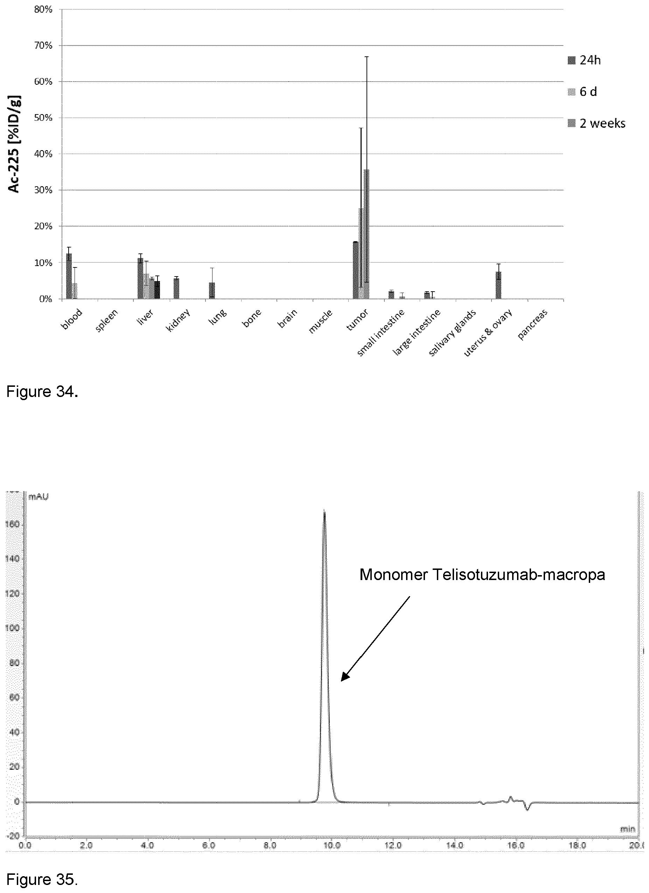

Biodistribution of Ac-225-macropa-lndusatumab was investigated in the GUCY2C-expressing lung metastasis of a colon carcinoma xenograft model T84. Female NODscid mice were inoculated with 1 × 10⁶ T84 cells on the right flank. Animals were pre-treated with 200 µg/mouse unspecific IgG2a antibody 24 h prior to hot dosing. Animals were treated with 500 kBq/kg Ac-225-macropa- Indusatumab IV at a total protein dose of 1.4 mg/kg. Animals were sacrificed 1, 6, and 14 days post treatment and Ac-225 in blood, tumor, liver, kidney, spleen, brain, muscle, small & large intestine, salivary glands, uterus & ovary, pancreas as well as femur was determined (Figure 34).

### Tissue-targeting compounds comprising Telisotuzumab (c-Met)

c-Met (mesenchymal-epithelial transition factor) is a receptor tyrosine kinase for hepatocyte growth factor (HGF)/Scatter Factor (SF). Normal functions of the c-Met pathway are largely restricted to organ morphogenesis during development as well as to tissue damage repair and regeneration in adults. In normal adult tissues expression is generally low. c-Met is a well-known target and has been found to be aberrantly activated in human cancers via mutation, amplification, or protein overexpression. It is highly expressed in multiple cancers, including lung adeno (38%), lung squamous (11%), CRC (23%), RCC (54-78%), melanoma (20%), mesothelioma (46%), thyroid (74%), gastric cancer (12%), pancreatic cancer (21%) and oesophageal cancer (22%). c-Met is an oncogenic driver in some patients, making it less likely to develop resistance through loss of target expression. c-Met expression on the cell membrane makes it an accessible target for targeting modalities binding to c-Met.

### ACC characterization by size exclusion chromatography and mass spectrometry

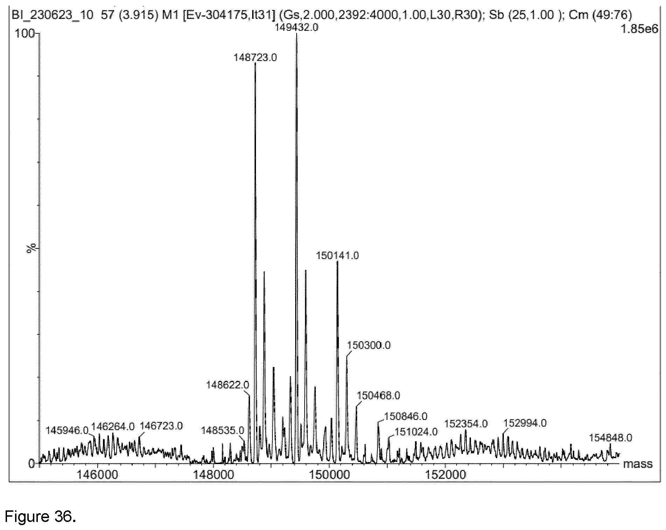

Purity (100%) and CAR (0.9) of Telisotuzumab-macropa were determined by SEC-UV (Figure 35) and SEC-MS (Figure 36), respectively.

### Flow cytometry

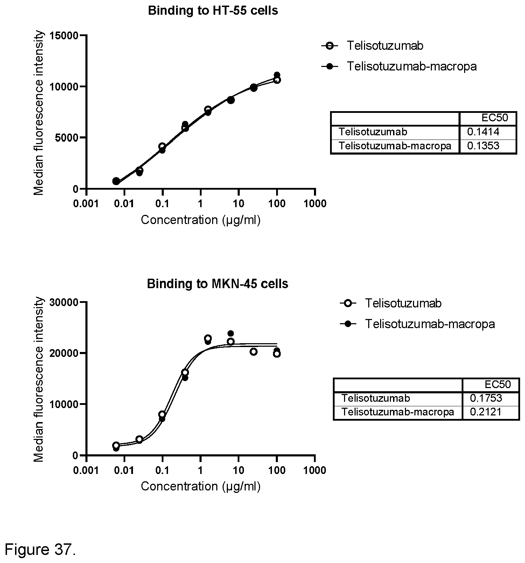

Cell surface binding of Telisotuzumab-macropa was compared to unconjugated Telisotuzumab and investigated by flow cytometry using MKN-45 and HT-55 cells expressing human c-Met (Figure 37). Telisotuzumab-macropa showed comparable EC50 in both cell lines as unconjugated Telisotuzumab, indicating that binding capacity of the Telisotuzumab-macropa was maintained after conjugation.

### Cell cytotoxicity

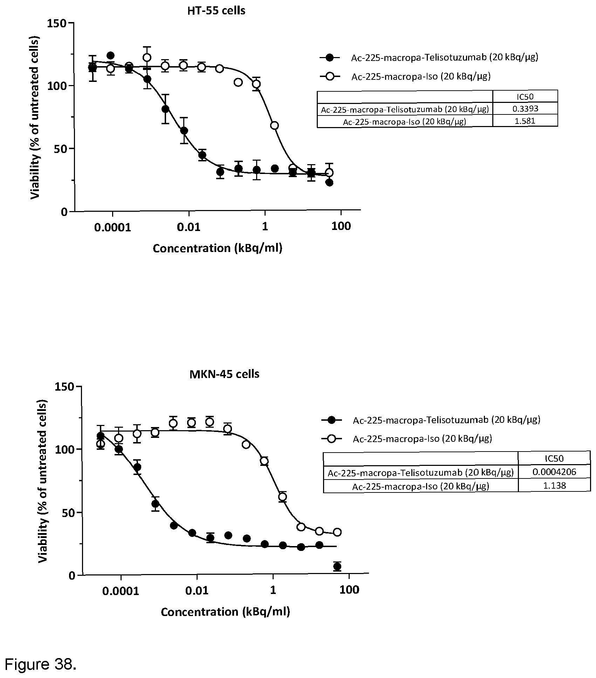

In-vitro cytotoxicity of Ac-225-macropa-Telisotuzumab was determined in MKN-45 and HT-55 cells. HT-55 cells were at an appropriate cell density in MEM + 20% FBS + 1x MEM-NEAA. MKN-45 cells were at an appropriate cell density in RPMI + 20% FBS. One day after seeding, Ac-225-macropa-Telisotuzumab at a specific activity of 20 kBq/µg was titrated from 50 kBq/ml in parallel with a relevant negative isotype control which was radiolabeled in the same conditions. At day 6 cell viability was determined using CellTiterGlo (Promega). A significant and specific cytotoxicity was observed on both cell lines (Figure 38).

### Tissue-targeting compounds comprising Farletuzumab (FolRa)

FOLR (folate receptor alpha) is an internalizing GPI-anchored membrane protein. FOLR1 is responsible for binding to folic acid and its derivatives, which becomes crucial during fetal development. By adding folate supplementation during pregnancy, neural tube defects in the fetus are prevented. Anti folates are used for treatment of various tumor indications. High FOLR1 levels are associated with poor differentiation, aggressive tumors & resistance to conventional chemotherapy. FOLR1 is a clinical validated target through the approval of mirvetuximab soravtansine. Elevated FOLR1 expression levels are seen in ovarian serous cystadenocarcinoma and a subset of breast invasive carcinoma, lung adenocarcinoma, and endometrial cancer.

### ACC characterization by size exclusion chromatography and mass spectrometry

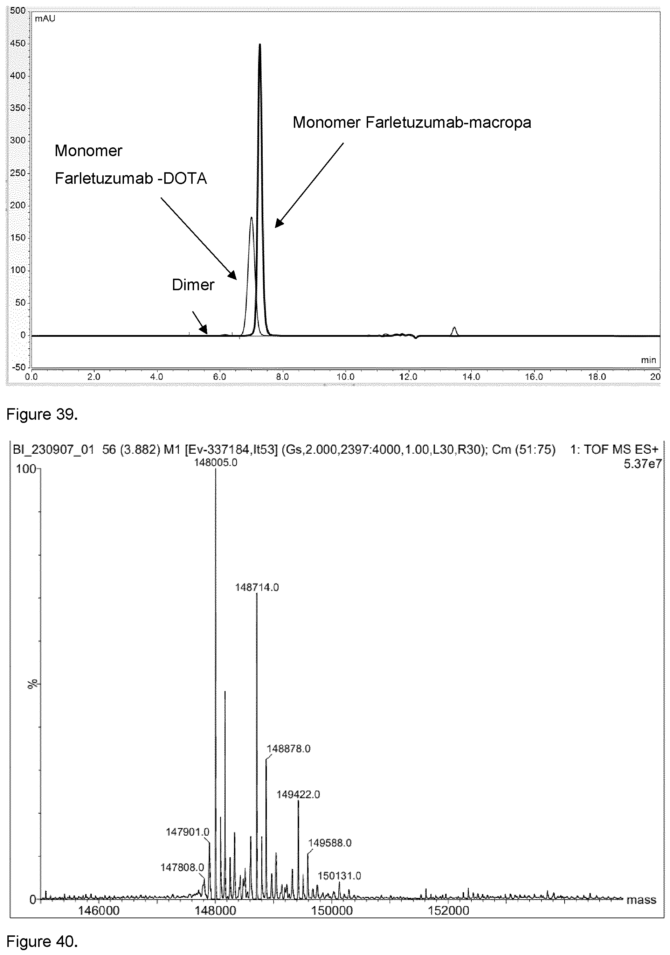

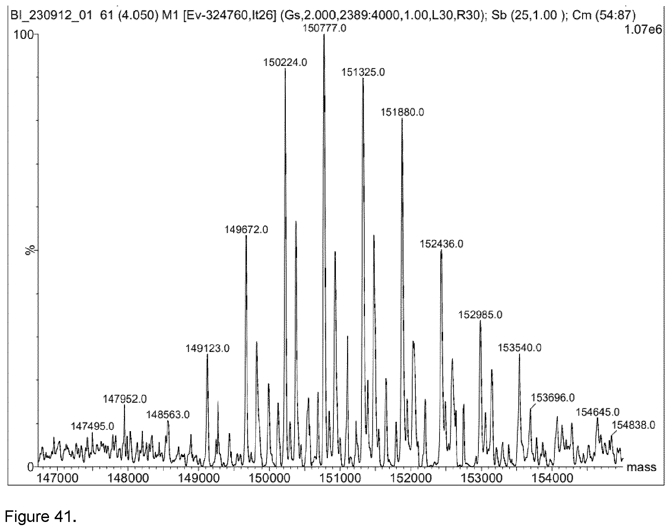

Purity (99.8%) and CAR (0.7) of Farletuzumab-macropa were determined by SEC-UV (Figure 39) and SEC-MS (Figure 40), respectively. Purity (98.8%) and CAR (5.8) of Farletuzumab-DOTA were determined by SEC-UV (Figure 39) and SEC-MS (Figure 41), respectively.

### Flow cytometry

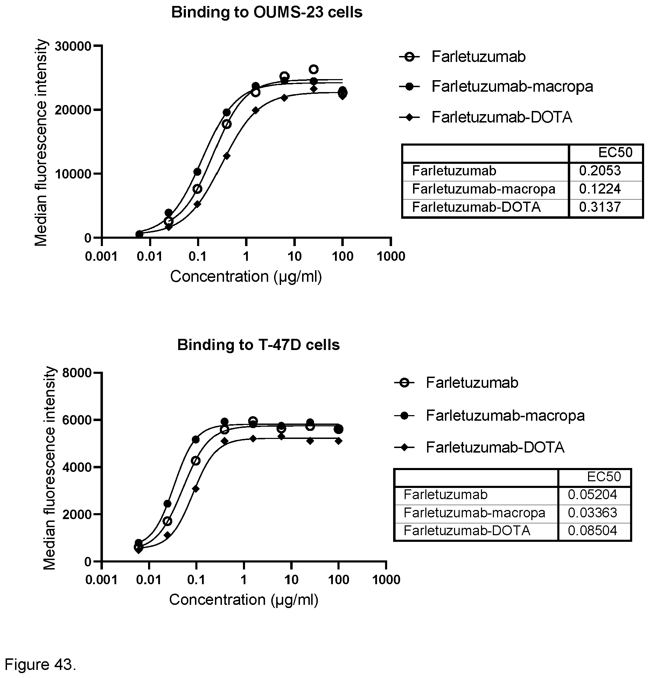

Cell surface binding of Farletuzumab-macropa and Farletuzumab-DOTA was compared to unconjugated Farletuzumab and investigated by flow cytometry using OUMS-23 and T-47D cells expressing human FolRa (Figure 43). Farletuzumab-macropa showed comparable EC50 in both cell lines as unconjugated Farletuzumab, indicating that binding capacity of the Farletuzumab-macropa was maintained after conjugation whereas Farletuzumab-DOTA showed higher EC50 than unconjugated Farletuzumab in both cell lines, indicating that binding capacity of the Farletuzumab-DOTA was reduced after conjugation.

### Cell cytotoxicity

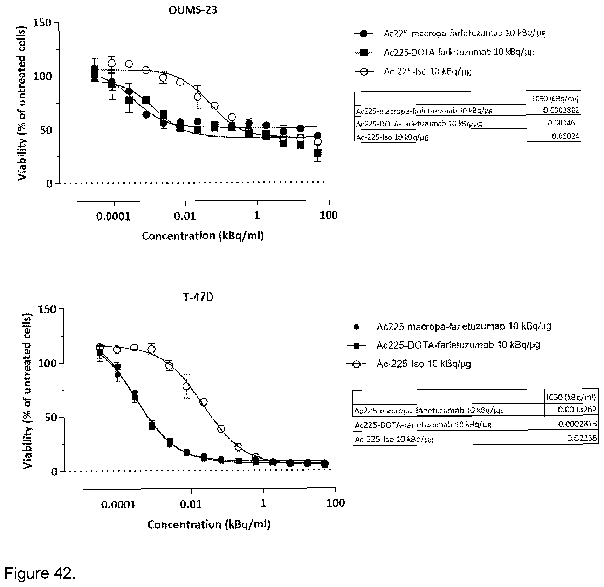

*In vitro* cytotoxicity of Ac-225-macropa-Farletuzumab was determined in cell lines expressing different levels of FOLR1. OUMS-23 and T-47D cells were seeded at an appropriate cell density in the cell media DMEM + 10% FBS + 1% P/S for OUMS-23 and RPMI + 10% FBS + 1% P/S + 10 pg/ml Insulin for T-47D. Six hours after seeding the compounds at the specific activity of 10 MBq/mg and radioactive concentration of 50 kBq/ml were titrated in parallel with a radiolabeled isotype control. Cells were incubated for 6 days and subsequentially cell viability was determined using Hoechst and PI staining and analyzed on Operetta CLS High Content Analysis System (Perkin Elmer). Specific cytotoxicity was observed in both cell lines (Figure 42).

### Tissue-targeting compounds comprising Mirvetuximab (FoIRa)

### ACC characterization by size exclusion chromatography and mass spectrometry

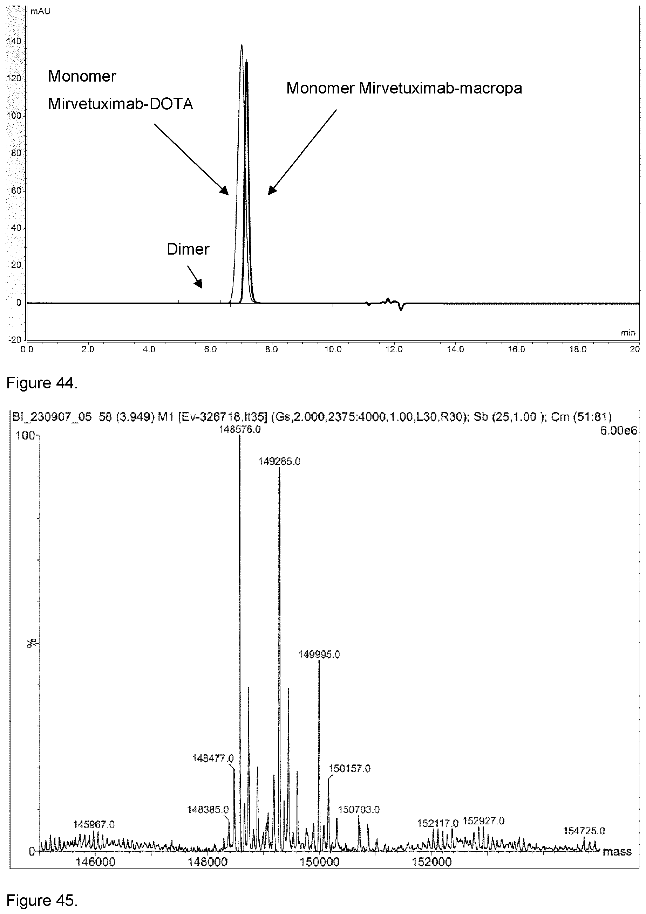

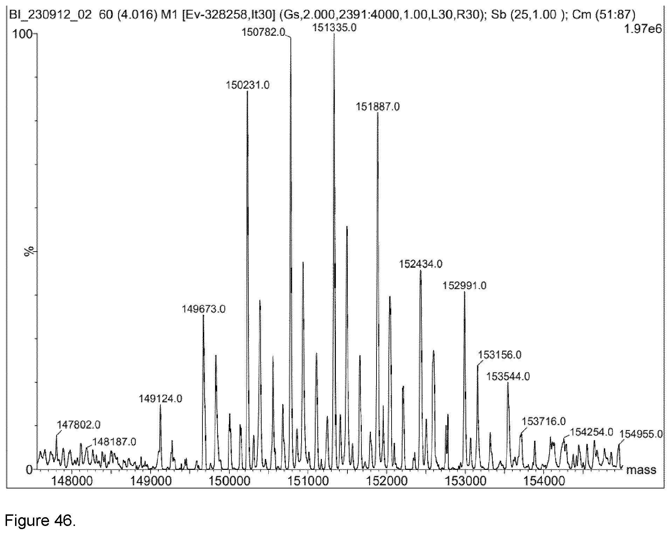

Purity (100%) and CAR (0.9) of Mirvetuximab-macropa were determined by SEC-UV (Figure 44) and SEC-MS (Figure 45), respectively. Purity (99.9%) and CAR (5.0) of Mirvetuximab-DOTA were determined by SEC-UV (Figure 44) and SEC-MS (Figure 46), respectively.

### Flow cytometry

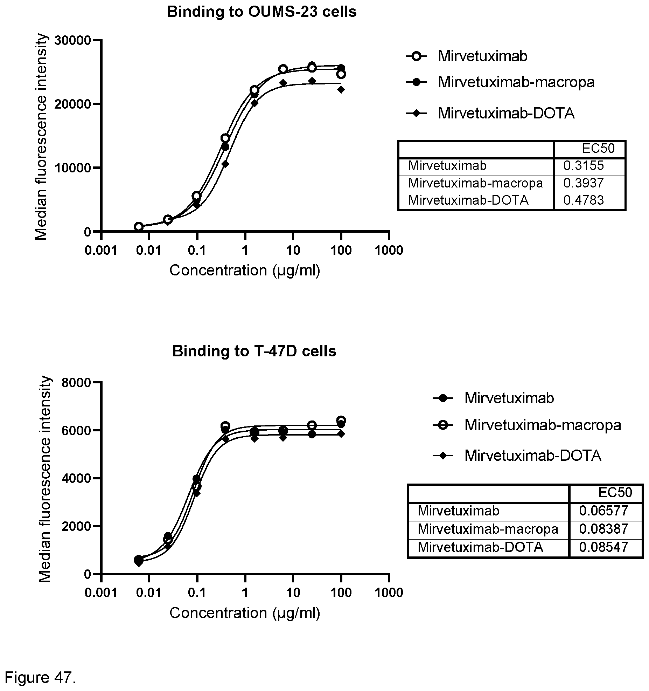

Cell surface binding of Mirvetuximab-macropa and Mirvetuximab-DOTA was compared to unconjugated Mirvetuximab and investigated by flow cytometry using OUMS-23 and T-47D cells expressing human FolRa (Figure 47). Mirvetuximab-macropa showed comparable EC50 in both cell lines as unconjugated Mirvetuximab, indicating that binding capacity of the Mirvetuximab-macropa was maintained after conjugation, whereas Mirvetuximab-DOTA showed higher EC50 than unconjugated Mirvetuximab in OUMS-23 cells, indicating that binding capacity of the Mirvetuximab-DOTA was reduced after conjugation.

### Cell cytotoxicity

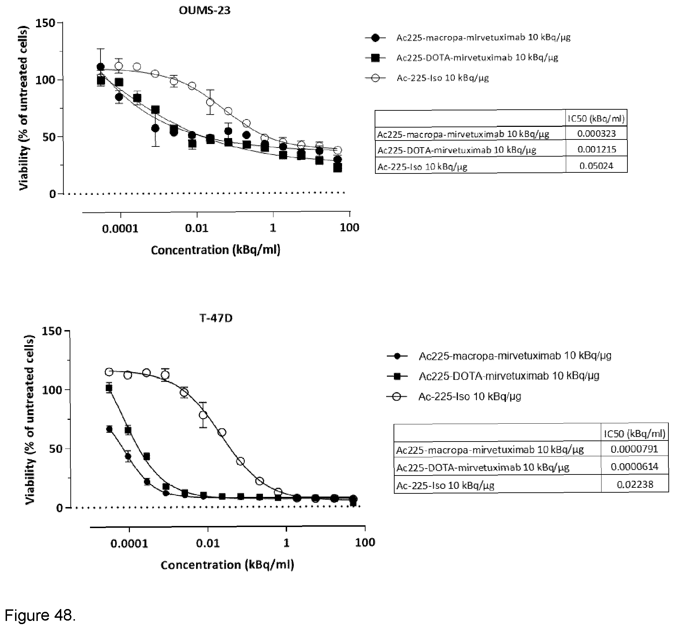

*In vitro* cytotoxicity of Ac-225-macropa-Mirvetuximab was determined in cell lines expressing different levels of FOLR1. OUMS-23 and T-47D cells were seeded at an appropriate cell density in the cell media DMEM + 10% FBS + 1% P/S for OUMS-23 and RPMI + 10% FBS + 1% P/S + 10 pg/ml Insulin for T-47D. Six hours after seeding the compounds at the specific activity of 10 MBq/mg and radioactive concentration of 50 kBq/ml were titrated in parallel with a radiolabeled isotype control. Cells were incubated for 6 days and subsequentially cell viability was determined using Hoechst and PI staining and analyzed on Operetta CLS High Content Analysis System (Perkin Elmer). Specific cytotoxicity was observed in both cell lines (Figure 48).

### Tissue-targeting compounds comprising Rovalpituzumab (DLL3)

### ACC characterization by size exclusion chromatography and mass spectrometry

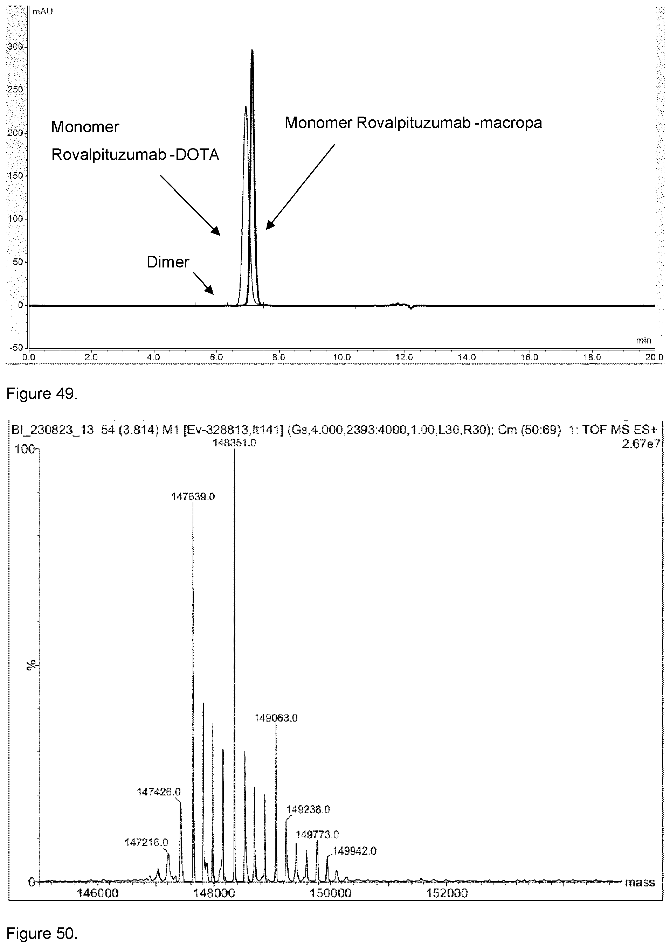

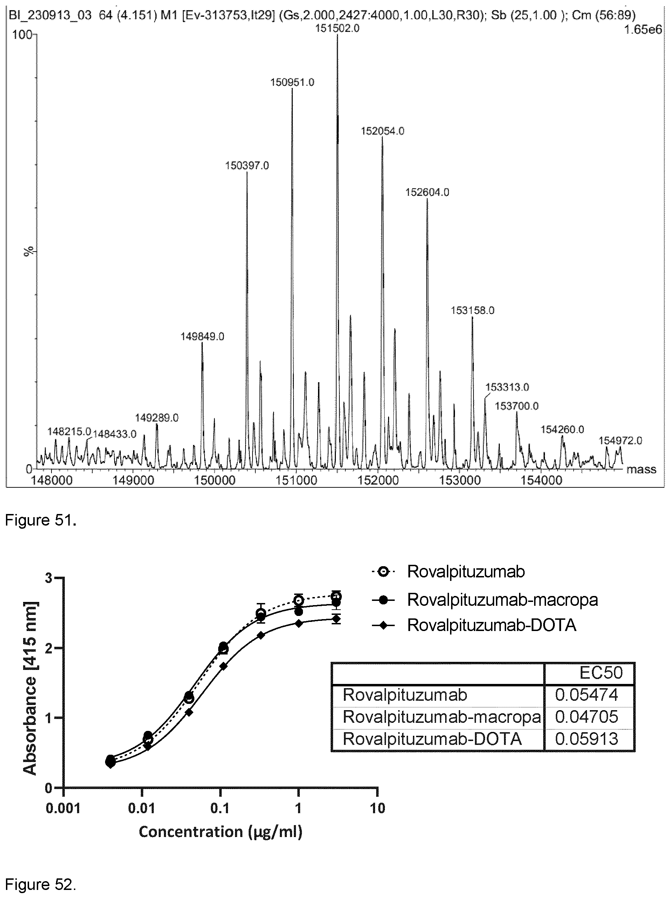

Purity (99.5%) and CAR (0.9) of Rovalpituzumab-macropa were determined by SEC-UV (chromatogram shown in Figure 49) and SEC-MS (Figure 50), respectively. Purity (99.9%) and CAR (7.1) of Rovalpituzumab-DOTA were determined by SEC-UV (chromatogram shown in Figure 49) and SEC-MS (Figure 51), respectively.

### ELISA

The binding potency of Rovalpituzumab-macropa, Rovalpituzumab-DOTA was compared to unconjugated Rovalpituzumab by using an ELISA assay. The plate was coated with human recombinant DLL3 antigen and blocked before incubation with ACCs and antibody. Slightly lower max absorbance value and increased EC50 were seen Rovalpituzumab-DOTA, indication that binding property of Rovalpituzumab-DOTA was slightly reduced after conjugation (Figure 52).

### Immunoreactive fraction (IRF)

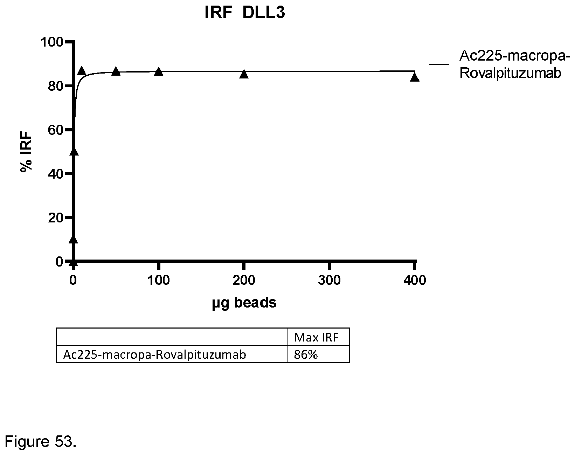

Shortly after radiolabeling, binding to of Ac-225-Macropa-Rovalpituzumab (20 kBq/µg) to recombinant human DLL3 coated M-270-carboxy Dynabeads was determined by IRF assay (as described above). The maximum binding of Ac-225-macropa-Rovalpituzumab (86%) was achieved with 10 µg of DLL3-coated beads (Figure 53).

### Cell cytotoxicity

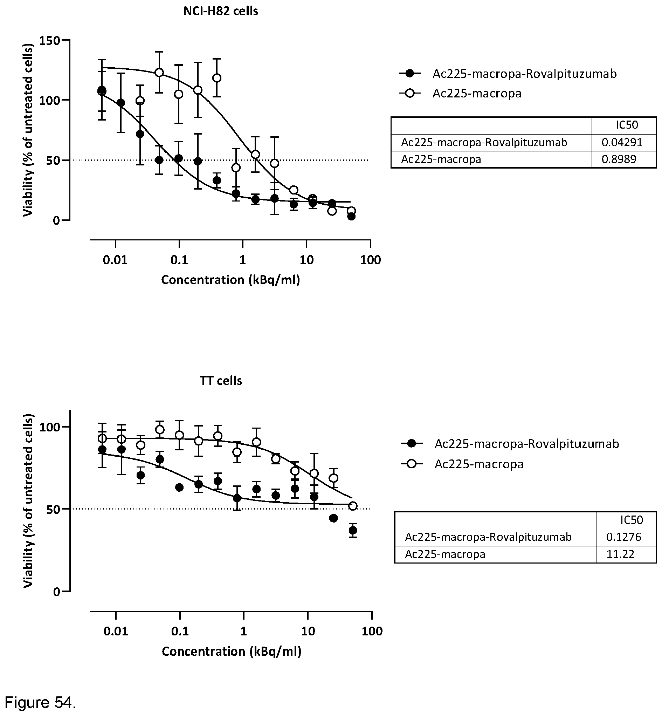

In-vitro cytotoxicity of Ac-225-macropa-Rovalpituzumab was determined in DLL3 expressing NCI-H82 and TT cells. The cells were seeded at an appropriate cell density in RPMI + 10% FBS and Ham's F-12K + 10 % FBS respectively. One day after seeding, Ac-225-macropa-Rovalpituzumab at a specific activity of 20 kBq/µg was titrated in parallel with a relevant negative Ac-225-macropa control which was radiolabeled in the same conditions. Cells were treated for 6 days and subsequentially cell viability was determined using CellTiterGlo (Promega). A strong and specific cytotoxicity was observed (Figure 54).

### Chelating bismuth-213 with pelgifatamab-macropa compared to pelgifatamab-DOTA

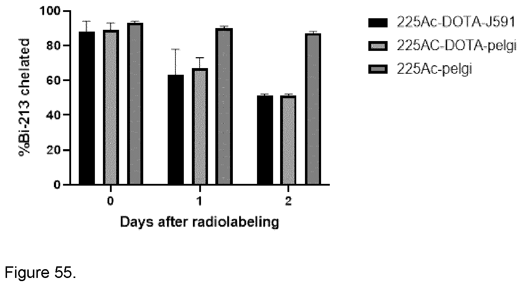

The PSMA-targeted actinium-225 conjugate ²²⁵Ac-pelgi (BAY 3546828) consisting of the alpha-particle emitter actinium-225 complexed to a macropa chelator covalently linked to the fully human PSMA-targeting antibody pelgifatamab (CAR 0.8), the murine monoclonal PSMA antibody J591 (CAR 12.7) and pelgifatamab-DOTA (CAR 12.7) were labeled with Actinium-225 in 0.1 M acetate buffer (pH 5). Pelgi-macropa labeling was performed for 60 min at room temperature the two DOTA compounds were labeled at 60°C for 60 min. The percentage chelation of Bi-213 was determined using iTLC and measured on the Germanium Detector using the specific energy of Bi-213. After initial radiolabelling, compounds were measured immediately and on Day 1 and Day 2 after incubation at room temperature. It was found that Bismuth-213 was chelated by macropa whereas Bismuth-213 chelation was not chelated to the same extent with DOTA (Figure 55). Since this is a feature that depends on the chelating nature of macropa, the same advantageous 213Bi chelation properties can be found in the compounds of formula (I) of the present invention.

## Claims

1. A tissue-targeting compound of formula (I) 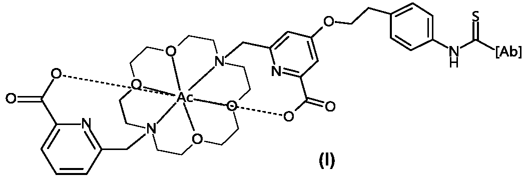 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1.

2. The tissue-targeting compound of formula (I) according to claim 1, wherein [Ab] is bound to the rest of the molecule via a terminal amino group of a Lysine residue of the monoclonal antibody or antigen-binding fragment thereof.

3. The tissue-targeting compound of formula (I) according to any of claims 1 or 2, wherein the monoclonal antibody [Ab] is Trastuzumab or an antigen-binding fragment thereof (HER2), Codrituzumab or an antigen-binding fragment thereof (GPC3), Labetuzumab or an antigen-binding fragment thereof (CEACAM5), Sacituzumab or an antigen-binding fragment thereof (TROP2), Indusatumab or an antigen-binding fragment thereof (GUCY2C), Telisotuzumab or an antigen-binding fragment thereof (cMet), Farletuzumab or an antigen-binding fragment thereof (FolRa), Mirvetuximab or an antigen-binding fragment thereof (FolRa), Rovalpituzumab or an antigen-binding fragment thereof (DLL3), Enfortumab or an antigen-binding fragment thereof (Nectin-4) or Vandortuzumab or an antigen-binding fragment thereof (STEAP-1).

4. The tissue-targeting compound of formula (I) according to any of claims 1 to 3, wherein [Ab] is Trastuzumab or an antigen-binding fragment thereof comprising at least the three CDR heavy chain sequences according to SEQ ID NO. 1, SEQ ID NO. 2 and SEQ ID NO. 3 and the three CDR light chain sequences according to SEQ ID NO. 4, SEQ ID NO. 5 and SEQ ID NO. 6., Labetuzumab or an antigen-binding fragment thereof comprising at least the three CDR heavy chain sequences according to SEQ ID NO. 7, SEQ ID NO. 8 and SEQ ID NO. 9 and the three CDR light chain sequences according to SEQ ID NO. 10, SEQ ID NO. 11 and SEQ ID NO. 12., Sacituzumab or an antigen-binding fragment thereof comprising at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18., Indusatumab or an antigen-binding fragment thereof comprising at least the three CDR heavy chain sequences according to SEQ ID NO. 19, SEQ ID NO. 20 and SEQ ID NO. 21 and the three CDR light chain sequences according to SEQ ID NO. 22, SEQ ID NO. 23 and SEQ ID NO. 24., Telisotuzumab or an antigen-binding fragment thereof comprising at least the three CDR heavy chain sequences according to SEQ ID NO. 25, SEQ ID NO. 26 and SEQ ID NO. 27 and the three CDR light chain sequences according to SEQ ID NO. 28, SEQ ID NO. 29 and SEQ ID NO. 30., Farletuzumab or an antigen-binding fragment thereof comprising at least the three CDR heavy chain sequences according to SEQ ID NO. 31, SEQ ID NO. 32 and SEQ ID NO. 33 and the three CDR light chain sequences according to SEQ ID NO. 34, SEQ ID NO. 35 and SEQ ID NO. 36., Mirvetuximab or an antigen-binding fragment thereof comprising at least the three CDR heavy chain sequences according to SEQ ID NO. 37, SEQ ID NO. 38 and SEQ ID NO. 39 and the three CDR light chain sequences according to SEQ ID NO. 40, SEQ ID NO. 41 and SEQ ID NO. 42., Rovalpituzumab or an antigen-binding fragment thereof comprising at least the three CDR heavy chain sequences according to SEQ ID NO. 43, SEQ ID NO. 44 and SEQ ID NO. 45 and the three CDR light chain sequences according to SEQ ID NO. 46, SEQ ID NO. 47 and SEQ ID NO. 48., Enfortumab or an antigen-binding fragment thereof comprising at least the three CDR heavy chain sequences according to SEQ ID NO. 49, SEQ ID NO. 50 and SEQ ID NO. 51 and the three CDR light chain sequences according to SEQ ID NO. 52, SEQ ID NO. 53 and SEQ ID NO. 54, Vandortuzumab or an antigen-binding fragment thereof comprising at least the three CDR heavy chain sequences according to SEQ ID NO. 55, SEQ ID NO. 56 and SEQ ID NO. 57 and the three CDR light chain sequences according to SEQ ID NO. 58, SEQ ID NO. 59 and SEQ ID NO. 60 or Codrituzumab or an antigen-binding fragment thereof comprising at least the three CDR heavy chain sequences according to SEQ ID NO. 61, SEQ ID NO. 62 and SEQ ID NO. 63 and the three CDR light chain sequences according to SEQ ID NO. 64, SEQ ID NO. 65 and SEQ ID NO. 66.

5. A process for the preparation of a tissue-targeting compound of formula (I) 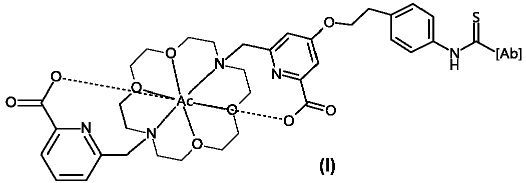
wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1,
said method comprising
coupling a chelating moiety of formula (II) 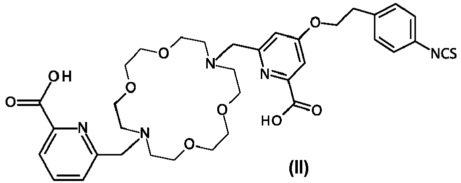
with a monoclonal antibody or an antigen-binding fragment thereof having binding affinity for HER2, GPC3, CEACAM5, TROP2, GUCY2C, cMet, FolRa, DLL3, Nectin-4 or STEAP1, and
contacting the resulting product with an aqueous solution comprising an ion of at least one alpha-emitting actinium isotope.

6. A tissue-targeting chelator of formula (III) 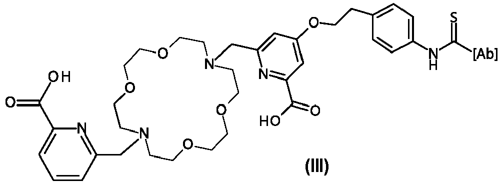 wherein [Ab] is a monoclonal antibody or an antigen-binding fragment thereof having binding affinity for HER2, GPC3, CEACAM5, TROP2, GUCY2C, cMet, FolRa, DLL3, Nectin-4 or STEAP1.

7. The tissue-targeting chelator of formula (III) according to claim 6, wherein [Ab] is Trastuzumab or an antigen-binding fragment thereof comprising at least the three CDR heavy chain sequences according to SEQ ID NO. 1, SEQ ID NO. 2 and SEQ ID NO. 3 and the three CDR light chain sequences according to SEQ ID NO. 4, SEQ ID NO. 5 and SEQ ID NO. 6., Labetuzumab or an antigen-binding fragment thereof comprising at least the three CDR heavy chain sequences according to SEQ ID NO. 7, SEQ ID NO. 8 and SEQ ID NO. 9 and the three CDR light chain sequences according to SEQ ID NO. 10, SEQ ID NO. 11 and SEQ ID NO. 12., Sacituzumab or an antigen-binding fragment thereof comprising at least the three CDR heavy chain sequences according to SEQ ID NO. 13, SEQ ID NO. 14 and SEQ ID NO. 15 and the three CDR light chain sequences according to SEQ ID NO. 16, SEQ ID NO. 17 and SEQ ID NO. 18., Indusatumab or an antigen-binding fragment thereof comprising at least the three CDR heavy chain sequences according to SEQ ID NO. 19, SEQ ID NO. 20 and SEQ ID NO. 21 and the three CDR light chain sequences according to SEQ ID NO. 22, SEQ ID NO. 23 and SEQ ID NO. 24., Telisotuzumab or an antigen-binding fragment thereof comprising at least the three CDR heavy chain sequences according to SEQ ID NO. 25, SEQ ID NO. 26 and SEQ ID NO. 27 and the three CDR light chain sequences according to SEQ ID NO. 28, SEQ ID NO. 29 and SEQ ID NO. 30., Farletuzumab or an antigen-binding fragment thereof comprising at least the three CDR heavy chain sequences according to SEQ ID NO. 31, SEQ ID NO. 32 and SEQ ID NO. 33 and the three CDR light chain sequences according to SEQ ID NO. 34, SEQ ID NO. 35 and SEQ ID NO. 36., Mirvetuximab or an antigen-binding fragment thereof comprising at least the three CDR heavy chain sequences according to SEQ ID NO. 37, SEQ ID NO. 38 and SEQ ID NO. 39 and the three CDR light chain sequences according to SEQ ID NO. 40, SEQ ID NO. 41 and SEQ ID NO. 42., Rovalpituzumab or an antigen-binding fragment thereof comprising at least the three CDR heavy chain sequences according to SEQ ID NO. 43, SEQ ID NO. 44 and SEQ ID NO. 45 and the three CDR light chain sequences according to SEQ ID NO. 46, SEQ ID NO. 47 and SEQ ID NO. 48., Enfortumab or an antigen-binding fragment thereof comprising at least the three CDR heavy chain sequences according to SEQ ID NO. 49, SEQ ID NO. 50 and SEQ ID NO. 51 and the three CDR light chain sequences according to SEQ ID NO. 52, SEQ ID NO. 53 and SEQ ID NO. 54, Vandortuzumab or an antigen-binding fragment thereof comprising at least the three CDR heavy chain sequences according to SEQ ID NO. 55, SEQ ID NO. 56 and SEQ ID NO. 57 and the three CDR light chain sequences according to SEQ ID NO. 58, SEQ ID NO. 59 and SEQ ID NO. 60 or Codrituzumab or an antigen-binding fragment thereof comprising at least the three CDR heavy chain sequences according to SEQ ID NO. 61, SEQ ID NO. 62 and SEQ ID NO. 63 and the three CDR light chain sequences according to SEQ ID NO. 64, SEQ ID NO. 65 and SEQ ID NO. 66.

8. Use of a tissue-targeting chelator of formula (III) according to any of claims 6 or 7 for the preparation of a tissue-targeting compound of formula (I) according to any of claims 1 to 4.

9. A tissue-targeting compound of formula (I) according to any of claims 1 to 4 for use in the treatment of disease, preferably wherein the disease is a hyperproliferative disease, more preferably wherein the hyperproliferative disease is cancer, more preferably wherein the hyperproliferative disease is bladder, brain, breast, colon, liver, lung, pancreatic, prostate, HNSCC or stomach cancer.

10. Use of a tissue-targeting compound of formula (I) according to any of claims 1 to 4 for the treatment of disease, preferably wherein the disease is a hyperproliferative disease, more preferably wherein the hyperproliferative disease is cancer, more preferably wherein the hyperproliferative disease is bladder, brain, breast, colon, liver, lung, pancreatic, prostate, HNSCC or stomach cancer.

11. Use of a tissue-targeting compound of formula (I) according to any of claims 1 to 4 for the preparation of a medicament for the treatment of disease, preferably wherein the disease is a hyperproliferative disease, more preferably wherein the hyperproliferative disease is cancer, more preferably wherein the hyperproliferative disease is bladder, brain, breast, colon, liver, lung, pancreatic, prostate, HNSCC or stomach cancer.

12. A method of treatment of disease, preferably wherein the disease is a hyperproliferative disease, more preferably wherein the hyperproliferative disease is cancer, more preferably wherein the hyperproliferative disease is bladder, brain, breast, colon, liver, lung, pancreatic, prostate, HNSCC or stomach cancer comprising administering to said subject a therapeutically effective amount of a tissue-targeting compound of formula (I) according to any of claims 1 to 4.

13. A composition comprising a tissue-targeting compound of formula (I) according to any of claims 1 to 4 together with pharmaceutically acceptable ingredients.

14. A kit comprising a combination of
a tissue-targeting compound of formula (I) according to any of claims 1 to 4 and a pharmaceutical agent;
and, optionally, one or more further pharmaceutical agents;
in which optionally both or either one of the tissue-targeting compound of formula (I) or the pharmaceutical agent are in the form of a pharmaceutical formulation which is ready for use to be administered simultaneously, concurrently, separately or sequentially.
